# EUROPEAN PATENT APPLICATION

(11) **EP 1 614 755 A1**
(43) Date of publication of application: **11.01.2006**
(21) Application number: 04103229.3
(22) Date of filing: 07.07.2004
(51) Int. Cl.: C12N 15/90, C12N 15/85, C12N 5/10, A01K 67/027

(54) **Target transgenesis of short hairpin RNA expression cassette using recombinase mediated cassette exchange**

(71) Applicant: ARTEMIS Pharmaceuticals GmbH, 51062 Köln (DE)
(72) Inventor: Seibler, Jost, dr., 50733 Köln (DE); Schwenk, Frieder, Dr., 50999 Köln (DE); Kern, Heidrun, 40227 Düsseldorf (DE)
(74) Representative: Helbing, Jörg

(57) **Abstract**

The invention provides a method for targeted transgenesis of short hairpin RNA expression cassettes using recombinase mediated cassette exchange. Suitable nucleotide acid sequences and vectors for the targeted transgenesis and recombinase mediated transgenesis are provided.

SEQUENCE LISTING

<110> ARTEMIS Pharmaceutical GmbH

<120> Targeted Transgenesis Using the Rosa26 Locus

<130> 032917wo JH/BM

<140>
<141>

<160> 9

<170> PatentIn Ver. 2.1

<210> 1
<211> 23
<212> DNA
<213> Artificial Sequence

<220>
<223> Description of Artificial Sequence: primer
      Rscreen1s

<400> 1
gacaggacag tgcttgttta agg                                         23


<210> 2
<211> 23
<212> DNA
<213> Artificial Sequence

<220>
<223> Description of Artificial Sequence: primer
      Rscreen1as

<400> 2
tgactacaca atattgctcg cac                                         23


<210> 3
<211> 13139
<212> DNA
<213> Mus musculus

<220>
<223> Description: Rosa26 locus

<400> 3
aagcttctca cgtagcaacc agagctccag agccagcagc tgctgccgcc ttgtatactc 60
actcctgtga tccaacacag gagcaacctt ttctttaccc cacccccact tcttaacaca 120
cttttttttg gggggggggg gggaacaagt gctccatgct ggaaggattg gaactatgct 180
tttagaaagg aacaatccta aggtcacttt taaattgagg tctttgattt gaaaatcaac 240
aaataccaaa ttccaaatat tcgttttaat taaaccagca atgtggatat aagcattaag 300
ttttagtttt aaaaaggtca attttccaaa cattcagcaa tcatatttaa atttacagct 360
aggaacaaga gccttgggtc atgtcctacc aaagaacata actcaatatt ctacacatga 420
caatctgaat aaccttaaag cctctaatcc cataacaggc cacaaatttt ggacagagaa 480
ctaatgatcc tcctgagaaa actggaagaa atccagggaa aagaaattcc tgtgtcctcc 540
aaactcagaa atctctaatt atgtcagtat tctctgcttt agtcctaggt cagattgcac 600
acatctaaaa taacctctta aagttttcct cctagcgacc taaaccatta ttaatatcaa 660
attaaccatc aaaacacttt cctctcaata tgctgcacac aaacctcctc ctggaacctc 720
ctccatctgg atcctcccca atcaaaagta taggtattta acatataagc aaggaagtaa 780

tgtaaacatg accttggtca caaatatgtc atctaaaaac aatttagtca aggtatggag 840
gaaattcgag aacctgaatc tttttaagta ttttgagcac aggaacaatt ggcaaaagga 900
atccaggtat agacaaaacc cagagcccag agctctgggc gaaaaatgag ttgctggtga 960
agacgttaca caagtaacat gagaaagcag aaaatgcagg tcatccacgc acccctgacc 1020
caggccagca gggcgggctg cagcatcagt acacaggaga aagatcctta ttcctaagaa 1080
tgagaaaggc aaaggcgccc gatagaataa attagcatag aaggggcttt cccaggagtt 1140
aaaactttcc ttctgagcga ttacctacta aaaccagggc ttttgcccac taccatttac 1200
ctaggatctt ggcttgcacg gattcatagg ggcatatccc tccccctctt ctttagagtc 1260
gttcttaaaa gatcgctctc cacgccctag gcagggaaaa cgacaaaatc tggctcaatt 1320
ccaggctaga accctacaaa ttcaacaggg atatcgcaag gatactgggg catacgccac 1380
agggagtcca agaatgtgag gtgggggtgg cgaaggtaat gtctttggtg tgggaaaagc 1440
agcagccatc tgagatagga actggaaaac cagaggagag gcgttcagga agattatgga 1500
ggggaggact gggcccccac gagcgaccag agttgtcaca aggccgcaag aacaggggag 1560
gtggggggct caggacagaa aaaaaaagta tgtgtatttt gagagcaggg ttgggaggcc 1620
tctcctgaaa agggtataaa cgtggagtag gcaataccca ggcaaaaagg ggagaccaga 1680
gtagggggag gggaagagtc ctgacccagg gaagacatta aaaaggtagt ggggtcgact 1740
agatgaagga gagcctttct ctctgggcaa gagcggtgca atggtgtgta aaggtagctg 1800
agaagacgaa aagggcaagc atcttcctgc taccaggctg ggaggcccca ggcccacgac 1860
cccgaggaga gggaacgcag ggagactgag gtgacccttc tttcccccgg ggcccggtcg 1920
tgtggttcgg tgtctctttt ctgttggacc cttaccttga cccaggcgct gccggggcct 1980
gggcccgggc tgcggcgcac ggcactcccg ggaggcagcg agactcgagt taggcccaac 2040
gcggcgccac ggcgtttcct ggccgggaat ggcccgtacc cgtgaggtgg gggtgggggg 2100
cagaaaaggc ggagcgagcc cgagcgggga gggggagggc caggggcgga gggggccggc 2160
actactgtgt tggcggactg gcgggactag ggctgcgtga gtctctgagc gcaggcgggc 2220
ggcggccgcc cctcccccgg cggcggcagc ggcggcagcg gcggcagctc actcagcccg 2280
ctgcccgagc ggaaacgcca ctgaccgcac ggggattccc agtgccggcg ccaggggcac 2340
gcgggacacg ccccctcccg ccgcgccatt ggcctctccg cccaccgccc cacacttatt 2400
ggccggtgcg ccgccaatca gcggaggctg ccggggccgc ctaaagaaga ggctgtgctt 2460
tggggctccg gctcctcaga gagcctcggc taggtagggg atcgggactc tggcgggagg 2520
gcggcttggt gcgtttgcgg ggatgggcgg ccgcggcagg ccctccgagc gtggtggagc 2580
cgttctgtga gacagccggg tacgagtcgt gacgctggaa ggggcaagcg ggtggtgggc 2640
aggaatgcgg tccgccctgc agcaaccgga gggggaggga gaagggagcg gaaaagtctc 2700
caccggacgc ggccatggct cggggggggg ggggcagcgg aggagcgctt ccggccgacg 2760
tctcgtcgct gattggcttc ttttcctccc gccgtgtgtg aaaacacaaa tggcgtgttt 2820
tggttggcgt aaggcgcctg tcagttaacg gcagccggag tgcgcagccg ccggcagcct 2880
cgctctgccc actgggtggg gcgggaggta ggtggggtga ggcgagctgg acgtgcgggc 2940
gcggtcggcc tctggcgggg cgggggaggg gagggagggt cagcgaaagt agctcgcgcg 3000
cgagcggccg cccaccctcc ccttcctctg ggggagtcgt tttacccgcc gccggccggg 3060
cctcgtcgtc tgattggctc tcgggcccca gaaaactggc ccttgccatt ggctcgtgtt 3120
cgtgcaagtt gagtccatcc gccggccagc gggggcggcg aggaggcgct cccaggttcc 3180
ggccctcccc tcggccccgc gccgcagagt ctggccgcgc gcccctgcgc aacgtggcag 3240
gaagcgcgcg ctgggggcgg ggacgggcag tagggctgag cggctgcggg gcgggtgcaa 3300
gcacgtttcc gacttgagtt gcctcaagag gggcgtgctg agccagacct ccatcgcgca 3360
ctccggggag tggagggaag gagcgagggc tcagttgggc tgttttggag gcaggaagca 3420
cttgctctcc caaagtcgct ctgagttgtt atcagtaagg gagctgcagt ggagtaggcg 3480
gggagaaggc cgcacccttc tccggagggg ggaggggagt gttgcaatac ctttctggga 3540
gttctctgct gcctcctggc ttctgaggac cgccctgggc ctgggagaat cccttccccc 3600
tcttccctcg tgatctgcaa ctccagtctt tctagaagat gggcgggagt cttctgggca 3660
ggcttaaagg ctaacctggt gtgtgggcgt tgtcctgcag gggaattgaa caggtgtaaa 3720
attggaggga caagacttcc cacagatttt cggttttgtc gggaagtttt ttaatagggg 3780
caaataagga aaatgggagg ataggtagtc atctggggtt ttatgcagca aaactacagg 3840
ttattattgc ttgtgatccg cctcggagta ttttccatcg aggtagatta aagacatgct 3900
cacccgagtt ttatactctc ctgcttgaga tccttactac agtatgaaat tacagtgtcg 3960
cgagttagac tatgtaagca gaattttaat catttttaaa gagcccagta cttcatatcc 4020
atttctcccg ctccttctgc agccttatca aaaggtattt tagaacactc attttagccc 4080
cattttcatt tattatactg gcttatccaa cccctagaca gagcattggc attttccctt 4140
tcctgatctt agaagtctga tgactcatga aaccagacag attagttaca tacaccacaa 4200
atcgaggctg tagctggggc ctcaacactg cagttctttt ataactcctt agtacacttt 4260
ttgttgatcc tttgccttga tccttaattt tcagtgtcta tcacctctcc cgtcagtggt 4320
gttccacatt tgggcctatt ctcagtccag ggagttttac aacaatagat gtattgagaa 4380
tccaacctaa agcttaactt tccactccca tgaatgcctc tctccttttt ctccatttat 4440
aaactgagct attaaccatt aatggttcca ggtggatgtc tcctccccat attacctgat 4500
gtatcttaca tattgccagg ctgatatttt aagacattaa aaggtatatt tcattattga 4560

gccacatggt attgattact gcttactaaa attttgtcat tgtacacatc tgtaaaaggt 4620
ggttcctttt ggaatgcaaa gttcaggtgt ttgttgtctt tcctgaccta aggtcttgtg 4680
agcttgtatt ttttctattt aagcagtgct ttctcttgga ctggcttgac tcatggcatt 4740
ctacacgtta ttgctggtct aaatgtgatt ttgccaagct tcttcaggac ctataatttt 4800
gcttgacttg tagccaaaca caagtaaaat gattaagcaa caaatgtatt tgtgaagctt 4860
ggtttttagg ttgttgtgtt gtgtgtgctt gtgctctata ataatactat ccaggggctg 4920
gagaggtggc tcggagttca agagcacaga ctgctcttcc agaagtcctg agttcaattc 4980
ccagcaacca catggtggct cacaaccatc tgtaatggga tctgatgccc tcttctggtg 5040
tgtctgaaga ccacaagtgt attcacatta aataaataaa tcctccttct tcttcttttt 5100
ttttttttta aagagaatac tgtctccagt agaatttact gaagtaatga aatactttgt 5160
gtttgttcca atatggtagc caataatcaa attactcttt aagcactgga aatgttacca 5220
aggaactaat tttatttgaa agtgtaactg tggacagagg agccataact gcagacttgt 5280
gggatacaga agaccaatgc agactttaat gtcttttctc ttacactaag caataaagaa 5340
ataaaaattg aacttctagt atcctatttg tttaaactgc tagctttact taacttttgt 5400
gcttcatcta tacaaagctg aaagctaagt ctgcagccat tactaaacat gaaagcaagt 5460
aatgataatt ttggatttca aaaatgtagg gccagagttt agccagccag tggtggtgct 5520
tgcctttatg cctttaatcc cagcactctg gaggcagaga caggcagatc tctgagtttg 5580
agcccagcct ggtctacaca tcaagttcta tctaggatag ccaggaatac acacagaaac 5640
cctgttgggg aggggggctc tgagatttca taaaattata attgaagcat tccctaatga 5700
gccactatgg atgtggctaa atccgtctac ctttctgatg agatttgggt attatttttt 5760
ctgtctctgc tgttggttgg gtcttttgac actgtgggct ttctttaaag cctccttcct 5820
gccatgtggt ctcttgtttg ctactaactt cccatggctt aaatggcatg gctttttgcc 5880
ttctaagggc agctgctgag atttgcagcc tgatttccag ggtggggttg ggaaatcttt 5940
caaacactaa aattgtcctt taattttttt tttaaaaaat gggttatata ataaacctca 6000
taaaatagtt atgaggagtg aggtggacta atattaaatg agtccctccc ctataaaaga 6060
gctattaagg ctttttgtct tatacttaac ttttttttta aatgtggtat ctttagaacc 6120
aagggtctta gagttttagt atacagaaac tgttgcatcg cttaatcaga ttttctagtt 6180
tcaaatccag agaatccaaa ttcttcacag ccaaagtcaa attaagaatt tctgactttt 6240
aatgttaatt tgcttactgt gaatataaaa atgatagctt ttcctgaggc agggtctcac 6300
tatgtatctc tgcctgatct gcaacaagat atgtagacta aagttctgcc tgcttttgtc 6360
tcctgaatac taaggttaaa atgtagtaat acttttggaa cttgcaggtc agattctttt 6420
ataggggaca cactaaggga gcttgggtga tagttggtaa aatgtgtttc aagtgatgaa 6480
aacttgaatt attatcaccg caacctactt tttaaaaaaa aaagccaggc ctgttagagc 6540
atgcttaagg gatccctagg acttgctgag cacacaagag tagttacttg gcaggctcct 6600
ggtgagagca tatttcaaaa aacaaggcag acaaccaaga aactacagtt aaggttacct 6660
gtctttaaac catctgcata tacacaggga tattaaaata ttccaaataa tatttcattc 6720
aagttttccc ccatcaaatt gggacatgga tttctccggt gaataggcag agttggaaac 6780
taaacaaatg ttggttttgt gatttgtgaa attgttttca agtgatagtt aaagcccatg 6840
agatacagaa caaagctgct atttcgaggt ctcttggttt atactcagaa gcacttcttt 6900
gggtttccct gcactatcct gatcatgtgc taggcctacc ttaggctgat tgttgttcaa 6960
ataaacttaa gtttcctgtc aggtgatgtc atatgatttc atatatcaag gcaaaacatg 7020
ttatatatgt taaacatttg tacttaatgt gaaagttagg tctttgtggg tttgattttt 7080
aattttcaaa acctgagcta aataagtcat ttttacatgt cttacatttg gtggaattgt 7140
ataattgtgg tttgcaggca agactctctg acctagtaac cctacctata gagcactttg 7200
ctgggtcaca agtctaggag tcaagcattt caccttgaag ttgagacgtt ttgttagtgt 7260
atactagttt atatgttgga ggacatgttt atccagaaga tattcaggac tatttttgac 7320
tgggctaagg aattgattct gattagcact gttagtgagc attgagtggc ctttaggctt 7380
gaattggagt cacttgtata tctcaaataa tgctggcctt ttttaaaaag cccttgttct 7440
ttatcaccct gttttctaca taatttttgt tcaaagaaat acttgtttgg atctcctttt 7500
gacaacaata gcatgttttc aagccatatt ttttttcctt tttttttttt tttttggttt 7560
ttcgagacag ggtttctctg tatagccctg gctgtcctgg aactcacttt gtagaccagg 7620
ctggcctcga actcagaaat ccgcctgcct ctgcctcctg agtgccggga ttaaaggcgt 7680
gcaccaccac gcctggctaa gttggatatt ttgttatata actataacca atactaactc 7740
cactgggtgg atttttaatt cagtcagtag tcttaagtgg tctttattgg cccttcatta 7800
aaatctactg ttcactctaa cagaggctgt tggtactagt ggcacttaag caacttccta 7860
cggatatact agcagattaa gggtcaggga tagaaactag tctagcgttt tgtataccta 7920
ccagctttat actaccttgt tctgatagaa atatttcagg acatctagag tgtactataa 7980
ggttgatggt aagcttataa ggaacttgaa agtggagtaa ctactccatt tctctgaggg 8040
gagaattaaa atttttgacc aagtgttgtt gagccactga gaatggtctc agaacataac 8100
ttcttaagga accttcccag attgccctca acactgcacc acatttggtc ctgcttgaac 8160
attgccatgg ctcttaaagt cttaattaag aatattaatt gtgtaattat tgtttttcct 8220
cctttagatc attccttgag gacaggacag tgcttgttta aggctatatt tctgctgtct 8280
gagcagcaac aggtcttcga gatcaacatg atgttcataa tcccaagatg ttgccattta 8340

tgttctcaga agcaagcaga ggcatgatgg tcagtgacag taatgtcact gtgttaaatg 8400
ttgctatgca gtttggattt ttctaatgta gtgtaggtag aacatatgtg ttctgtatga 8460
attaaactct taagttacac cttgtataat ccatgcaatg tgttatgcaa ttaccatttt 8520
aagtattgta gctttctttg tatgtgagga taaaggtgtt tgtcataaaa tgttttgaac 8580
atttccccaa agttccaaat tataaaacca caacgttaga acttatttat gaacaatggt 8640
tgtagtttca tgcttttaaa atgcttaatt attcaattaa caccgtttgt gttataatat 8700
atataaaact gacatgtaga agtgtttgtc cagaacattt cttaaatgta tactgtcttt 8760
agagagttta atatagcatg tcttttgcaa catactaact tttgtgttgg tgcgagcaat 8820
attgtgtagt cattttgaaa ggagtcattt caatgagtgt cagattgttt tgaatgttat 8880
tgaacatttt aaatgcagac ttgttcgtgt tttagaaagc aaaactgtca gaagctttga 8940
actagaaatt aaaaagctga agtatttcag aagggaaata agctacttgc tgtattagtt 9000
gaaggaaagt gtaatagctt agaaaattta aaaccatata gttgtcattg ctgaatatct 9060
ggcagatgaa aagaaatact cagtggttct tttgagcaat ataacagctt gttatattaa 9120
aaattttccc cacagatata aactctaatc tataactcat aaatgttaca aatggatgaa 9180
gcttacaaat gtggcttgac ttgtcactgt gcttgtttta gttatgtgaa agtttggcaa 9240
taaacctatg tcctaaatag tcaaactgtg gaatgacttt ttaatctatt ggtttgtcta 9300
gaacagttat gttgccattt gccctaatgg tgaaagaaaa agtggggagt gccttggcac 9360
tgttcatttg tggtgtgaac caaagagggg ggcatgcact tacacttcaa acatcctttt 9420
gaaagactga caagtttggg tcttcacagt tggaattggg catccctttt gtcagggagg 9480
gagggaggga gggaggctgg cttgttatgc tgacaagtgt gattaaattc aaactttgag 9540
gtaagttgga ggaacttgta cattgttagg agtgtgacaa tttggactct taatgatttg 9600
gtcatacaaa atgaacctag accaacttct ggaagatgta tataataact ccatgttaca 9660
ttgatttcac ctgactaata cttatccctt atcaattaaa tacagaagat gccagccatc 9720
tgggcctttt aacccagaaa tttagtttca aactcctagg ttagtgttct cactgagcta 9780
catcctgatc tagtcctgaa aataggacca ccatcacccc caaaaaaatc tcaaataaga 9840
tttatgctag tgtttcaaaa ttttaggaat aggtaagatt agaaagtttt aaattttgag 9900
aaatggcttc tctagaaaga tgtacatagt gaacactgaa tggctcctaa agagcctaga 9960
aaactggtac tgagcacaca ggactgagag gtctttcttg aaaagcatgt attgctttac 10020
gtgggtcaca gaaggcaggc aggaagaact tgggctgaaa ctggtgtctt aagtggctaa 10080
catcttcaca actgatgagc aagaacttta tcctgatgca aaaaccatcc aaacaaacta 10140
agtgaaaggt ggcaatggat cccaggctgc tctagaggag gacttgactt ctcatcccat 10200
cacccacacc agatagctca tagactgcca attaacacca gcttctagcc tccacaggca 10260
cctgcactgg tacacataat ttcacacaaa cacagtaaga agccttccac ctggcatggt 10320
attgcttatc tttagttccc aacacttggg aggcagaggc cagccagggc tatgtgacaa 10380
aaaccttgtc tagaggagaa acttcatagc ttatttccta ttcacgtaac caggttagca 10440
aaatttacca gccagagatg aagctaacag tgtccactat atttgtagtg ttttaagtca 10500
attttttaaa tatacttaat agaattaaag ctatggtgaa ccaagtacaa acctggtgta 10560
ttaacttgag aacttagcat aaaaagtagt tcatttgttc agtaaatatt aaatgcttac 10620
tggcaaagat tatgtcagga acttggtaaa tggtgatgaa acaatcatag ttgtacatct 10680
tggttctgtg atcaccttgg tttgaggtaa aagtggttcc tttgatcaag gatggaattt 10740
taagtttata ttcaatcaat aatgtattat tttgtgattg caaaattgcc tatctagggt 10800
ataaaacctt taaaaatttc ataataccag ttcattctcc agttactaat tccaaaaagc 10860
cactgactat ggtgccaatg tggattctgt tctcaaagga aggattgtct gtgcccttta 10920
ttctaataga aacatcacac tgaaaatcta agctgaaaga agccagactt tcctaaataa 10980
ataactttcc ataaagctca aacaaggatt acttttagga ggcactgtta aggaactgat 11040
aagtaatgag gttacttata taatgatagt cccacaagac tatctgagga aaaatcagta 11100
caactcgaaa acagaacaac cagctaggca ggaataacag ggctcccaag tcaggaggtc 11160
tatccaacac ccttttctgt tgagggcccc agacctacat attgtataca aacagggagg 11220
tgggtgattt taactctcct gaggtacctt ggtaaatctt tgtcctgagt aagcagtaca 11280
gtgtacagtt tacattttca tttaaagata cattagctcc ctctaccccc taagactgac 11340
aggcactttg ggggtgggga gggctttgga aaataacgct tccatacact aaaagagaaa 11400
tttctttaat taggcttgtt ggttccatac atctactggt gtttctacta cttagtaata 11460
ttataatagt cacacaagca tctttgctct gtttaggttg tatatttatt ttaaggcaga 11520
tgataaaact gtagatctta agggatgctt ctgcttctga gatgatacaa agaatttaga 11580
ccataaaaca gtaggttgca caagcaatag aatatggcct aaagtgttct gacacttaga 11640
agccaagcag tgtaggcttc ttaagaaata ccattacaat caccttgcta gaaatcaagc 11700
attctggagt ggtcaagcag tgtaacctgt actgtaagtt acttttctgc tatttttctc 11760
ccaaagcaag ttctttatgc tgatatttcc agtgttagga actacaaata ttaataagtt 11820
gtcttcactc ttttctttac caaggagggt ctcttccttc atcttgatct gaaggatgaa 11880
caaaggcttg agcagtgcgc tttagaagat aaactgcagc atgaaggccc ccgatgttca 11940
cccagactac atggaccttt cgccacacat gtcccattcc agataaggcc tggcacacac 12000
aaaaaacata agtcattagg ctaccagtct gattctaaaa caacctaaaa tcttcccact 12060
taaatgctat gggtggtggg ttggaaagtt gactcagaaa atcacttgct gtttttagag 12120

aggatctggg ttcagtttct gatacattgt ggcttacaac tataactcca gttctagggg 12180
gtccatccaa catcctcttc tgttgagggc accaaataaa tgtattgtgt acaaacaggg 12240
aggtgagtga tttaactctc gtgtatagta ccttggtaaa acatttcttg tcctgagtaa 12300
gcagtacagc tctgcctgtc cctggtctac agacacggct catttcccga aggcaagctg 12360
gatagagatt ccaatttctc ttcttggatc ccatcctata aaagaaggtc aagtttaatc 12420
tattgcaaaa ggtaaatagg tagtttctta catgagacaa gaacaaatct taggtgtgaa 12480
gcagtcatct tttacaggcc agagcctcta ttctatgcca atgaaggaaa ctgttagtcc 12540
agtgttatag agttagtcca gtgtatagtt ttctatcaga acactttttt tttaaacaac 12600
tgcaacttag cttattgaag acaaaccacg agtagaaatc tgtccaagaa gcaagtgctt 12660
ctcagcctac aatgtggaat aggaccatgt aatggtacag tgagtgaaat gaattatggc 12720
atgtttttct gactgagaag acagtacaat aaaaggtaaa ctcatggtat ttatttaaaa 12780
agaatccaat ttctaccttt ttccaaatgg catatctgtt acaataatat ccacagaagc 12840
agttctcagt gggaggttgc agatatccca ctgaacagca tcaatggcca aaccccaggt 12900
tgtttttctg tggagacaaa ggtaagatat ttcaatatat tttcccaagc taatgagatg 12960
gctcagcaaa taatggtact ggccattaag tctcatgacc tgagcttgat cctcagggac 13020
catgtggtac aaggagagac ctaaatcctt cagttggact tcaatcttct accctcatgt 13080
ccacacacaa ataaatacaa taaaaaacat tctgcagtcg aatttctaaa agggcgaat 13139

<210> 4
<211> 1073
<212> DNA
<213> Mus musculus

<220>
<223> Description: 5' arm for Rosa26

<400> 4
caggccctcc gagcgtggtg gagccgttct gtgagacagc cgggtacgag tcgtgacgct 60
ggaaggggca agcgggtggt gggcaggaat gcggtccgcc ctgcagcaac cggaggggga 120
gggagaaggg agcggaaaag tctccaccgg acgcggccat ggctcggggg ggggggggca 180
gcggaggagc gcttccggcc gacgtctcgt cgctgattgg cttcttttcc tcccgccgtg 240
tgtgaaaaca caaatggcgt gttttggttg gcgtaaggcg cctgtcagtt aacggcagcc 300
ggagtgcgca gccgccggca gcctcgctct gcccactggg tggggcggga ggtaggtggg 360
gtgaggcgag ctggacgtgc gggcgcggtc ggcctctggc ggggcggggg aggggaggga 420
gggtcagcga aagtagctcg cgcgcgagcg gccgcccacc ctccccttcc tctgggggag 480
tcgttttacc cgccgccggc cgggcctcgt cgtctgattg gctctcgggg cccagaaaac 540
tggcccttgc cattggctcg tgttcgtgca agttgagtcc atccgccggc cagcgggggc 600
ggcgaggagg cgctcccagg ttccggccct cccctcggcc ccgcgccgca gagtctggcc 660
gcgcgcccct gcgcaacgtg gcaggaagcg cgcgctgggg gcggggacgg gcagtagggc 720
tgagcggctg cggggcgggt gcaagcacgt ttccgacttg agttgcctca agaggggcgt 780
gctgagccag acctccatcg cgcactccgg ggagtggagg gaaggagcga gggctcagtt 840
gggctgtttt ggaggcagga agcacttgct ctcccaaagt cgctctgagt tgttatcagt 900
aagggagctg cagtggagta ggcggggaga aggccgcacc cttctccgga gggggagggg 960
gagtgttgca atacctttct gggagttctc tgctgcctcc tggcttctga ggaccgccct 1020
gggcctggga gaatcccttc cccctcttcc ctcgtgatct gcaactccag tct 1073

<210> 5
<211> 4333
<212> DNA
<213> Mus musculus

<220>
<223> Description: 3' arm for Rosa26

<400> 5
tagaagatgg gcgggagtct tctgggcagg cttaaaggct aacctggtgt gtgggcgttg 60
tcctgcaggg gaattgaaca ggtgtaaaat tggagggaca agacttccca cagattttcg 120
gttttgtcgg gaagtttttt aataggggca aataaggaaa atgggaggat aggtagtcat 180
ctggggtttt atgcagcaaa actacaggtt attattgctt gtgatccgcc tcggagtatt 240
ttccatcgag gtagattaaa gacatgctca cccgagtttt atactctcct gcttgagatc 300
cttactacag tatgaaatta cagtgtcgcg agttagacta tgtaagcaga attttaatca 360

tttttaaaga gcccagtact tcatatccat ttctcccgct ccttctgcag ccttatcaaa 420
aggtatttta gaacactcat tttagcccca ttttcattta ttatactggc ttatccaacc 480
cctagacaga gcattggcat tttccctttc ctgatcttag aagtctgatg actcatgaaa 540
ccagacagat tagttacata caccacaaat cgaggctgta gctggggcct caacactgca 600
gttcttttat aactccttag tacacttttt gttgatcctt tgccttgatc cttaattttc 660
agtgtctatc acctctcccg tcagtggtgt tccacatttg ggcctattct cagtccaggg 720
agttttacaa caatagatgt attgagaatc caacctaaag cttaactttc cactcccatg 780
aatgcctctc tcctttttct ccatttataa actgagctat taaccattaa tggttccagg 840
tggatgtctc ctccccatat tacctgatgt atcttacata ttgccaggct gatattttaa 900
gacattaaaa ggtatatttc attattgagc cacatggtat tgattactgc ttactaaaat 960
tttgtcattg tacacatctg taaaaggtgg ttccttttgg aatgcaaagt tcaggtgttt 1020
gttgtctttc ctgacctaag gtcttgtgag cttgtatttt ttctatttaa gcagtgcttt 1080
ctcttggact ggcttgactc atggcattct acacgttatt gctggtctaa atgtgatttt 1140
gccaagcttc ttcaggacct ataattttgc ttgacttgta gccaaacaca agtaaaatga 1200
ttaagcaaca aatgtatttg tgaagcttgg tttttaggtt gttgtgttgt gtgtgcttgt 1260
gctctataat aatactatcc aggggctgga gaggtggctc ggagttcaag agcacagact 1320
gctcttccag aagtcctgag ttcaattccc agcaaccaca tggtggctca caaccatctg 1380
taatgggatc tgatgccctc ttctggtgtg tctgaagacc acaagtgtat tcacattaaa 1440
taaataaatc ctccttcttc ttcttttttt tttttttaaa gagaatactg tctccagtag 1500
aatttactga agtaatgaaa tactttgtgt ttgttccaat atggtagcca ataatcaaat 1560
tactctttaa gcactggaaa tgttaccaag gaactaattt ttatttgaag tgtaactgtg 1620
gacagaggag ccataactgc agacttgtgg gatacagaag accaatgcag actttaatgt 1680
cttttctctt acactaagca ataaagaaat aaaaattgaa cttctagtat cctatttgtt 1740
taaactgcta gctttactta acttttgtgc ttcatctata caaagctgaa agctaagtct 1800
gcagccatta ctaaacatga aagcaagtaa tgataatttt ggatttcaaa aatgtagggc 1860
cagagtttag ccagccagtg gtggtgcttg cctttatgcc tttaatccca gcactctgga 1920
ggcagagaca ggcagatctc tgagtttgag cccagcctgg tctacacatc aagttctatc 1980
taggatagcc aggaatacac acagaaaccc tgttggggag gggggctctg agatttcata 2040
aaattataat tgaagcattc cctaatgagc cactatggat gtggctaaat ccgtctacct 2100
ttctgatgag atttgggtat tattttttct gtctctgctg ttggttgggt cttttgacac 2160
tgtgggcttt ctttaaagcc tccttcctgc catgtggtct cttgtttgct actaacttcc 2220
catggcttaa atggcatggc tttttgcctt ctaagggcag ctgctgagat ttgcagcctg 2280
atttccaggg tggggttggg aaatctttca aacactaaaa ttgtccttta attttttttt 2340
taaaaaatgg gttatataat aaacctcata aaatagttat gaggagtgag gtggactaat 2400
attaaatgag tccctcccct ataaaagagc tattaaggct tttgtcttta tacttaactt 2460
tttttttaaa tgtggtatct ttagaaccaa gggtcttaga gttttagtat acagaaactg 2520
ttgcatcgct taatcagatt ttctagtttc aaatccagag aatccaaatt cttcacagcc 2580
aaagtcaaat taagaatttc tgacttttaa tgttaatttg cttactgtga atataaaaat 2640
gatagctttt cctgaggcag ggtctcacta tgtatctctg cctgatctgc aacaagatat 2700
gtagactaaa gttctgcctg cttttgtctc ctgaatacta aggttaaaat gtagtaatac 2760
ttttggaact tgcaggtcag attcttttat aggggacaca ctaagggagc ttgggtgata 2820
gttggtaaaa tgtgtttcaa gtgatgaaaa cttgaattat tatcaccgca acctactttt 2880
taaaaaaaaa agccaggcct gttagagcat gcttaaggga tccctaggac ttgctgagca 2940
cacaagagta gttacttggc aggctcctgg tgagagcata tttcaaaaaa caaggcagac 3000
aaccaagaaa ctacagttaa ggttacctgt ctttaaacca tctgcatata cacagggata 3060
ttaaaatatt ccaaataata tttcattcaa gttttccccc atcaaattgg gacatggatt 3120
tctccggtga ataggcagag ttggaaacta aacaaatgtt ggttttgtga tttgtgaaat 3180
tgttttcaag tgatagttaa agcccatgag atacagaaca aagctgctat ttcgaggtct 3240
cttggtttat actcagaagc acttctttgg gtttccctgc actatcctga tcatgtgcta 3300
ggcctacctt aggctgattg ttgttcaaat aaacttaagt ttcctgtcag gtgatgtcat 3360
atgatttcat atatcaaggc aaaacatgtt atatatgtta aacatttgta cttaatgtga 3420
aagttaggtc tttgtgggtt tgatttttaa ttttcaaaac ctgagctaaa taagtcattt 3480
ttacatgtct tacatttggt ggaattgtat aattgtggtt tgcaggcaag actctctgac 3540
ctagtaaccc tacctataga gcactttgct gggtcacaag tctaggagtc aagcatttca 3600
ccttgaagtt gagacgtttt gttagtgtat actagtttat atgttggagg acatgtttat 3660
ccagaagata ttcaggacta tttttgactg ggctaaggaa ttgattctga ttagcactgt 3720
tagtgagcat tgagtggcct ttaggcttga attggagtca cttgtatatc tcaaataatg 3780
ctggcctttt ttaaaaagcc cttgttcttt atcaccctgt tttctacata atttttgttc 3840
aaagaaatac ttgtttggat ctccttttga caacaatagc atgttttcaa gccatatttt 3900
ttttcctttt tttttttttt tttggttttt cgagacaggg tttctctgta tagccctggc 3960
tgtcctggaa ctcactttgt agaccaggct ggcctcgaac tcagaaatcc gcctgcctct 4020
gcctcctgag tgccgggatt aaaggcgtgc accaccacgc ctggctaagt tggatatttt 4080
gttatataac tataaccaat actaactcca ctgggtggat ttttaattca gtcagtagtc 4140

ttaagtggtc tttattggcc cttcattaaa atctactgtt cactctaaca gaggctgttg 4200
gtactagtgg cacttaagca acttcctacg gatatactag cagattaagg gtcagggata 4260
gaaactagtc tagcgttttg tatacctacc agctttatac taccttgttc tgatagaaat 4320
atttcaggac atc 4333

<210> 6
<211> 6039
<212> DNA
<213> Artificial Sequence

<220>
<223> Description of Artificial Sequence: Insert
      CAGGAS-creER

<400> 6
attgattatt gactagttat taatagtaat caattacggg gtcattagtt catagcccat 60
atatggagtt ccgcgttaca taacttacgg taaatggccc gcctggctga ccgcccaacg 120
acccccgccc attgacgtca ataatgacgt atgttcccat agtaacgcca atagggactt 180
tccattgacg tcaatgggtg gactatttac ggtaaactgc ccacttggca gtacatcaag 240
tgtatcatat gccaagtacg ccccctattg acgtcaatga cggtaaatgg cccgcctggc 300
attatgccca gtacatgacc ttatgggact ttcctacttg gcagtacatc tacgtattag 360
tcatcgctat taccatgggt cgaggtgagc cccacgttct gcttcactct ccccatctcc 420
cccccctccc cacccccaat tttgtattta tttatttttt aattattttg tgcagcgatg 480
ggggcggggg gggggggggc gcgcgccagg cggggcgggg cggggcgagg ggcggggcgg 540
ggcgaggcgg agaggtgcgg cggcagccaa tcagagcggc gcgctccgaa agtttccttt 600
tatggcgagg cggcggcggc ggcggcccta taaaaagcga agcgcgcggc gggcgggagt 660
cgctgcgttg ccttcgcccc gtgccccgct ccgcgccgcc tcgcgccgcc cgccccggct 720
ctgactgacc gcgttactcc cacaggtgag cgggcgggac ggcccttctc ctccgggctg 780
taattagcgc ttggtttaat gacggctcgt ttcttttctg tggctgcgtg aaagccttaa 840
agggctccgg gagggccctt tgtgcggggg ggagcggctc ggggggtgcg tgcgtgtgtg 900
tgtgcgtggg gagcgccgcg tgcggcccgc gctgcccggc ggctgtgagc gctgcgggcg 960
cggcgcgggg ctttgtgcgc tccgcgtgtg cgcgagggga gcgcggccgg gggcggtgcc 1020
ccgcggtgcg ggggggctgc gaggggaaca aaggctgcgt gcggggtgtg tgcgtggggg 1080
ggtgagcagg gggtgtgggc gcggcggtcg ggctgtaacc cccccctgca cccccctccc 1140
cgagttgctg agcacggccc ggcttcgggt gcggggctcc gtgcggggcg tggcgcgggg 1200
ctcgccgtgc cgggcggggg gtggcggcag gtgggggtgc cgggcggggc ggggccgcct 1260
cgggccgggg agggctcggg ggaggggcgc ggcggccccg gagcgccggc ggctgtcgag 1320
gcgcggcgag ccgcagccat tgccttttat ggtaatcgtg cgagagggcg cagggacttc 1380
ctttgtccca aatctggcgg agccgaaatc tgggaggcgc cgccgcaccc cctctagcgg 1440
gcgcgggcga agcggtgcgg cgccggcagg aaggaaatgg gcggggaggg ccttcgtgcg 1500
tcgccgcgcc gccgtcccct tctccatctc cagcctcggg gctgccgcag ggggacggct 1560
gccttcgggg gggacggggc agggcggggt tcggcttctg gcgtgtgacc ggcggctcta 1620
gaagcgttgg ggtgagtact ccctctcaaa agcgggcatg acttctgcgc taagattgtc 1680
agtttccaaa aacgaggagg atttgatatt cacctggccc gcggtgatgc ctttgagggt 1740
ggccgcgtcc atctggtcag aaaagacaat ctttttgttg tcaagcttga ggtgtggcag 1800
gcttgagatc tggccataca cttgagtgac attgacatcc actttgcctt tctctccaca 1860
ggtgtccact cccagggcgg cctccggagc gatcgccggt ccgcctaggc tagccggccg 1920
cgtcgaccat gtccaattta ctgaccgtac accaaaattt gcctgcatta ccggtcgatg 1980
caacgagtga tgaggttcgc aagaacctga tggacatgtt cagggatcgc caggcgtttt 2040
ctgagcatac ctggaaaatg cttctgtccg tttgccggtc gtgggcggca tggtgcaagt 2100
tgaataaccg gaaatggttt cccgcagaac ctgaagatgt tcgcgattat cttctatatc 2160
ttcaggcgcg cggtctggca gtaaaaacta tccagcaaca tttgggccag ctaaacatgc 2220
ttcatcgtcg gtccgggctg ccacgaccaa gtgacagcaa tgctgtttca ctggttatgc 2280
ggcggatccg aaaagaaaac gttgatgccg gtgaacgtgc aaaacaggct ctagcgttcg 2340
aacgcactga tttcgaccag gttcgttcac tcatggaaaa tagcgatcgc tgccaggata 2400
tacgtaatct ggcatttctg gggattgctt ataacaccct gttacgtata gccgaaattg 2460
ccaggatcag ggttaaagat atctcacgta ctgacggtgg gagaatgtta atccatattg 2520
gcagaacgaa aacgctggtt agcaccgcag gtgtagagaa ggcacttagc ctgggggtaa 2580
ctaaactggt cgagcgatgg atttccgtct ctggtgtagc tgatgatccg aataactacc 2640
tgttttgccg ggtcagaaaa aatggtgttg ccgcgccatc tgccaccagc cagctatcaa 2700
ctcgcgccct ggaagggatt tttgaagcaa ctcatcgatt gatttacggc gctaaggatg 2760
actctggtca gagatacctg gcctggtctg gacacagtgc ccgtgtcgga gccgcgcgag 2820

atatggcccg cgctggagtt tcaataccgg agatcatgca agctggtggc tggaccaatg 2880
taaatattgt catgaactat atccgtaacc tggatagtga aacaggggca atggtgcgcc 2940
tgctggaaga tggcgattct gctggagaca tgagagctgc caacctttgg ccaagcccgc 3000
tcatgatcaa acgctctaag aagaacagcc tggccttgtc cctgacggcc gaccagatgg 3060
tcagtgcctt gttggatgct gagcccccca tactctattc cgagtatgat cctaccagac 3120
ccttcagtga agcttcgatg atgggcttac tgaccaacct ggcagacagg gagctggttc 3180
acatgatcaa ctgggcgaag agggtgccag gctttgtgga tttgaccctc catgatcagg 3240
tccaccttct agaatgtgcc tggctagaga tcctgatgat tggtctcgtc tggcgctcca 3300
tggagcaccc agtgaagcta ctgtttgctc ctaacttgct cttggacagg aaccagggaa 3360
aatgtgtaga gggcatggtg gagatcttcg acatgctgct ggctacatca tctcggttcc 3420
gcatgatgaa tctgcaggga gaggagtttg tgtgcctcaa atctattatt ttgcttaatt 3480
ctggagtgta cacatttctg tccagcaccc tgaagtctct ggaagagaag gaccatatcc 3540
accgagtcct ggacaagatc acagacactt tgatccacct gatggccaag gcaggcctga 3600
ccctgcagca gcagcaccag cggctggccc agctcctcct catcctctcc cacatcaggc 3660
acatgagtaa caaaggcatg gagcatctgt acagcatgaa gtgcaagaac gtggtgcccc 3720
tctatgacct gctgctggag gcggccgacg cccaccgcct acatgcgccc actagccgtg 3780
gaggggcatc cgtggaggag acggaccaaa gccacttggc cactgcgggc tctacttcat 3840
cgcattcctt gcaaaagtat tacatcacgg gggaggcaga gggtttccct gccacagtct 3900
gagcggccga ccggttcgag atccaggcgc ggatcaataa aagatcatta ttttcaatag 3960
atctgtgtgt tggttttttg tgtgccttgg gggaggggga ggccagaatg aggcgcggcc 4020
aagggggagg gggaggccag aatgaccttg ggggaggggg aggccagaat gaccttgggg 4080
gagggggagg ccagaatgag gcgcgccggt aaccgaagtt cctatacttt ctagagaata 4140
ggaacttcgg aataggaact tcttaggtca attctaccgg gtaggggagg cgcttttccc 4200
aaggcagtct ggagcatgcg ctttagcagc cccgctgggc acttggcgct acacaagtgg 4260
cctctggcct cgcacacatt ccacatccac cggtaggcgc caaccggctc cgttctttgg 4320
tggccccttc gcgccacctt ctactcctcc cctagtcagg aagttccccc ccgccccgca 4380
gctcgcgtcg tgcaggacgt gacaaatgga agtagcacgt ctcactagtc tcgtgcagat 4440
ggacagcacc gctgagcaat ggaagcgggt aggcctttgg ggcagcggcc aatagcagct 4500
ttgctccttc gctttctggg ctcagaggct gggaaggggt gggtccgggg gcgggctcag 4560
gggcgggctc aggggcgggg cgggcgcccg aaggtcctcc ggaggcccgg cattctgcac 4620
gcttcaaaag cgcacgtctg ccgcgctgtt ctcctcttcc tcatctccgg gcctttcgac 4680
ctgcagccaa tatgggatcg gccattgaac aagatggatt gcacgcaggt tctccggccg 4740
cttgggtgga gaggctattc ggctatgact gggcacaaca gacaatcggc tgctctgatg 4800
ccgccgtgtt ccggctgtca gcgcaggggc gcccggttct ttttgtcaag accgacctgt 4860
ccggtgccct gaatgaactg caggacgagg cagcgcggct atcgtggctg gccacgacgg 4920
gcgttccttg cgcagctgtg ctcgacgttg tcactgaagc gggaagggac tggctgctat 4980
tgggcgaagt gccggggcag gatctcctgt catctcacct tgctcctgcc gagaaagtat 5040
ccatcatggc tgatgcaatg cggcggctgc atacgcttga tccggctacc tgcccattcg 5100
accaccaagc gaaacatcgc atcgagcgag cacgtactcg gatggaagcc ggtcttgtcg 5160
atcaggatga tctggacgaa gagcatcagg ggctcgcgcc agccgaactg ttcgccaggc 5220
tcaaggcgcg catgcccgac ggcgaggatc tcgtcgtgac ccatggcgat gcctgcttgc 5280
cgaatatcat ggtggaaaat ggccgctttt ctggattcat cgactgtggc cggctgggtg 5340
tggcggaccg ctatcaggac atagcgttgg ctacccgtga tattgctgaa gagcttggcg 5400
gcgaatgggc tgaccgcttc ctcgtgcttt acggtatcgc cgctcccgat tcgcagcgca 5460
tcgccttcta tcgccttctt gacgagttct tctgagggga tcgatccgct gtaagtctgc 5520
agaaattgat gatctattaa acaataaaga tgtccactaa aatggaagtt tttcctgtca 5580
tactttgtta agaagggtga gaacagagta cctacatttt gaatggaagg attggagcta 5640
cgggggtggg ggtggggtgg gattagataa atgcctgctc tttactgaag gctctttact 5700
attgctttat gataatgttt catagttgga tatcataatt taaacaagca aaaccaaatt 5760
aagggccagc tcattcctcc cactcatgat ctatagatct atagatctct cgtgggatca 5820
ttgtttttct cttgattccc actttgtggt tctaagtact gtggtttcca aatgtgtcag 5880
tttcatagcc tgaagaacga gatcagcagc ctctgttcca catacacttc attctcagta 5940
ttgttttgcc aagttctaat tccatcagaa gctgactcta gatcccgcgc cgaagttcct 6000
atactttcta gagaatagga acttcggaat aggaacttc 6039

<210> 7
<211> 14411
<212> DNA
<213> Artificial Sequence

<220>
<223> Description of Artificial Sequence: targeting

vector for Rosa26 locus with a CAGGS-creER insert

<400> 7
   tttgagtgag ctgataccgc tcgccgcagc cgaacgaccg agcgcagcga gtcagtgagc 60
   gaggaagcgg aagagcgccc aatacgcaaa ccgcctctcc ccgcgcgttg gccgattcat 120
   taatgcagct ggcacgacag gtttcccgac tggaaagcgg gcagtgagcg caacgcaatt 180
   aatgtgagtt agctcactca ttaggcaccc caggctttac actttatgct tccggctcgt 240
   atgttgtgtg gaattgtgag cggataacaa tttcacacag gaaacagcta tgaccatgat 300
   tacgccaagc gcgcaattaa ccctcactaa agggaacaaa agctgtcgag atctagatat 360
   cgatggccat agagttacgc tagggataac agggtaatat agccgcggca ggccctccga 420
   gcgtggtgga gccgttctgt gagacagccg ggtacgagtc gtgacgctgg aaggggcaag 480
   cgggtggtgg gcaggaatgc ggtccgccct gcagcaaccg gagggggagg gagaagggag 540
   cggaaaagtc tccaccggac gcggccatgg ctcggggggg ggggggcagc ggaggagcgc 600
   ttccggccga cgtctcgtcg ctgattggct tcttttcctc ccgccgtgtg tgaaaacaca 660
   aatggcgtgt tttggttggc gtaaggcgcc tgtcagttaa cggcagccgg agtgcgcagc 720
   cgccggcagc ctcgctctgc ccactgggtg gggcgggagg taggtggggt gaggcgagct 780
   ggacgtgcgg gcgcggtcgg cctctggcgg ggcgggggag ggagggaggg gtcagcgaaa 840
   gtagctcgcg cgcgagcggc cgcccaccct ccccttcctc tgggggagtc gttttacccg 900
   ccgccggccg ggcctcgtcg tctgattggc tctcggggcc cagaaaactg gcccttgcca 960
   ttggctcgtg ttcgtgcaag ttgagtccat ccgccggcca gcgggggcgg cgaggaggcg 1020
   ctcccaggtt ccggccctcc cctcggcccc gcgccgcaga gtctggccgc gcgcccctgc 1080
   gcaacgtggc aggaagcgcg cgctgggggc ggggacgggc agtagggctg agcggctgcg 1140
   gggcgggtgc aagcacgttt ccgacttgag ttgcctcaag aggggcgtgc tgagccagac 1200
   ctccatcgcg cactccgggg agtggaggga aggagcgagg gctcagttgg gctgttttgg 1260
   aggcaggaag cacttgctct cccaaagtcg ctctgagttg ttatcagtaa gggagctgca 1320
   gtggagtagg cggggagaag gccgcaccct tctccggagg ggggagggga gtgttgcaat 1380
   acctttctgg gagttctctg ctgcctcctg gcttctgagg accgccctgg gcctgggaga 1440
   atcccttccc cctcttccct cgtgatctgc aactccagtc tttctaggta accgatatcc 1500
   ctgcaggttt tcgacattga ttattgacta gttattaata gtaatcaatt acggggtcat 1560
   tagttcatag cccatatatg gagttccgcg ttacataact tacggtaaat ggcccgcctg 1620
   gctgaccgcc caacgacccc cgcccattga cgtcaataat gacgtatgtt cccatagtaa 1680
   cgccaatagg gactttccat tgacgtcaat gggtggacta tttacggtaa actgcccact 1740
   tggcagtaca tcaagtgtat catatgccaa gtacgccccc tattgacgtc aatgacggta 1800
   aatggcccgc ctggcattat gcccagtaca tgaccttatg ggactttcct acttggcagt 1860
   acatctacgt attagtcatc gctattacca tgggtcgagg tgagccccac gttctgcttc 1920
   actctcccca tctccccccc ctccccaccc ccaattttgt atttatttat tttttaatta 1980
   ttttgtgcag cgatgggggc gggggggggg ggggcgcgcg ccaggcgggg cggggcgggg 2040
   cgaggggcgg ggcggggcga ggcggagagg tgcggcggca gccaatcaga gcggcgcgct 2100
   ccgaaagttt ccttttatgg cgaggcggcg gcggcggcgg ccctataaaa agcgaagcgc 2160
   gcggcgggcg ggagtcgctg cgttgccttc gccccgtgcc ccgctccgcg ccgcctcgcg 2220
   ccgcccgccc cggctctgac tgaccgcgtt actcccacag gtgagcgggc gggacggccc 2280
   ttctcctccg ggctgtaatt agcgcttggt ttaatgacgg ctcgtttctt ttctgtggct 2340
   gcgtgaaagc cttaaagggc tccgggaggg ccctttgtgc gggggggagc ggctcggggg 2400
   gtgcgtgcgt gtgtgtgtgc gtggggagcg ccgcgtgcgg cccgcgctgc ccggcggctg 2460
   tgagcgctgc gggcgcggcg cggggctttg tgcgctccgc gtgtgcgcga ggggagcgcg 2520
   gccgggggcg gtgccccgcg gtgcgggggg gctgcgaggg gaacaaaggc tgcgtgcggg 2580
   gtgtgtgcgt gggggggtga gcagggggtg tgggcgcggc ggtcgggctg taaccccccc 2640
   ctgcaccccc ctccccgagt tgctgagcac ggcccggctt cgggtgcggg gctccgtgcg 2700
   gggcgtggcg cggggctcgc cgtgccgggc ggggggtggc ggcaggtggg ggtgccgggc 2760
   ggggcggggc cgcctcgggc cggggagggc tcgggggagg ggcgcggcgg ccccggagcg 2820
   ccggcggctg tcgaggcgcg gcgagccgca gccattgcct tttatggtaa tcgtgcgaga 2880
   gggcgcaggg acttcctttg tcccaaatct ggcggagccg aaatctggga ggcgccgccg 2940
   caccccctct agcgggcgcg ggcgaagcgg tgcggcgccg gcaggaagga aatgggcggg 3000
   gagggccttc gtgcgtcgcc gcgccgccgt ccccttctcc atctccagcc tcggggctgc 3060
   cgcaggggga cggctgcctt cgggggggac ggggcagggc ggggttcggc ttctggcgtg 3120
   tgaccggcgg ctctagaagc gttggggtga gtactccctc tcaaaagcgg gcatgacttc 3180
   tgcgctaaga ttgtcagttt ccaaaaacga ggaggatttg atattcacct ggcccgcggt 3240
   gatgcctttg agggtggccg cgtccatctg gtcagaaaag acaatctttt tgttgtcaag 3300
   cttgaggtgt ggcaggcttg agatctggcc atacacttga gtgacattga catccacttt 3360
   gcctttctct ccacaggtgt ccactcccag ggcggcctcc ggagcgatcg ccggtccgcc 3420
   taggctagcc ggccgcgtcg accatgtcca atttactgac cgtacaccaa aatttgcctg 3480
   cattaccggt cgatgcaacg agtgatgagg ttcgcaagaa cctgatggac atgttcaggg 3540
   atcgccaggc gttttctgag catacctgga aaatgcttct gtccgtttgc cggtcgtggg 3600
   cggcatggtg caagttgaat aaccggaaat ggtttcccgc agaacctgaa gatgttcgcg 3660
   attatcttct atatcttcag gcgcgcggtc tggcagtaaa aactatccag caacatttgg 3720
   gccagctaaa catgcttcat cgtcggtccg ggctgccacg accaagtgac agcaatgctg 3780
   tttcactggt tatgcggcgg atccgaaaag aaaacgttga tgccggtgaa cgtgcaaaac 3840
   aggctctagc gttcgaacgc actgatttcg accaggttcg ttcactcatg gaaaatagcg 3900
   atcgctgcca ggatatacgt aatctggcat ttctggggat tgcttataac accctgttac 3960
   gtatagccga aattgccagg atcagggtta aagatatctc acgtactgac ggtgggagaa 4020
   tgttaatcca tattggcaga acgaaaacgc tggttagcac cgcaggtgta gagaaggcac 4080
   ttagcctggg ggtaactaaa ctggtcgagc gatggatttc cgtctctggt gtagctgatg 4140
   atccgaataa ctacctgttt tgccgggtca gaaaaaatgg tgttgccgcg ccatctgcca 4200
   ccagccagct atcaactcgc gccctggaag ggatttttga agcaactcat cgattgattt 4260
   acggcgctaa ggatgactct ggtcagagat acctggcctg gtctggacac agtgcccgtg 4320
   tcggagccgc gcgagatatg gcccgcgctg gagtttcaat accggagatc atgcaagctg 4380
   gtggctggac caatgtaaat attgtcatga actatatccg taacctggat agtgaaacag 4440
   gggcaatggt gcgcctgctg gaagatggcg attctgctgg agacatgaga gctgccaacc 4500
   tttggccaag cccgctcatg atcaaacgct ctaagaagaa cagcctggcc ttgtccctga 4560
   cggccgacca gatggtcagt gccttgttgg atgctgagcc ccccatactc tattccgagt 4620
   atgatcctac cagacccttc agtgaagctt cgatgatggg cttactgacc aacctggcag 4680
   acagggagct ggttcacatg atcaactggg cgaagagggt gccaggcttt gtggatttga 4740
   ccctccatga tcaggtccac cttctagaat gtgcctggct agagatcctg atgattggtc 4800
   tcgtctggcg ctccatggag cacccagtga agctactgtt tgctcctaac ttgctcttgg 4860
   acaggaacca gggaaaatgt gtagagggca tggtggagat cttcgacatg ctgctggcta 4920
   catcatctcg gttccgcatg atgaatctgc agggagagga gtttgtgtgc ctcaaatcta 4980
   ttattttgct taattctgga gtgtacacat ttctgtccag caccctgaag tctctggaag 5040
   agaaggacca tatccaccga gtcctggaca agatcacaga cactttgatc cacctgatgg 5100
   ccaaggcagg cctgaccctg cagcagcagc accagcggct ggcccagctc ctcctcatcc 5160
   tctcccacat caggcacatg agtaacaaag gcatggagca tctgtacagc atgaagtgca 5220
   agaacgtggt gcccctctat gacctgctgc tggaggcggc cgacgcccac cgcctacatg 5280
   cgcccactag ccgtggaggg gcatccgtgg aggagacgga ccaaagccac ttggccactg 5340
   cgggctctac ttcatcgcat tccttgcaaa agtattacat cacgggggag gcagagggtt 5400
   tccctgccac agtctgagcg gccgaccggt tcgagatcca ggcgcggatc aataaaagat 5460
   cattattttc aatagatctg tgtgttggtt ttttgtgtgc cttgggggag gggaggccca 5520
   gaatgaggcg cggccaaggg ggagggggag gccagaatga ccttggggga gggggaggcc 5580
   agaatgacct tgggggaggg ggaggccaga atgaggcgcg ccggtaaccg aagttcctat 5640
   actttctaga gaataggaac ttcggaatag gaacttctta ggtcaattct accgggtagg 5700
   ggaggcgctt ttcccaaggc agtctggagc atgcgcttta gcagccccgc tgggcacttg 5760
   gcgctacaca agtggcctct ggcctcgcac acattccaca tccaccggta ggcgccaacc 5820
   ggctccgttc tttggtggcc ccttcgcgcc accttctact cctcccctag tcaggaagtt 5880
   cccccccgcc ccgcagctcg cgtcgtgcag gacgtgacaa atggaagtag cacgtctcac 5940
   tagtctcgtg cagatggaca gcaccgctga gcaatggaag cgggtaggcc tttggggcag 6000
   cggccaatag cagctttgct ccttcgcttt ctgggctcag aggctgggaa ggggtgggtc 6060
   cgggggcggg ctcaggggcg ggctcagggg cggggcgggc gcccgaaggt cctccggagg 6120
   cccggcattc tgcacgcttc aaaagcgcac gtctgccgcg ctgttctcct cttcctcatc 6180
   tccgggcctt tcgacctgca gccaatatgg gatcggccat tgaacaagat ggattgcacg 6240
   caggttctcc ggccgcttgg gtggagaggc tattcggcta tgactgggca caacagacaa 6300
   tcggctgctc tgatgccgcc gtgttccggc tgtcagcgca ggggcgcccg gttctttttg 6360
   tcaagaccga cctgtccggt gccctgaatg aactgcagga cgaggcagcg cggctatcgt 6420
   ggctggccac gacgggcgtt ccttgcgcag ctgtgctcga cgttgtcact gaagcgggaa 6480
   gggactggct gctattgggc gaagtgccgg ggcaggatct cctgtcatct caccttgctc 6540
   ctgccgagaa agtatccatc atggctgatg caatgcggcg gctgcatacg cttgatccgg 6600
   ctacctgccc attcgaccac caagcgaaac atcgcatcga gcgagcacgt actcggatgg 6660
   aagccggtct tgtcgatcag gatgatctgg acgaagagca tcaggggctc gcgccagccg 6720
   aactgttcgc caggctcaag gcgcgcatgc ccgacggcga ggatctcgtc gtgacccatg 6780
   gcgatgcctg cttgccgaat atcatggtgg aaaatggccg cttttctgga ttcatcgact 6840
   gtggccggct gggtgtggcg gaccgctatc aggacatagc gttggctacc cgtgatattg 6900
   ctgaagagct tggcggcgaa tgggctgacc gcttcctcgt gctttacggt atcgccgctc 6960
   ccgattcgca gcgcatcgcc ttctatcgcc ttcttgacga gttcttctga ggggatcgat 7020
   ccgctgtaag tctgcagaaa ttgatgatct attaaacaat aaagatgtcc actaaaatgg 7080
   aagtttttcc tgtcatactt tgttaagaag ggtgagaaca gagtacctac attttgaatg 7140
   gaaggattgg agctacgggg gtgggggtgg ggtgggatta gataaatgcc tgctctttac 7200
   tgaaggctct ttactattgc tttatgataa tgtttcatag ttggatatca taatttaaac 7260
   aagcaaaacc aaattaaggg ccagctcatt cctcccactc atgatctata gatctataga 7320
   tctctcgtgg gatcattgtt tttctcttga ttcccacttt gtggttctaa gtactgtggt 7380
   ttccaaatgt gtcagtttca tagcctgaag aacgagatca gcagcctctg ttccacatac 7440
   acttcattct cagtattgtt ttgccaagtt ctaattccat cagaagctga ctctagatcc 7500
   cgcgccgaag ttcctatact ttctagagaa taggaacttc ggaataggaa cttcaagctt 7560
   aagcgctaga agatgggcgg gagtcttctg ggcaggctta aaggctaacc tggtgtgtgg 7620
   gcgttgtcct gcaggggaat tgaacaggtg taaaattgga gggacaagac ttcccacaga 7680
   ttttcggttt tgtcgggaag ttttttaata ggggcaaata aggaaaatgg gaggataggt 7740
   agtcatctgg ggttttatgc agcaaaacta caggttatta ttgcttgtga tccgcctcgg 7800
   agtattttcc atcgaggtag attaaagaca tgctcacccg agttttatac tctcctgctt 7860
   gagatcctta ctacagtatg aaattacagt gtcgcgagtt agactatgta agcagaattt 7920
   taatcatttt taaagagccc agtacttcat atccatttct cccgctcctt ctgcagcctt 7980
   atcaaaaggt attttagaac actcatttta gccccatttt catttattat actggcttat 8040
   ccaaccccta gacagagcat tggcattttc cctttcctga tcttagaagt ctgatgactc 8100
   atgaaaccag acagattagt tacatacacc acaaatcgag gctgtagctg gggcctcaac 8160
   actgcagttc ttttataact ccttagtaca ctttttgttg atcctttgcc ttgatcctta 8220
   attttcagtg tctatcacct ctcccgtcag tggtgttcca catttgggcc tattctcagt 8280
   ccagggagtt ttacaacaat agatgtattg agaatccaac ctaaagctta actttccact 8340
   cccatgaatg cctctctcct ttttctccat ttataaactg agctattaac cattaatggt 8400
   tccaggtgga tgtctcctcc ccatattacc tgatgtatct tacatattgc caggctgata 8460
   ttttaagaca ttaaaaggta tatttcatta ttgagccaca tggtattgat tactgcttac 8520
   taaaattttg tcattgtaca catctgtaaa aggtggttcc ttttggaatg caaagttcag 8580
   gtgtttgttg tctttcctga cctaaggtct tgtgagcttg tattttttct atttaagcag 8640
   tgctttctct tggactggct tgactcatgg cattctacac gttattgctg gtctaaatgt 8700
   gattttgcca agcttcttca ggacctataa ttttgcttga cttgtagcca aacacaagta 8760
   aaatgattaa gcaacaaatg tatttgtgaa gcttggtttt taggttgttg tgttgtgtgt 8820
   gcttgtgctc tataataata ctatccaggg gctggagagg tggctcggag ttcaagagca 8880
   cagactgctc ttccagaagt cctgagttca attcccagca accacatggt ggctcacaac 8940
   catctgtaat gggatctgat gccctcttct ggtgtgtctg aagaccacaa gtgtattcac 9000
   attaaataaa taaatcctcc ttcttcttct tttttttttt tttaaagaga atactgtctc 9060
   cagtagaatt tactgaagta atgaaatact ttgtgtttgt tccaatatgg tagccaataa 9120
   tcaaattact ctttaagcac tggaaatgtt accaaggaac taatttttat ttgaagtgta 9180
   actgtggaca gaggagccat aactgcagac ttgtgggata cagaagacca atgcagactt 9240
   taatgtcttt tctcttacac taagcaataa agaaataaaa attgaacttc tagtatccta 9300
   tttgtttaaa ctgctagctt tacttaactt ttgtgcttca tctatacaaa gctgaaagct 9360
   aagtctgcag ccattactaa acatgaaagc aagtaatgat aattttggat ttcaaaaatg 9420
   tagggccaga gtttagccag ccagtggtgg tgcttgcctt tatgccttta atcccagcac 9480
   tctggaggca gagacaggca gatctctgag tttgagccca gcctggtcta cacatcaagt 9540
   tctatctagg atagccagga atacacacag aaaccctgtt ggggaggggg gctctgagat 9600
   ttcataaaat tataattgaa gcattcccta atgagccact atggatgtgg ctaaatccgt 9660
   ctacctttct gatgagattt gggtattatt ttttctgtct ctgctgttgg ttgggtcttt 9720
   tgacactgtg ggctttcttt aaagcctcct tcctgccatg tggtctcttg tttgctacta 9780
   acttcccatg gcttaaatgg catggctttt tgccttctaa gggcagctgc tgagatttgc 9840
   agcctgattt ccagggtggg gttgggaaat ctttcaaaca ctaaaattgt cctttaattt 9900
   tttttttaaa aaatggttta tataataaac ctcataaaat agttatgagg agtgaggtgg 9960
   actaatatta aatgagtccc tcccctataa aagagctatt aaggcttttt gtcttatact 10020
   taactttttt tttaaatgtg gtatctttag aaccaagggt cttagagttt tagtatacag 10080
   aaactgttgc atcgcttaat cagattttct agtttcaaat ccagagaatc caaattcttc 10140
   acagccaaag tcaaattaag aatttctgac ttttaatgtt aatttgctta ctgtgaatat 10200
   aaaaatgata gcttttcctg aggcagggtc tcactatgta tctctgcctg atctgcaaca 10260
   agatatgtag actaaagttc tgcctgcttt tgtctcctga atactaaggt taaaatgtag 10320
   taatactttt ggaacttgca ggtcagattc ttttataggg gacacactaa gggagcttgg 10380
   gtgatagttg gtaaaatgtg tttcaagtga tgaaaacttg aattattatc accgcaacct 10440
   actttttaaa aaaaaaagcc aggcctgtta gagcatgctt aagggatccc taggacttgc 10500
   tgagcacaca agagtagtta cttggcaggc tcctggtgag agcatatttc aaaaaacaag 10560
   gcagacaacc aagaaactac agttaaggtt acctgtcttt aaaccatctg catatacaca 10620
   gggatattaa aatattccaa ataatatttc attcaagttt tcccccatca aattgggaca 10680
   tggatttctc cggtgaatag gcagagttgg aaactaaaca aatgttggtt ttgtgatttg 10740
   tgaaattgtt ttcaagtgat agttaaagcc catgagatac agaacaaagc tgctatttcg 10800
   aggtctcttg gtttatactc agaagcactt ctttgggttt ccctgcacta tcctgatcat 10860
   gtgctaggcc taccttaggc tgattgttgt tcaaataaac ttaagtttcc tgtcaggtga 10920
   tgtcatatga tttcatatat caaggcaaaa catgttatat atgttaaaca tttgtactta 10980
   atgtgaaagt taggtctttg tgggtttgat ttttaatttt caaaacctga gctaaataag 11040
   tcatttttac atgtcttaca tttggtggaa ttgtataatt gtggtttgca ggcaagactc 11100
   tctgacctag taaccctacc tatagagcac tttgctgggt cacaagtcta ggagtcaagc 11160
   atttcacctt gaagttgaga cgttttgtta gtgtatacta gtttatatgt tggaggacat 11220
   gtttatccag aagatattca ggactatttt tgactgggct aaggaattga ttctgattag 11280
   cactgttagt gagcattgag tggcctttag gcttgaattg gagtcacttg tatatctcaa 11340
   ataatgctgg ccttttttaa aaagcccttg ttctttatca ccctgttttc tacataattt 11400
   ttgttcaaag aaatacttgt ttggatctcc ttttgacaac aatagcatgt tttcaagcca 11460
   tatttttttt cctttttttt tttttttttg gtttttcgag acagggtttc tctgtatagc 11520
   cctggctgtc ctggaactca ctttgtagac caggctggcc tcgaactcag aaatccgcct 11580
   gcctctgcct cctgagtgcc gggattaaag gcgtgcacca ccacgcctgg ctaagttgga 11640
   tattttgtta tataactata accaatacta actccactgg gtggattttt aattcagtca 11700
   gtagtcttaa gtggtcttta ttggcccttc attaaaatct actgttcact ctaacagagg 11760
   ctgttggtac tagtggcact taagcaactt cctacggata tactagcaga ttaagggtca 11820
   gggatagaaa ctagtctagc gttttgtata cctaccagct ttatactacc ttgttctgat 11880
   agaaatattt caggacatct agcacccaat tcgccctata gtgagtcgta ttacaattca 11940
   ctggccgtcg ttttacaacg tcgtgactgg gaaaaccctg gcgttaccca acttaatcgc 12000
   cttgcagcac atcccccttt cgccagctgg cgtaatagcg aagaggcccg caccgatcgc 12060
   ccttcccaac agttgcgcag cctgaatggc gaatggacgc cgccctgtag cggcgcatta 12120
   agcgcggcgg gtgtggtggt tacgcgcagc gtgaccgcta cacttgccag cgccctagcg 12180
   cccgctcctt tcgctttctt cccttccttt ctcgccacgt tcgccggctt tccccgtcaa 12240
   gctctaaatc gggggctccc tttagggttc cgatttagtg ctttacggca cctcgacccc 12300
   aaaaaacttg attagggtga tggttcacgt agtgggccat cgccctgata gacggttttt 12360
   cgccctttga cgttggagtc cacgttcttt aatagtggac tcttgttcca aactggaaca 12420
   acactcaacc ctatctcggt ctattctttt gatttataag ggattttgcc gatttcggcc 12480
   tattggttaa aaaatgagct gatttaacaa aaatttaacg cgaattttaa caaaatatta 12540
   acgcttacaa tttaggtggc acttttcggg gaaatgtgcg cggaacccct atttgtttat 12600
   ttttctaaat acattcaaat atgtatccgc tcatgagaca ataaccctga taaatgcttc 12660
   aataatattg aaaaaggaag agtatgagta ttcaacattt ccgtgtcgcc cttattccct 12720
   tttttgcggc attttgcctt cctgtttttg ctcacccaga aacgctggtg aaagtaaaag 12780
   atgctgaaga tcagttgggt gcacgagtgg gttacatcga actggatctc aacagcggta 12840
   agatccttga gagttttcgc cccgaagaac gttttccaat gatgagcact tttaaagttc 12900
   tgctatgtgg cgcggtatta tcccgtattg acgccgggca agagcaactc ggtcgccgca 12960
   tacactattc tcagaatgac ttggttgagt actcaccagt cacagaaaag catcttacgg 13020
   atggcatgac agtaagagaa ttatgcagtg ctgccataac catgagtgat aacactgcgg 13080
   ccaacttact tctgacaacg atcggaggac cgaaggagct aaccgctttt ttgcacaaca 13140
   tgggggatca tgtaactcgc cttgatcgtt gggaaccgga gctgaatgaa gccataccaa 13200
   acgacgagcg tgacaccacg atgcctgtag caatggcaac aacgttgcgc aaactattaa 13260
   ctggcgaact acttactcta gcttcccggc aacaattaat agactggatg gaggcggata 13320
   aagttgcagg accacttctg cgctcggccc ttccggctgg ctggtttatt gctgataaat 13380
   ctggagccgg tgagcgtggg tctcgcggta tcattgcagc actggggcca gatggtaagc 13440
   cctcccgtat cgtagttatc tacacgacgg ggagtcaggc aactatggat gaacgaaata 13500
   gacagatcgc tgagataggt gcctcactga ttaagcattg gtaactgtca gaccaagttt 13560
   actcatatat actttagatt gatttaaaac ttcattttta atttaaaagg atctaggtga 13620
   agatcctttt tgataatctc atgaccaaaa tcccttaacg tgagttttcg ttccactgag 13680
   cgtcagaccc cgtagaaaag atcaaaggat cttcttgaga tccttttttt ctgcgcgtaa 13740
   tctgctgctt gcaaacaaaa aaaccaccgc taccagcggt ggtttgtttg ccggatcaag 13800
   agctaccaac tctttttccg aaggtaactg gcttcagcag agcgcagata ccaaatactg 13860
   tccttctagt gtagccgtag ttaggccacc acttcaagaa ctctgtagca ccgcctacat 13920
   acctcgctct gctaatcctg ttaccagtgg ctgctgccag tggcgataag tcgtgtctta 13980
   ccgggttgga ctcaagacga tagttaccgg ataaggcgca gcggtcgggc tgaacggggg 14040
   gttcgtgcac acagcccagc ttggagcgaa cgacctacac cgaactgaga tacctacagc 14100
   gtgagctatg agaaagcgcc acgcttcccg aagggagaaa ggcggacagg tatccggtaa 14160
   gcggcagggt cggaacagga gagcgcacga gggagcttcc agggggaaac gcctggtatc 14220
   tttatagtcc tgtcgggttt cgccacctct gacttgagcg tcgatttttg tgatgctcgt 14280
   caggggggcg gagcctatgg aaaaacgcca gcaacgcggc ctttttacgg ttcctggcct 14340
   tttgctggcc ttttgctcac atgttctttc ctgcgttatc ccctgattct gtggataacc 14400
   gtattaccgc c 14411

<210> 8
<211> 18866
<212> DNA
<213> Artificial Sequence

<220>

<223> Description of Artificial Sequence: FABP-Cre
      targeting vector

<400> 8
tcgagacatt cctgcgcatc gtgcagtgcc gctctgtgga gggcagctgt ggcttctagc 60
tgcccgggtg gcatccctgt gacccctccc cagtgcctct cctggccctg gaagttgcca 120
ctccagtgcc caccagcctt gtcctaataa aattaagttg catcattttg tctgactagg 180
tgtccttcta taatattatg gggtggaggg gggtggtatg gagcaagggg caagttggga 240
agacaacctg tagggcctgc ggggtctatt gggaaccaag ctggagtgca gtggcacaat 300
cttggctcac tgcaatctcc gcctcctggg ttcaagcgat tctcctgcct cagcctcccg 360
agttgttggg attccaggca tgcatgacca ggctcagcta atttttgttt ttttggtaga 420
gacggggttt caccatattg gccaggctgg tctccaactc ctaatctcag gtgatctacc 480
caccttggcc tcccaaattg ctgggattac aggcgtgaac cactgctccc ttccctgtcc 540
ttctgatttt aaaataacta taccagcagg aggacgtcca gacacagcat aggctacctg 600
gccatgccca accggtggga catttgagtt gcttgcttgg cactgtcctc tcatgcgttg 660
ggtccactca gtagatgcct gttgaattaa gcttgatatc gaattctcag aatacaaaac 720
agctttaggg actgataaaa tatatgtaaa attatgtttg tataataata gaaaaattga 780
gaaataaaca taaggctacg tgaaaaggtt ttgagttcaa aggtattttt ctccacgtaa 840
tgcacatata catgtgtgtt tatatatgtg catatgtata tgtagctaca tgtgtataaa 900
tatattacat atacaagtgt gcacatgtat aaacacatac atatgtacac ataggtatat 960
atgcatgtat gcattgctag agatgtgatt cacatgtctg ttgcacatat atacatctgt 1020
caacatacat ttcatgtatg cactcttatt tcatgagcgg tgataagaca ccaaaaatgc 1080
accatttaca gagagctttg cccttgattg gactcactaa tgtttgctga attagaacaa 1140
acttctgcct tgcccattct gatttaaatt agaacaaact tctgccttgc ccattctgat 1200
ttttatcgtt agaacaaact tctgccttgc ccattctgat ttttatcgtt agaacaaact 1260
tctgccttgc ccattctgat ttttatcgtt agaacaaact tctgccttgc ccattctgat 1320
tttgattttt atcgttgacc attgctctca ggagttaatg tttgatcctg gccataaaga 1380
aatcgacaat cactgaccta tggcctatat ttgaggagga agaagcccct tataaaatag 1440
ccaacagtgg gtggcctggc agacagagct gttgtggtca gggggatcaa ataggccggc 1500
ctaggcgcgt aagcgttggg gtgagtactc cctctcaaaa gcgggcatga cttctgcgct 1560
aagattgtca gtttccaaaa acgaggagga tttgatattc acctggcccg cggtgatgcc 1620
tttgagggtg gccgcgtcca tctggtcaga aaagacaatc tttttgttgt caagcttgag 1680
gtgtggcagg cttgagatct ggccatacac ttgagtgaca ttgacatcca ctttgccttt 1740
ctctccacag gtgtccactc ccagggcggc ctcgaccatg cccaagaaga agaggaaggt 1800
gtccaattta ctgaccgtac accaaaattt gcctgcatta ccggtcgatg caacgagtga 1860
tgaggttcgc aagaacctga tggacatgtt cagggatcgc caggcgtttt ctgagcatac 1920
ctggaaaatg cttctgtccg tttgccggtc gtgggcggca tggtgcaagt tgaataaccg 1980
gaaatggttt cccgcagaac ctgaagatgt tcgcgattat cttctatatc ttcaggcgcg 2040
cggtctggca gtaaaaacta tccagcaaca tttgggccag ctaaacatgc ttcatcgtcg 2100
gtccgggctg ccacgaccaa gtgacagcaa tgctgtttca ctggttatgc ggcggatccg 2160
aaaagaaaac gttgatgccg gtgaacgtgc aaaacaggct ctagcgttcg aacgcactga 2220
tttcgaccag gttcgttcac tcatggaaaa tagcgatcgc tgccaggata tacgtaatct 2280
ggcatttctg gggattgctt ataacaccct gttacgtata gccgaaattg ccaggatcag 2340
ggttaaagat atctcacgta ctgacggtgg gagaatgtta atccatattg gcagaacgaa 2400
aacgctggtt agcaccgcag gtgtagagaa ggcacttagc ctgggggtaa ctaaactggt 2460
cgagcgatgg atttccgtct ctggtgtagc tgatgatccg aataactacc tgttttgccg 2520
ggtcagaaaa aatggtgttg ccgcgccatc tgccaccagc cagctatcaa ctcgcgccct 2580
ggaagggatt tttgaagcaa ctcatcgatt gatttacggc gctaaggatg actctggtca 2640
gagatacctg gcctggtctg gacacagtgc ccgtgtcgga gccgcgcgag atatggcccg 2700
cgctggagtt tcaataccgg agatcatgca agctggtggc tggaccaatg taaatattgt 2760
catgaactat atccgtaacc tggatagtga aacaggggca atggtgcgcc tgctggaaga 2820
tggcgattag ccattaacgc gtaaatgatt gcagatccac tagttctagg gccgcgtcga 2880
cctcgagatc caggcgcgga tcaataaaag atcattattt tcaatagatc tgtgtgttgg 2940
ttttttgtgt gccttggggg aggggaggc cagaatgagg cgcggccaag gggagggggg 3000
aggccagaat gaccttgggg gagggggagg ccagaatgac cttgggggag ggggaggcca 3060
gaatgaggcg cgccggtaac cgaagttcct atactttcta gagaatagga acttcggaat 3120
aggaacttct taggtcaatt ctaccgggta ggggaggcgc ttttcccaag gcagtctgga 3180
gcatgcgctt tagcagcccc gctgggcact tggcgctaca caagtggcct ctggcctcgc 3240
acacattcca catccaccgg taggcgccaa ccggctccgt tctttggtgg ccccttcgcg 3300
ccaccttcta ctcctcccct agtcaggaag ttcccccccg ccccgcagct cgcgtcgtgc 3360
aggacgtgac aaatggaagt agcacgtctc actagtctcg tgcagatgga cagcaccgct 3420
gagcaatgga agcgggtagg cctttggggc agcggccaat agcagctttg ctccttcgct 3480
ttctgggctc agaggctggg aaggggtggg tccgggggcg ggctcagggg cgggctcagg 3540

ggcggggcgg gcgcccgaag gtcctccgga ggcccggcat tctgcacgct tcaaaagcgc 3600
acgtctgccg cgctgttctc ctcttcctca tctccgggcc tttcgacctg cagccaatat 3660
gggatcggcc attgaacaag atggattgca cgcaggttct ccggccgctt gggtggagag 3720
gctattcggc tatgactggg cacaacagac aatcggctgc tctgatgccg ccgtgttccg 3780
gctgtcagcg caggggcgcc cggttctttt tgtcaagacc gacctgtccg gtgccctgaa 3840
tgaactgcag gacgaggcag cgcggctatc gtggctggcc acgacgggcg ttccttgcgc 3900
agctgtgctc gacgttgtca ctgaagcggg aagggactgg ctgctattgg gcgaagtgcc 3960
ggggcaggat ctcctgtcat ctcaccttgc tcctgccgag aaagtatcca tcatggctga 4020
tgcaatgcgg cggctgcata cgcttgatcc ggctacctgc ccattcgacc accaagcgaa 4080
acatcgcatc gagcgagcac gtactcggat ggaagccggt cttgtcgatc aggatgatct 4140
ggacgaagag catcaggggc tcgcgccagc cgaactgttc gccaggctca aggcgcgcat 4200
gcccgacggc gaggatctcg tcgtgaccca tggcgatgcc tgcttgccga atatcatggt 4260
ggaaaatggc cgcttttctg gattcatcga ctgtggccgg ctgggtgtgg cggaccgcta 4320
tcaggacata gcgttggcta cccgtgatat tgctgaagag cttggcggcg aatgggctga 4380
ccgcttcctc gtgctttacg gtatcgccgc tcccgattcg cagcgcatcg ccttctatcg 4440
ccttcttgac gagttcttct gaggggatcg atccgctgta agtctgcaga aattgatgat 4500
ctattaaaca ataaagatgt ccactaaaat ggaagttttt cctgtcatac tttgttaaga 4560
agggtgagaa cagagtacct acattttgaa tggaaggatt ggagctacgg gggtgggggt 4620
ggggtgggat tagataaatg cctgctcttt actgaaggct ctttactatt gctttatgat 4680
aatgtttcat agttggatat cataatttaa acaagcaaaa ccaaattaag ggccagctca 4740
ttcctcccac tcatgatcta tagatctata gatctctcgt gggatcattg tttttctctt 4800
gattcccact ttgtggttct aagtactgtg gtttccaaat gtgtcagttt catagcctga 4860
agaacgagat cagcagcctc tgttccacat acacttcatt ctcagtattg ttttgccaag 4920
ttctaattcc atcagaagct gactctagat cccgcgccga agttcctata ctttctagag 4980
aataggaact tcggaatagg aacttcaagc ttaagcgcta gaagatgggc gggagtcttc 5040
tgggcaggct taaaggctaa cctggtgtgt gggcgttgtc ctgcagggga attgaacagg 5100
tgtaaaattg gagggacaag acttcccaca gattttcggt tttgtcggga agttttttaa 5160
taggggcaaa taaggaaaat gggaggatag gtagtcatct ggggttttat gcagcaaaac 5220
tacaggttat tattgcttgt gatccgcctc ggagtatttt ccatcgaggt agattaaaga 5280
catgctcacc cgagttttat actctcctgc ttgagatcct tactacagta tgaaattaca 5340
gtgtcgcgag ttagactatg taagcagaat tttaatcatt tttaaagagc ccagtacttc 5400
atatccattt ctcccgctcc ttctgcagcc ttatcaaaag gtattttaga acactcattt 5460
tagccccatt ttcatttatt atactggctt atccaacccc tagacagagc attggcattt 5520
tccctttcct gatcttagaa gtctgatgac tcatgaaacc agacagatta gttacataca 5580
ccacaaatcg aggctgtagc tggggcctca acactgcagt tcttttataa ctccttagta 5640
cactttttgt tgatcctttg ccttgatcct taattttcag tgtctatcac ctctcccgtc 5700
agtggtgttc cacatttggg cctattctca gtccagggag ttttacaaca atagatgtat 5760
tgagaatcca acctaaagct taactttcca ctcccatgaa tgcctctctc ctttttctcc 5820
atttataaac tgagctatta accattaatg gttccaggtg gatgtctcct ccccatatta 5880
cctgatgtat cttacatatt gccaggctga tattttaaga cattaaaagg tatatttcat 5940
tattgagcca catggtattg attactgctt actaaaattt tgtcattgta cacatctgta 6000
aaaggtggtt ccttttggaa tgcaaagttc aggtgtttgt tgtctttcct gacctaaggt 6060
cttgtgagct tgtatttttt ctatttaagc agtgctttct cttggactgg cttgactcat 6120
ggcattctac acgttattgc tggtctaaat gtgattttgc caagcttctt caggacctat 6180
aattttgctt gacttgtagc caaacacaag taaaatgatt aagcaacaaa tgtatttgtg 6240
aagcttggtt tttaggttgt tgtgttgtgt gtgcttgtgc tctataataa tactatccag 6300
gggctggaga ggtggctcgg agttcaagag cacagactgc tcttccagaa gtcctgagtt 6360
caattcccag caaccacatg gtggctcaca accatctgta atgggatctg atgccctctt 6420
ctggtgtgtc tgaagaccac aagtgtattc acattaaata aataaatcct ccttcttctt 6480
cttttttttt tttttaaaga gaatactgtc tccagtagaa tttactgaag taatgaaata 6540
ctttgtgttt gttccaatat ggtagccaat aatcaaatta ctctttaagc actggaaatg 6600
ttaccaagga actaattttt atttgaagtg taactgtgga cagaggagcc ataactgcag 6660
acttgtggga tacagaagac caatgcagac tttaatgtct tttctcttac actaagcaat 6720
aaagaaataa aaattgaact tctagtatcc tatttgttta aactgctagc tttacttaac 6780
ttttgtgctt catctataca aagctgaaag ctaagtctgc agccattact aaacatgaaa 6840
gcaagtaatg ataattttgg atttcaaaaa tgtagggcca gagtttagcc agccagtggt 6900
ggtgcttgcc tttatgcctt taatcccagc actctggagg cagagacagg cagatctctg 6960
agtttgagcc cagcctggtc tacacatcaa gttctatcta ggatagccag gaatacacac 7020
agaaaccctg ttggggaggg gggctctgag atttcataaa attataattg aagcattccc 7080
taatgagcca ctatggatgt ggctaaatcc gtctaccttt ctgatgagat ttgggtatta 7140
ttttttctgt ctctgctgtt ggttgggtct tttgacactg tgggctttct ttaaagcctc 7200
cttcctgcca tgtggtctct tgtttgctac taacttccca tggcttaaat ggcatggctt 7260
tttgccttct aagggcagct gctgagattt gcagcctgat ttccagggtg gggttgggaa 7320

atctttcaaa cactaaaatt gtcctttaat ttttttttta aaaaatgggt tatataataa 7380
acctcataaa atagttatga ggagtgaggt ggactaatat taaatgagtc cctcccctat 7440
aaaagagcta ttaaggcttt ttgtcttata cttaactttt tttttaaatg tggtatcttt 7500
agaaccaagg gtcttagagt tttagtatac agaaactgtt gcatcgctta atcagatttt 7560
ctagtttcaa atccagagaa tccaaattct tcacagccaa agtcaaatta agaatttctg 7620
acttttaatg ttaatttgct tactgtgaat ataaaaatga tagcttttcc tgaggcaggg 7680
tctcactatg tatctctgcc tgatctgcaa caagatatgt agactaaagt tctgcctgct 7740
tttgtctcct gaatactaag gttaaaatgt agtaatactt ttggaacttg caggtcagat 7800
tcttttatag gggacacact aagggagctt gggtgatagt tggtaaaatg tgtttcaagt 7860
gatgaaaact tgaattatta tcaccgcaac ctacttttta aaaaaaaaag ccaggcctgt 7920
tagagcatgc ttaagggatc cctaggactt gctgagcaca caagagtagt tacttggcag 7980
gctcctggtg agagcatatt tcaaaaaaca aggcagacaa ccaagaaact acagttaagg 8040
ttacctgtct ttaaaccatc tgcatataca caggatattt aaaatattcc aaataatatt 8100
tcattcaagt tttcccccat caaattggga catggatttc tccggtgaat aggcagagtt 8160
ggaaactaaa caaatgttgg ttttgtgatt tgtgaaattg ttttcaagtg atagttaaag 8220
cccatgagat acagaacaaa gctgctattt cgaggtctct tggtttatac tcagaagcac 8280
ttctttgggt ttccctgcac tatcctgatc atgtgctagg cctaccttag gctgattgtt 8340
gttcaaataa acttaagttt cctgtcaggt gatgtcatat gatttcatat atcaaggcaa 8400
aacatgttat atatgttaaa catttgtact taatgtgaaa gttaggtctt tgtgggtttg 8460
atttttaatt ttcaaaacct gagctaaata agtcattttt acatgtctta catttggtgg 8520
aattgtataa ttgtggtttg caggcaagac tctctgacct agtaacccta cctatagagc 8580
actttgctgg gtcacaagtc taggagtcaa gcatttcacc ttgaagttga gacgttttgt 8640
tagtgtatac tagtttatat gttggaggac atgtttatcc agaagatatt caggactatt 8700
tttgactggg ctaaggaatt gattctgatt agcactgtta gtgagcattg agtggccttt 8760
aggcttgaat tggagtcact tgtatatctc aaataatgct ggcctttttt aaaaagccct 8820
tgttctttat caccctgttt tctacataat ttttgttcaa agaaatactt gtttggatct 8880
ccttttgaca acaatagcat gttttcaagc catatttttt ttcctttttt tttttttttt 8940
tggtttttcg agacagggtt tctctgtata gccctggctg tcctggaact cactttgtag 9000
accaggctgg cctcgaactc agaaatccgc ctgcctctgc ctcctgagtg ccgggattaa 9060
aggcgtgcac caccacgcct ggctaagttg gatattttgt tatataacta taaccaatac 9120
taactccact gggtggattt ttaattcagt cagtagtctt aagtggtctt tattggccct 9180
tcattaaaat ctactgttca ctctaacaga ggctgttggt actagtggca cttaagcaac 9240
ttcctacgga tatactagca gattaagggt caggatagaa aactagtcta gcgttttgta 9300
tacctaccag ctttatacta ccttgttctg atagaaatat ttcaggacat ctagagtgta 9360
ctataaggtt gatggtaagc ttataaggaa cttgaaagtg gagtaactac tccatttctc 9420
tgaggggaga attaaaattt ttgaccaagt gttgttgagc cactgagaat ggtctcagaa 9480
cataacttct taaggaacct tcccagattg ccctcaacac tgcaccacat ttggtcctgc 9540
ttgaacattg ccatggctct taaagtctta attaagaata ttaattgtgt aattattgtt 9600
tttcctcctt tagatcattc cttgaggaca ggacagtgct tgtttaaggc tatatttctg 9660
ctgtctgagc agcaacaggt cttcgagatc aacatgatgt tcataatccc aagatgttgc 9720
catttatgtt ctcagaagca agcagaggca tgatggtcag tgacagtaat gtcactgtgt 9780
taaatgttgc tatgcagttt ggatttttct aatgtagtgt aggtagaaca tatgtgttct 9840
gtatgaatta aactcttaag ttacaccttg tataatccat gcaatgtgtt atgcaattac 9900
cattttaagt attgtagctt tctttgtatg tgaggataaa ggtgtttgtc ataaaatgtt 9960
ttgaacattt ccccaaagtt ccaaattata aaaccacaac gttagaactt atttatgaac 10020
aatggttgta gtttcatgct tttaaaatgc ttaattattc aattaacacc gtttgtgtta 10080
taatatatat aaaactgaca tgtagaagtg tttgtccaga acatttctta aatgtatact 10140
gtctttagag agtttaatat agcatgtctt ttgcaacata ctaacttttg tgttggtgcg 10200
agcaatattg tgtagtcatt ttgaaaggag tcatttcaat gagtgtcaga ttgttttgaa 10260
tgttattgaa cattttaaat gcagacttgt tcgtgtttta gaaagcaaaa ctgtcagaag 10320
ctttgaacta gaaattaaaa agctgaagta tttcagaagg gaaataagct acttgctgta 10380
ttagttgaag gaaagtgtaa tagcttagaa aatttaaaac catatagttg tcattgctga 10440
atatctggca gatgaaaaga aatactcagt ggttcttttg agcaatataa cagcttgtta 10500
tattaaaaat tttccccaca gatataaact ctaatctata actcataaat gttacaaatg 10560
gatgaagctt acaaatgtgg cttgacttgt cactgtgctt gttttagtta tgtgaaagtt 10620
tggcaataaa cctatgtcct aaatagtcaa actgtggaat gactttttaa tctattggtt 10680
tgtctagaac agttatgttg ccatttgccc taatggtgaa agaaaaagtg gggagtgcct 10740
tggcactgtt catttgtggt gtgaaccaaa gaggggggca tgcacttaca cttcaaacat 10800
ccttttgaaa gactgacaag tttgggtctt cacagttgga attgggcatc ccttttgtca 10860
gggagggagg gagggaggga ggctggcttg ttatgctgac aagtgtgatt aaattcaaac 10920
tttgaggtaa gttggaggaa cttgtacatt gttaggagtg tgacaatttg gactcttaat 10980
gatttggtca tacaaaatga acctagacca acttctggaa gatgtatata ataactccat 11040
gttacattga tttcacctga ctaatactta tcccttatca attaaataca gaagatgcca 11100

gccatctggg ccttttaacc cagaaattta gtttcaaact cctaggttag tgttctcact 11160
gagctacatc ctgatctagt cctgaaaata ggaccaccat cacccccaaa aaaatctcaa 11220
ataagattta tgctagtgtt tcaaaatttt aggaataggt aagattagaa agttttaaat 11280
tttgagaaat ggcttctcta gaaagatgta catagtgaac actgaatggc tcctaaagag 11340
cctagaaaac tggtactgag cacacaggac tgagaggtct ttcttgaaaa gcatgtattg 11400
ctttacgtgg gtcacagaag gcaggcagga agaacttggg ctgaaactgg tgtcttaagt 11460
ggctaacatc ttcacaactg atgagcaaga actttatcct gatgcaaaaa ccatccaaac 11520
aaactaagtg aaaggtggca atggatccca ggctgctcta gaggaggact tgacttctca 11580
tcccatcacc cacaccagat agctcataga ctgccaatta acaccagctt ctagcctcca 11640
caggcacctg cactggtaca cataatttca cacaaacaca gtaagaagcc ttccacctgg 11700
catggtattg cttatcttta gttcccaaca cttgggaggc agaggccagc cagggctatg 11760
tgacaaaaac cttgtctaga ggagaaactt catagcttat ttcctattca cgtaaccagg 11820
ttagcaaaat ttaccagcca gagatgaagc taacagtgtc cactatattt gtagtgtttt 11880
aagtcaattt tttaaatata cttaatagaa ttaaagctat ggtgaaccaa gtacaaacct 11940
ggtgtattaa cttgagaact tagcataaaa agtagttcat ttgttcagta aatattaaat 12000
gcttactggc aaagattatg tcaggaactt ggtaaatggt gatgaaacaa tcatagttgt 12060
acatcttggt tctgtgatca ccttggtttg aggtaaaagt ggttcctttg atcaaggatg 12120
gaattttaag tttatattca atcaataatg tattattttg tgattgcaaa attgcctatc 12180
tagggtataa aacctttaaa aatttcataa taccagttca ttctccagtt actaattcca 12240
aaaagccact gactatggtg ccaatgtgga ttctgttctc aaaggaagga ttgtctgtgc 12300
cctttattct aatagaaaca tcacactgaa aatctaagct gaaagaagcc agactttcct 12360
aaataaataa ctttccataa agctcaaaca aggattactt ttaggaggca ctgttaagga 12420
actgataagt aatgaggtta cttatataat gatagtccca caagactatc tgaggaaaaa 12480
tcagtacaac tcgaaaacag aacaaccagc taggcaggaa taacagggct cccaagtcag 12540
gaggtctatc caacaccctt ttctgttgag ggccccagac ctacatattg tatacaaaca 12600
gggaggtggg tgattttaac tctcctgagg taccttggta aatctttgtc ctgagtaagc 12660
agtacagtgt acagtttaca ttttcattta aagatacatt agctccctct accccctaag 12720
actgacaggc actttggggg tggggagggc tttggaaaat aacgcttcca tacactaaaa 12780
gagaaatttc tttaattagg cttgttggtt ccatacatct actggtgttt ctactactta 12840
gtaatattat aatagtcaca caagcatctt tgctctgttt aggttgtata tttattttaa 12900
ggcagatgat aaaactgtag atcttaaggg atgcttctgc ttctgagatg atacaaagaa 12960
tttagaccat aaaacagtag gttgcacaag caatagaata tggcctaaag tgttctgaca 13020
cttagaagcc aagcagtgta ggcttcttaa gaaataccat tacaatcacc ttgctagaaa 13080
tcaagcattc tggagtggtc aagcagtgta acctgtactg taagttactt ttctgctatt 13140
tttctcccaa agcaagttct ttatgctgat atttccagtg ttaggaacta caaatattaa 13200
taagttgtct tcactctttt ctttaccaag gagggtctct tccttcatct tgatctgaag 13260
gatgaacaaa ggcttgagca gtgcgcttta gaagataaac tgcagcatga aggcccccga 13320
tgttcaccca gactacatgg acctttcgcc acacatgtcc cattccagat aaggcctggc 13380
acacacaaaa aacataagtc attaggctac cagtctgatt ctaaaacaac ctaaaatctt 13440
cccacttaaa tgctatgggt ggtgggttgg aaagttgact cagaaaatca cttgctgttt 13500
ttagagagga tctgggttca gtttctgata cattgtggct tacaactata actccagttc 13560
tagggggtcc atccaacatc ctcttctgtt gagggcacca aataaatgta ttgtgtacaa 13620
acagggaggt gagtgattta actctcgtgt atagtacctt ggtaaaacat ttcttgtcct 13680
gagtaagcag tacagctctg cctgtccctg gtctacagac acggctcatt tcccgaaggc 13740
aagctggata gagattccaa tttctcttct tggatcccat cctataaaag aaggtcaagt 13800
ttaatctatt gcaaaaggta aataggtagt ttcttacatg agacaagaac aaatcttagg 13860
tgtgaagcag tcatctttta caggccagag cctctattct atgccaatga aggaaactgt 13920
tagtccagtg ttatagagtt agtccagtgt atagttttct atcagaacac ttttttttta 13980
aacaactgca acttagctta ttgaagacaa accacgagta gaaatctgtc caagaagcaa 14040
gtgcttctca gcctacaatg tggaatagga ccatgtaatg gtacagtgag tgaaatgaat 14100
tatggcatgt ttttctgact gagaagacag tacaataaaa ggtaaactca tggtatttat 14160
ttaaaaagaa tccaatttct acctttttcc aaatggcata tctgttacaa taatatccac 14220
agaagcagtt ctcagtggga ggttgcagat gacctcgagg gggggcccgg tacccagctt 14280
ttgttccctt tagtgagggt taattgcgcg cttggcgtaa tcatggtcat agctgtttcc 14340
tgtgtgaaat tgttatccgc tcacaattcc acacaacata cgagccggaa gcataaagtg 14400
taaagcctgg ggtgcctaat gagtgagcta actcacatta attgcgttgc gctcactgcc 14460
cgctttccag tcgggaaacc tgtcgtgcca gctgcattaa tgaatcggcc aacgcgcggg 14520
gagaggcggt ttgcgtattg ggcgctcttc cgctccaatt cgccctatag tgagtcgtat 14580
tacaattcac tggccgtcgt tttacaacgt cgtgactggg aaaaccctgg cgttacccaa 14640
cttaatcgcc ttgcagcaca tccccctttc gccagctggc gtaatagcga agaggcccgc 14700
accgatcgcc cttcccaaca gttgcgcagc ctgaatggcg aatgggacgc gccctgtagc 14760
ggcgcattaa gcgcggcggg tgtggtggtt acgcgcagcg tgaccgctac acttgccagc 14820
gccctagcgc ccgctccttt cgctttcttc ccttcctttc tcgccacgtt cgccggcttt 14880

ccccgtcaag ctctaaatcg ggggctccct ttagggttcc gatttagtgc tttacggcac 14940
ctcgacccca aaaaacttga ttagggtgat ggttcacgta gtgggccatc gccctgatag 15000
acggtttttc gccctttgac gttggagtcc acgttcttta atagtggact cttgttccaa 15060
actggaacaa cactcaaccc tatctcggtc tattcttttg atttataagg gattttgccg 15120
atttcggcct attggttaaa aaatgagctg atttaacaaa aatttaacgc gaattttaac 15180
aaaatattaa cgcttacaat ttaggtggca cttttcgggg aaatgtgcgc ggaaccccta 15240
tttgtttatt tttctaaata cattcaaata tgtatccgct catgagacaa taaccctgat 15300
aaatgcttca ataatattga aaaaggaaga gtatgagtat tcaacatttc cgtgtcgccc 15360
ttattccctt ttttgcggca ttttgccttc ctgtttttgc tcacccagaa acgctggtga 15420
aagtaaaaga tgctgaagat cagttgggtg cacgagtggg ttacatcgaa ctggatctca 15480
acagcggtaa gatccttgag agttttcgcc ccgaagaacg ttttccaatg atgagcactt 15540
ttaaagttct gctatgtggc gcggtattat cccgtattga cgccgggcaa gagcaactcg 15600
gtcgccgcat acactattct cagaatgact tggttgagta ctcaccagtc acagaaaagc 15660
atcttacgga tggcatgaca gtaagagaat tatgcagtgc tgccataacc atgagtgata 15720
acactgcggc caacttactt ctgacaacga tcggaggacc gaaggagcta accgcttttt 15780
tgcacaacat gggggatcat gtaactcgcc ttgatcgttg ggaaccggag ctgaatgaag 15840
ccataccaaa cgacgagcgt gacaccacga tgcctgtagc aatggcaaca acgttgcgca 15900
aactattaac tggcgaacta cttactctag cttcccggca acaattaata gactggatgg 15960
aggcggataa agttgcagga ccacttctgc gctcggccct tccggctggc tggtttattg 16020
ctgataaatc tggagccggt gagcgtgggt ctcgcggtat cattgcagca ctggggccag 16080
atggtaagcc ctcccgtatc gtagttatct acacgacggg gagtcaggca actatggatg 16140
aacgaaatag acagatcgct gagataggtg cctcactgat taagcattgg taactgtcag 16200
accaagttta ctcatatata ctttagattg atttaaaact tcatttttaa tttaaaagga 16260
tctaggtgaa gatccttttt gataatctca tgaccaaaat cccttaacgt gagttttcgt 16320
tccactgagc gtcagacccc gtagaaaaga tcaaaggatc ttcttgagat cctttttttc 16380
tgcgcgtaat ctgctgcttg caaacaaaaa aaccaccgct accagcggtg gtttgtttgc 16440
cggatcaaga gctaccaact ctttttccga aggtaactgg cttcagcaga gcgcagatac 16500
caaatactgt ccttctagtg tagccgtagt taggccacca cttcaagaac tctgtagcac 16560
cgcctacata cctcgctctg ctaatcctgt taccagtggc tgctgccagt ggcgataagt 16620
cgtgtcttac cgggttggac tcaagacgat agttaccgga taaggcgcag cggtcgggct 16680
gaacgggggg ttcgtgcaca cagcccagct tggagcgaac gacctacacc gaactgagat 16740
acctacagcg tgagctatga gaaagcgcca cgcttcccga agggagaaag gcggacaggt 16800
atccggtaag cggcagggtc ggaacaggag agcgcacgag ggagcttcca gggggaaacg 16860
cctggtatct ttatagtcct gtcgggtttc gccacctctg acttgagcgt cgatttttgt 16920
gatgctcgtc aggggggcgg agcctatgga aaaacgccag caacgcggcc tttttacggt 16980
tcctggcctt ttgctggcct tttgctcaca tgttctttcc tgcgttatcc cctgattctg 17040
tggataaccg tattaccgcc tttgagtgag ctgataccgc tcgccgcagc cgaacgaccg 17100
agcgcagcga gtcagtgagc gaggaagcgg aagagcgccc aatacgcaaa ccgcctctcc 17160
ccgcgcgttg gccgattcat taatgcagct ggcacgacag gtttcccgac tggaaagcgg 17220
gcagtgagcg caacgcaatt aatgtgagtt agctcactca ttaggcaccc caggctttac 17280
actttatgct tccggctcgt atgttgtgtg gaattgtgag cggataacaa tttcacacag 17340
gaaacagcta tgaccatgat tacgccaagc gcgcaattaa ccctcactaa agggaacaaa 17400
agctgtcgag atctagatat cgatggccat agagttacgc tagggataac agggtaatat 17460
agccgcggca ggccctccga gcgtggtgga gccgttctgt gagacagccg ggtacgagtc 17520
gtgacgctgg aaggggcaag cgggtggtgg gcaggaatgc ggtccgccct gcagcaaccg 17580
gagggggagg gagaagggag cggaaaagtc tccaccggac gcggccatgg ctcggggggg 17640
ggggggcagc ggaggagcgc ttccggccga cgtctcgtcg ctgattggct tcttttcctc 17700
ccgccgtgtg tgaaaacaca aatggcgtgt tttggttggc gtaaggcgcc tgtcagttaa 17760
cggcagccgg agtgcgcagc cgccggcagc ctcgctctgc ccactgggtg gggcgggagg 17820
taggtggggt gaggcgagct ggacgtgcgg gcgcggtcgg cctctggcgg ggcgggggag 17880
gggagggagg gtcagcgaaa gtagctcgcg cgcgagcggc cgcccaccct ccccttcctc 17940
tgggggagtc gttttacccg ccgccggccg ggcctcgtcg tctgattggc tctcggggcc 18000
cagaaaactg gcccttgcca ttggctcgtg ttcgtgcaag ttgagtccat ccgccggcca 18060
gcgggggcgg cgaggaggcg ctcccaggtt ccggccctcc cctcggcccc gcgccgcaga 18120
gtctggccgc gcgcccctgc gcaacgtggc aggaagcgcg cgctgggggc ggggacgggc 18180
agtagggctg agcggctgcg gggcgggtgc aagcacgttt ccgacttgag ttgcctcaag 18240
aggggcgtgc tgagccagac ctccatcgcg cactccgggg agtggaggga aggagcgagg 18300
gctcagttgg gctgttttgg aggcaggaag cacttgctct cccaaagtcg ctctgagttg 18360
ttatcagtaa gggagctgca gtggagtagg cggggagaag gccgcaccct tctccggagg 18420
ggggagggga gtgttgcaat acctttctgg gagttctctg ctgcctcctg gcttctgagg 18480
accgccctgg gcctgggaga atcccttccc cctcttccct cgtgatctgc aactccagtc 18540
tttctaggta accgatatcc ctgcaggggt gacctgcacg tctagggcgc agtagtccag 18600
ggtttccttg atgatgtcat acttatcctg tccctttttt ttccacagct cgcggttgag 18660

gacaaactct tcgcggtctt tccagtactc ctgcaggtga ctgactgagt cgacttaatt 18720
aaggccatag cggccatttc tagacttaga tccccgcggg atcttcaagc agacctacag 18780
caagttcgac acaaactcac acaacgatga cgcactactc aagaactacg ggctgctcta 18840
ctgcttcagg aaggacatgg acaagg 18866

<210> 9
<211> 21730
<212> DNA
<213> Artificial Sequence

<220>
<223> Description of Artificial Sequence: Cre substrate
      reporter

<400> 9
taaagggaac aaaagctgtc gagatctaga tatcgatggc catagagtta cgctagggat 60
aacagggtaa tatagccgcg gtcaggccct ccgagcgtgg tggagccgtt ctgtgagaca 120
gccggatcgg caggccctcc gagcgtggtg gagccgttct gtgagacagc cgggtacgag 180
tcgtgacgct ggaaggggca agcgggtggt gggcaggaat gcggtccgcc ctgcagcaac 240
cggaggggga gggagaaggg agcggaaaag tctccaccgg acgcggccat ggctcggggg 300
ggggggggca gcggaggagc gcttccggcc gacgtctcgt cgctgattgg cttcttttcc 360
tcccgccgtg tgtgaaaaca caaatggcgt gttttggttg gcgtaaggcg cctgtcagtt 420
aacggcagcc ggagtgcgca gccgccggca gcctcgctct gcccactggg tggggcggga 480
ggtaggtggg gtgaggcgag ctggacgtgc gggcgcggtc ggcctctggc ggggcggggg 540
aggggaggga gggtcagcga aagtagctcg cgcgcgagcg gccgcccacc ctccccttcc 600
tctgggggag tcgttttacc cgccgccggc cgggcctcgt cgtctgattg gctctcgggg 660
cccagaaaac tggcccttgc cattggctcg tgttcgtgca agttgagtcc atccgccggc 720
cagcgggggc ggcgaggagg cgctcccagg ttccggccct cccctcggcc ccgcgccgca 780
gagtctggcc gcgcgcccct gcgcaacgtg gcaggaagcg cgcgctgggg gcggggacgg 840
gcagtagggc tgagcggctg cggggcgggt gcaagcacgt ttccgacttg agttgcctca 900
agaggggcgt gctgagccag acctccatcg cgcactccgg ggagtggagg gaaggagcga 960
gggctcagtt gggctgtttt ggaggcagga agcacttgct ctcccaaagt cgctctgagt 1020
tgttatcagt aagggagctg cagtggagta ggcggggaga aggccgcacc cttctccgga 1080
ggggggaggg gagtgttgca atacctttct gggagttctc tgctgcctcc tggcttctga 1140
ggaccgccct gggcctggga gaatcccttc cccctcttcc ctcgtgatct gcaactccag 1200
tctttcctag gtaaccgata tccctgcagg ttttcgacat tgattattga ctagttatta 1260
atagtaatca attacggggt cattagttca tagcccatat atggagttcc gcgttacata 1320
acttacggta aatggcccgc ctggctgacc gcccaacgac ccccgcccat tgacgtcaat 1380
aatgacgtat gttcccatag taacgccaat agggactttc cattgacgtc aatgggtgga 1440
ctatttacgg taaactgccc acttggcagt acatcaagtg tatcatatgc caagtacgcc 1500
ccctattgac gtcaatgacg gtaaatggcc cgcctggcat tatgcccagt acatgacctt 1560
atgggacttt cctacttggc agtacatcta cgtattagtc atcgctatta ccatgggtcg 1620
aggtgagccc cacgttctgc ttcactctcc ccatctcccc cccctcccca cccccaattt 1680
tgtatttatt tattttttaa ttattttgtg cagcgatggg ggcggggggg gggggggcgc 1740
gcgccaggcg gggcggggcg gggcgagggg cggggcgggg cgaggcggag aggtgcggcg 1800
gcagccaatc agagcggcgc gctccgaaag tttcctttta tggcgaggcg gcggcggcgg 1860
cggccctata aaaagcgaag cgcgcggcgg gcgggagtcg ctgcgttgcc ttcgccccgt 1920
gccccgctcc gcgccgcctc gcgccgcccg ccccggctct gactgaccgc gttactccca 1980
caggtgagcg ggcgggacgg cccttctcct ccgggctgta attagcgctt ggtttaatga 2040
cggctcgttt cttttctgtg gctgcgtgaa agccttaaag ggctccggga gggccctttg 2100
tgcggggggg agcggctcgg ggggtgcgtg cgtgtgtgtg tgcgtgggga gcgccgcgtg 2160
cggcccgcgc tgcccggcgg ctgtgagcgc tgcgggcgcg gcgcggggct ttgtgcgctc 2220
cgcgtgtgcg cgaggggagc gcggccgggg gcggtgcccc gcggtgcggg ggggctgcga 2280
ggggaacaaa ggctgcgtgc ggggtgtgtg cgtggggggg tgagcagggg gtgtgggcgc 2340
ggcggtcggg ctgtaacccc cccctgcacc cccctccccg agttgctgag cacggcccgg 2400
cttcgggtgc ggggctccgt gcggggcgtg gcgcggggct cgccgtgccg ggcggggggt 2460
ggcggcaggt gggggtgccg ggcggggcgg ggccgcctcg ggccggggag ggctcggggg 2520
aggggcgcgg cggccccgga gcgccggcgg ctgtcgaggc gcggcgagcc gcagccattg 2580
ccttttatgg taatcgtgcg agagggcgca gggacttcct ttgtcccaaa tctggcggag 2640
ccgaaatctg ggaggcgccg ccgcaccccc tctagcgggc gcgggcgaag cggtgcggcg 2700
ccggcaggaa ggaaatgggc ggggagggcc ttcgtgcgtc gccgcgccgc cgtccccttc 2760
tccatctcca gcctcggggc tgccgcaggg ggacggctgc cttcgggggg gacggggcag 2820

ggcggggttc ggcttctggc gtgtgaccgg cggctctaga agcgttgggg tgagtactcc 2880
ctctcaaaag cgggcatgac ttctgcgcta agattgtcag tttccaaaaa cgaggaggat 2940
ttgatattca cctggcccgc ggtgatgcct ttgagggtgg ccgcgtccat ctggtcagaa 3000
aagacaatct ttttgttgtc aagcttgagg tgtggcaggc ttgagatctg gccatacact 3060
tgagtgacat tgacatccac tttgcctttc tctccacagg tgtccactcc cagggcggcc 3120
tccggagcga tcgccggtcc gcctaggcta gccggccgcg tcgacttaat taaggccata 3180
gcggccattt tcgacgacac tgcagagacc tacttcacta acaaccggta cagttcggtg 3240
gagtacgcgc ccggggagcc caagggcacg ccctggcacc cgcaccgcgg cgaataactt 3300
cgtatagcat acattatacg aagttataag ctcgatgaat tctaccgggt aggggaggcg 3360
cttttcccaa ggcagtctgg agcatgcgct ttagcagccc cgctggcact tggcgctaca 3420
caagtggcct ctggcctcgc acacattcca catccaccgg tagcgccaac cggctccgtt 3480
ctttggtggc cccttcgcgc caccttctac tcctccccta gtcaggaagt tcccccccgc 3540
cccgcagctc gcgtcgtgca ggacgtgaca aatggaagta gcacgtctca ctagtctcgt 3600
gcagatggac agcaccgctg agcaatggaa gcgggtaggc ctttggggca gcggccaata 3660
gcagctttgc tccttcgctt tctgggctca gaggctggga aggggtgggt ccgggggcgg 3720
gctcaggggc gggctcaggg gcggggcggg cgcgaaggtc ctcccgaggc ccggcattct 3780
cgcacgcttc aaaagcgcac gtctgccgcg ctgttctcct cttcctcatc tccgggcctt 3840
tcgacgatcc agccgccacc atgaaaaagc ctgaactcac cgcgacgtct gtcgagaagt 3900
ttctgatcga aaagttcgac agcgtctccg acctgatgca gctctcggag ggcgaagaat 3960
ctcgtgcttt cagcttcgat gtaggagggc gtggatatgt cctgcgggta aatagctgcg 4020
ccgatggttt ctacaaagat cgttatgttt atcggcactt tgcatcggcc gcgctcccga 4080
ttccggaagt gcttgacatt ggggaattca gcgagagcct gacctattgc atctcccgcc 4140
gtgcacaggg tgtcacgttg caagacctgc ctgaaaccga actgcccgct gttctgcagc 4200
cggtcgcgga ggccatggat gcgatcgctg cggccgatct tagccagacg agcgggttcg 4260
gcccattcgg accgcaagga atcggtcaat acactacatg gcgtgatttc atatgcgcga 4320
ttgctgatcc ccatgtgtat cactggcaaa ctgtgatgga cgacaccgtc agtgcgtccg 4380
tcgcgcaggc tctcgatgag ctgatgcttt gggccgagga ctgccccgaa gtccggcacc 4440
tcgtgcacgc ggatttcggc tccaacaatg tcctgacgga caatggccgc ataacagcgg 4500
tcattgactg gagcgaggcg atgttcgggg attcccaata cgaggtcgcc aacatcttct 4560
tctggaggcc gtggttggct tgtatggagc agcagacgcg ctacttcgag cggaggcatc 4620
cggagcttgc aggatcgccg cggctccggg cgtatatgct ccgcattggt cttgaccaac 4680
tctatcagag cttggttgac ggcaatttcg atgatgcagc ttgggcgcag ggtcgatgcg 4740
acgcaatcgt ccgatccgga gccgggactg tcgggcgtac acaaatcgcc cgcagaagcg 4800
cggccgtctg gaccgatggc tgtgtagaag tactcgccga tagtggaaac cgacgcccca 4860
gcactcgtcc gagggcaaag gaatagtcga tgcagaaatt gatgatctat taaacaataa 4920
agatgtccac taaaatggaa gtttttcctg tcatactttg ttaagaaggg tgagaacaga 4980
gtacctacat tttgaatgga aggattggag ctacgggggt gggggtgggg tgggattaga 5040
taaatgcctg ctctttactg aaggctcttt actattgctt tatgataatg tttcatagtt 5100
ggatatcata atttaaacaa gcaaaaccaa attaagggcc agctcattcc tcccactcat 5160
gatctataga tctatagatc tctcgtggga tcattgtttt tctcttgatt cccactttgt 5220
ggttctaagt actgtggttt ccaaatgtgt cagtttcata gcctgaagaa cgagatcagc 5280
agcctctgtt ccacatacac ttcattctca gtattgtttt gccaagttct aattccatca 5340
gaagcttcag ctgctcgact agaggatcat aatcagccat accacatttg tagaggtttt 5400
acttgcttta aaaaacctcc cacacctccc cctgaacctg aaacataaaa tgaatgcaat 5460
tgttgttgtt aacttgttta ttgcagctta taatggttac aaataaagca atagcatcac 5520
aaatttcaca aataaagcat ttttttcact gcattctagt tgtggtttgt ccaaactcat 5580
caatgtatct tatcatgtct ggatccctac gcccccaact gagagaactc aaaggttacc 5640
ccagttgggg cactacggat ccataacttc gtatagcata cattatacga agttataccg 5700
ggccaccggc gcgccatgcc taagaagaag aggaaggttg tcgcgagtag cttggcactg 5760
gccgtcgttt tacaacgtcg tgactgggaa aaccctggcg ttacccaact taatcgcctt 5820
gcagcacatc cccctttcgc cagctggcgt aatagcgaag aggcccgcac cgatcgccct 5880
tcccaacagt tgcgcagcct gaatggcgaa tggcgctttg cctggtttcc ggcaccagaa 5940
gcggtgccgg aaagctggct ggagtgcgat cttcctgagg ccgatactgt cgtcgtcccc 6000
tcaaactggc agatgcacgg ttacgatgcg cccatctaca ccaacgtaac ctatcccatt 6060
acggtcaatc cgccgtttgt tcccacggag aatccgacgg gttgttactc gctcacattt 6120
aatgttgatg aaagctggct acaggaaggc cagacgcgaa ttatttttga tggcgttaac 6180
tcggcgtttc atctgtggtg caacgggcgc tgggtcggtt acggccagga cagtcgtttg 6240
ccgtctgaat ttgacctgag cgcattttta cgcgccggag aaaaccgcct cgcggtgatg 6300
gtgctgcgtt ggagtgacgg cagttatctg gaagatcagg atatgtggcg gatgagcggc 6360
attttccgtg acgtctcgtt gctgcataaa ccgactacac aaatcagcga tttccatgtt 6420
gccactcgct ttaatgatga tttcagccgc gctgtactgg aggctgaagt tcagatgtgc 6480
ggcgagttgc gtgactacct acgggtaaca gtttctttat ggcaggtgaa aacgcaggtc 6540
gccagcggca ccgcgccttt cggcggtgaa attatcgatg agcgtggtgg ttatgccgat 6600

cgcgtcacac tacgtctgaa cgtcgaaaac ccgaaactgt ggagcgccga aatcccgaat 6660
ctctatcgtg cggtggttga actgcacacc gccgacggca cgctgattga agcagaagcc 6720
tgcgatgtcg gtttccgcga ggtgcggatt gaaaatggtc tgctgctgct gaacggcaag 6780
ccgttgctga ttcgaggcgt taaccgtcac gagcatcatc ctctgcatgg tcaggtcatg 6840
gatgagcaga cgatggtgca ggatatcctg ctgatgaagc agaacaactt taacgccgtg 6900
cgctgttcgc attatccgaa ccatccgctg tggtacacgc tgtgcgaccg ctacggcctg 6960
tatgtggtgg atgaagccaa tattgaaacc cacggcatgg tgccaatgaa tcgtctgacc 7020
gatgatccgc gctggctacc ggcgatgagc gaacgcgtaa cgcgaatggt gcagcgcgat 7080
cgtaatcacc cgagtgtgat catctggtcg ctggggaatg aatcaggcca cggcgctaat 7140
cacgacgcgc tgtatcgctg gatcaaatct gtcgatcctt cccgcccggt gcagtatgaa 7200
ggcggcggag ccgacaccac ggccaccgat attatttgcc cgatgtacgc gcgcgtggat 7260
gaagaccagc ccttcccggc tgtgccgaaa tggtccatca aaaaatggct ttcgctacct 7320
ggagagacgc gcccgctgat cctttgcgaa tacgcccacg cgatgggtaa cagtcttggc 7380
ggtttcgcta aatactggca ggcgtttcgt cagtatcccc gtttacaggg cggcttcgtc 7440
tgggactggg tggatcagtc gctgattaaa tatgatgaaa acggcaaccc gtggtcggct 7500
tacggcggtg attttggcga tacgccgaac gatcgccagt tctgtatgaa cggtctggtc 7560
tttgccgacc gcacgccgca tccagcgctg acggaagcaa aacaccagca gcagtttttc 7620
cagttccgtt tatccgggca aaccatcgaa gtgaccagcg aatacctgtt ccgtcatagc 7680
gataacgagc tcctgcactg gatggtggcg ctggatggta agccgctggc aagcggtgaa 7740
gtgcctctgg atgtcgctcc acaaggtaaa cagttgattg aactgcctga actaccgcag 7800
ccggagagcg ccgggcaact ctggctcaca gtacgcgtag tgcaaccgaa cgcgaccgca 7860
tggtcagaag ccgggcacat cagcgcctgg cagcagtggc gtctggcgga aaacctcagt 7920
gtgacgctcc ccgccgcgtc ccacgccatc ccgcatctga ccaccagcga aatggatttt 7980
tgcatcgagc tgggtaataa gcgttggcaa tttaaccgcc agtcaggctt tctttcacag 8040
atgtggattg gcgataaaaa acaactgctg acgccgctgc gcgatcagtt cacccgtgca 8100
ccgctggata acgacattgg cgtaagtgaa gcgacccgca ttgaccctaa cgcctgggtc 8160
gaacgctgga aggcggcggg ccattaccag gccgaagcag cgttgttgca gtgcacggca 8220
gatacacttg ctgatgcggt gctgattacg accgctcacg cgtggcagca tcaggggaaa 8280
accttattta tcagccggaa aacctaccgg attgatggta gtggtcaaat ggcgattacc 8340
gttgatgttg aagtggcgag cgatacaccg catccggcgc ggattggcct gaactgccag 8400
ctggcgcagg tagcagagcg ggtaaactgg ctcggattag ggccgcaaga aaactatccc 8460
gaccgcctta ctgccgcctg ttttgaccgc tgggatctgc cattgtcaga catgtatacc 8520
ccgtacgtct tcccgagcga aaacggtctg cgctgcggga cgcgcgaatt gaattatggc 8580
ccacaccagt ggcgcggcga cttccagttc aacatcagcc gctacagtca acagcaactg 8640
atggaaacca gccatcgcca tctgctgcac gcggaagaag gcacatggct gaatatcgac 8700
ggtttccata tggggattgg tggcgacgac tcctggagcc cgtcagtatc ggcggaattc 8760
cagctgagcg ccggtcgcta ccattaccag ttggtctggt gtcaaaaata ataataaccg 8820
ggcagggggg atctttgtga aggaacctta cttctgtggt gtgacataat tggacaaact 8880
acctacagag atttaaagct ctaaggtaaa tataaaattt ttaagtgtat aatgtgttaa 8940
actactgatt ctaattgttt gtgtatttta gattccaacc tatggaactg atgaatggga 9000
gcagtggtgg aatgccagat ccagacatga taagatacat tgatgagttt ggacaaacca 9060
caactagaat gcagtgaaaa aaatgcttta tttgtgaaat ttgtgatgct attgctttat 9120
ttgtaaccat tataagctgc aataaacaag ttaacaacaa caattgcatt cattttatgt 9180
ttcaggttca gggggaggtg tgggaggttt tttaaagcaa gtaaaacctc tacaaatgtg 9240
gtatggctga ttatgatctg cggccaaatc ggccggccta ggcgcgccgg taaccgaagt 9300
tcctatactt tctagagaat aggaacttcg gaataggaac ttcaagctta agcgctagca 9360
ctagaagatg ggcgggagtc ttctgggcag gcttaaaggc taacctggtg tgtgggcgtt 9420
gtcctgcagg ggaattgaac aggtgtaaaa ttggagggac aagacttccc acagattttc 9480
ggttttgtcg ggaagttttt taataggggc aaataaggaa aatgggagga taggtagtca 9540
tctggggttt tatgcagcaa aactacaggt tattattgct tgtgatccgc ctcggagtat 9600
tttccatcga ggtagattaa agacatgctc acccgagttt tatactctcc tgcttgagat 9660
ccttactaca gtatgaaatt acagtgtcgc gagttagact atgtaagcag aattttaatc 9720
atttttaaag agcccagtac ttcatatcca tttctcccgc tccttctgca gccttatcaa 9780
aaggtatttt agaacactca ttttagcccc attttcattt attatactgg cttatccaac 9840
ccctagacag agcattggca ttttcccttt cctgatctta gaagtctgat gactcatgaa 9900
accagacaga ttagttacat acaccacaaa tcgaggctgt agctggggcc tcaacactgc 9960
agttctttta taactcctta gtacactttt tgttgatcct ttgccttgat ccttaatttt 10020
cagtgtctat cacctctccc gtcagtggtg ttccacattt gggcctattc tcagtccagg 10080
gagttttaca acaatagatg tattgagaat ccaacctaaa gcttaacttt ccactcccat 10140
gaatgcctct ctcctttttc tccatttata aactgagcta ttaaccatta atggttccag 10200
gtggatgtct cctccccata ttacctgatg tatcttacat attgccaggc tgatatttta 10260
agacattaaa aggtatattt cattattgag ccacatggta ttgattactg cttactaaaa 10320
ttttgtcatt gtacacatct gtaaaaggtg gttccttttg gaatgcaaag ttcaggtgtt 10380

tgttgtcttt cctgacctaa ggtcttgtga gcttgtattt tttctattta agcagtgctt 10440
tctcttggac tggcttgact catggcattc tacacgttat tgctggtcta aatgtgattt 10500
tgccaagctt cttcaggacc tataattttg cttgacttgt agccaaacac aagtaaaatg 10560
attaagcaac aaatgtattt gtgaagcttg gtttttaggt tgttgtgttg tgtgtgcttg 10620
tgctctataa taatactatc caggggctgg agaggtggct cggagttcaa gagcacagac 10680
tgctcttcca gaagtcctga gttcaattcc cagcaaccac atggtggctc acaaccatct 10740
gtaatgggat ctgatgccct cttctggtgt gtctgaagac cacaagtgta ttcacattaa 10800
ataaataaat cctccttctt cttctttttt ttttttttaa agagaatact gtctccagta 10860
gaatttactg aagtaatgaa atactttgtg tttgttccaa tatggtagcc aataatcaaa 10920
ttactcttta agcactggaa atgttaccaa ggaactaatt tttatttgaa gtgtaactgt 10980
ggacagagga gccataactg cagacttgtg ggatacagaa gaccaatgca gactttaatg 11040
tcttttctct tacactaagc aataaagaaa taaaaattga acttctagta tcctatttgt 11100
ttaaactgct agctttactt aacttttgtg cttcatctat acaaagctga aagctaagtc 11160
tgcagccatt actaaacatg aaagcaagta atgataattt tggatttcaa aatgtagggg 11220
ccagagttta gccagccagt ggtggtgctt gcctttatgc ctttaatccc agcactctgg 11280
aggcagagac aggcagatct ctgagtttga gcccagcctg gtctacacat caagttctat 11340
ctaggatagc caggaataca cacagaaacc ctgttgggga ggggggctct gagatttcat 11400
aaaattataa ttgaagcatt ccctaatgag ccactatgga tgtggctaaa tccgtctacc 11460
tttctgatga gatttgggta ttattttttc tgtctctgct gttggttggg tcttttgaca 11520
ctgtgggctt tctttaaagc ctccttcctg ccatgtggtc tcttgtttgc tactaacttc 11580
ccatggctta aatggcatgg ctttttgcct tctaagggca gctgctgaga tttgcagcct 11640
gatttccagg gtggggttgg gaaatctttc aaacactaaa attgtccttt aatttttttt 11700
ttaaaaaatg ggttatataa taaacctcat aaaatagtta tgaggagtga ggtggactaa 11760
tattaaatga gtccctcccc tataaaagag ctattaaggc tttttgtctt atacttaact 11820
ttttttttaa atgtggtatc tttagaacca agggtcttag agttttagta tacagaaact 11880
gttgcatcgc ttaatcagat tttctagttt caaatccaga gaatccaaat tcttcacagc 11940
caaagtcaaa ttaagaattt ctgactttta atgttaattt gcttactgtg aatataaaaa 12000
tgatagcttt tcctgaggca gggtctcact atgtatctct gcctgatctg caacaagata 12060
tgtagactaa agttctgcct gcttttgtct cctgaatact aaggttaaaa tgtagtaata 12120
cttttggaac ttgcaggtca gattctttta taggggacac actaagggag cttgggtgat 12180
agttggtaaa atgtgtttca agtgatgaaa acttgaatta ttatcaccgc aacctacttt 12240
ttaaaaaaaa aagccaggcc tgttagagca tgcttaaggg atccctagga cttgctgagc 12300
acacaagagt agttacttgg caggctcctg gtgagagcat atttcaaaaa acaaggcaga 12360
caaccaagaa actacagtta aggttacctg tctttaaacc atctgcatat acacagggat 12420
attaaaatat tccaaataat atttcattca agttttcccc catcaaattg ggacatggat 12480
ttctccggtg aataggcaga gttggaaact aaacaaatgt tggttttgtg atttgtgaaa 12540
ttgttttcaa gtgatagtta aagcccatga gatacagaac aaagctgcta tttcgaggtc 12600
tcttggttta tactcagaag cacttctttg ggtttccctg cactatcctg atcatgtgct 12660
aggcctacct taggctgatt gttgttcaaa taaacttaag tttcctgtca ggtgatgtca 12720
tatgatttca tatatcaagg caaaacatgt tatatatgtt aaacatttgt acttaatgtg 12780
aaagttaggt ctttgtgggt ttgattttta attttcaaaa cctgagctaa ataagtcatt 12840
tttacatgtc ttacatttgg tggaattgta taattgtggt ttgcaggcaa gactctctga 12900
cctagtaacc ctacctatag agcactttgc tgggtcacaa gtctaggagt caagcatttc 12960
accttgaagt tgagacgttt tgttagtgta tactagttta tatgttggag gacatgttta 13020
tccagaagat attcaggact atttttgact gggctaagga attgattctg attagcactg 13080
ttagtgagca ttgagtggcc tttaggcttg aattggagtc acttgtatat ctcaaataat 13140
gctggccttt tttaaaaagc ccttgttctt tatcaccctg ttttctacat aatttttgtt 13200
caaagaaata cttgtttgga tctccttttg acaacaatag catgttttca agccatattt 13260
tttttccttt tttttttttt ttttggtttt tcgagacagg gtttctctgt atagccctgg 13320
ctgtcctgga actcactttg tagaccaggc tggcctcgaa ctcagaaatc cgcctgcctc 13380
tgcctcctga gtgccgggat taaaggcgtg caccaccacg cctggctaag ttggatattt 13440
tgttatataa ctataaccaa tactaactcc actgggtgga tttttaattc agtcagtagt 13500
cttaagtggt ctttattggc ccttcattaa aatctactgt tcactctaac agaggctgtt 13560
ggtactagtg gcacttaagc aacttcctac ggatatacta gcagattaag ggtcagggat 13620
agaaactagt ctagcgtttt gtatacctac cagctttata ctaccttgtt ctgatagaaa 13680
tatttcagga catctagagt gtactataag gttgatggta agcttataag gaacttgaaa 13740
gtggagtaac tactccattt ctctgagggg agaattaaaa tttttgacca agtgttgttg 13800
agccactgag aatggtctca gaacataact tcttaaggaa ccttcccaga ttgccctcaa 13860
cactgcacca catttggtcc tgcttgaaca ttgccatggc tcttaaagtc ttaattaaga 13920
atattaattg tgtaattatt gtttttcctc ctttagatca ttccttgagg acaggacagt 13980
gcttgtttaa ggctatattt ctgctgtctg agcagcaaca ggtcttcgag atcaacatga 14040
tgttcataat cccaagatgt tgccatttat gttctcagaa gcaagcagag gcatgatggt 14100
cagtgacagt aatgtcactg tgttaaatgt tgctatgcag tttggatttt tctaatgtag 14160

tgtaggtaga acatatgtgt tctgtatgaa ttaaactctt aagttacacc ttgtataatc 14220
catgcaatgt gttatgcaat taccatttta agtattgtag ctttctttgt atgtgaggat 14280
aaaggtgttt gtcataaaat gttttgaaca tttccccaaa gttccaaatt ataaaaccac 14340
aacgttagaa cttatttatg aacaatggtt gtagtttcat gcttttaaaa tgcttaatta 14400
ttcaattaac accgtttgtg ttataatata tataaaactg acatgtagaa gtgtttgtcc 14460
agaacatttc ttaaatgtat actgtcttta gagagtttaa tatagcatgt cttttgcaac 14520
atactaactt ttgtgttggt gcgagcaata ttgtgtagtc attttgaaag gagtcatttc 14580
aatgagtgtc agattgtttt gaatgttatt gaacatttta aatgcagact tgttcgtgtt 14640
ttagaaagca aaactgtcag aagctttgaa ctagaaatta aaaagctgaa gtatttcaga 14700
agggaaataa gctacttgct gtattagttg aaggaaagtg taatagctta gaaaatttaa 14760
aaccatatag ttgtcattgc tgaatatctg gcagatgaaa agaaatactc agtggttctt 14820
ttgagcaata taacagcttg ttatattaaa aattttcccc acagatataa actctaatct 14880
ataactcata aatgttacaa atggatgaag cttacaaatg tggcttgact tgtcactgtg 14940
cttgttttag ttatgtgaaa gtttggcaat aaacctatgt cctaaatagt caaactgtgg 15000
aatgactttt taatctattg gtttgtctag aacagttatg ttgccatttg ccctaatggt 15060
gaaagaaaaa gtggggagtg ccttggcact gttcatttgt ggtgtgaacc aaagaggggg 15120
gcatgcactt acacttcaaa catccttttg aaagactgac aagtttgggt cttcacagtt 15180
ggaattgggc atcccttttg tcagggaggg agggagggag ggaggctggc ttgttatgct 15240
gacaagtgtg attaaattca aactttgagg taagttggag gaacttgtac attgttagga 15300
gtgtgacaat ttggactctt aatgatttgg tcatacaaaa tgaacctaga ccaacttctg 15360
gaagatgtat ataataactc catgttacat tgatttcacc tgactaatac ttatccctta 15420
tcaattaaat acagaagatg ccagccatct gggcctttta acccagaaat ttagtttcaa 15480
actcctaggt tagtgttctc actgagctac atcctgatct agtcctgaaa ataggaccac 15540
catcaccccc aaaaaaatct caaataagat ttatgctagt gtttcaaaat tttaggaata 15600
ggtaagatta gaaagtttta aattttgaga aatggcttct ctagaaagat gtacatagtg 15660
aacactgaat ggctcctaaa gagcctagaa aactggtact gagcacacag gactgagagg 15720
tctttcttga aaagcatgta ttgctttacg tgggtcacag aaggcaggca ggaagaactt 15780
gggctgaaac tggtgtctta agtggctaac atcttcacaa ctgatgagca agaactttat 15840
cctgatgcaa aaaccatcca aacaaactaa gtgaaaggtg gcaatggatc ccaggctgct 15900
ctagaggagg acttgacttc tcatcccatc acccacacca gatagctcat agactgccaa 15960
ttaacaccag cttctagcct ccacaggcac ctgcactggt acacataatt tcacacaaac 16020
acagtaagaa gccttccacc tggcatggta ttgcttatct ttagttccca acacttggga 16080
ggcagaggcc agccagggct atgtgacaaa aaccttgtct agaggagaaa cttcatagct 16140
tatttcctat tcacgtaacc aggttagcaa aatttaccag ccagagatga agctaacagt 16200
gtccactata tttgtagtgt tttaagtcaa ttttttaaat atacttaata gaattaaagc 16260
tatggtgaac caagtacaaa cctggtgtat taacttgaga acttagcata aaaagtagtt 16320
catttgttca gtaaatatta aatgcttact ggcaaagatt atgtcaggaa cttggtaaat 16380
ggtgatgaaa caatcatagt tgtacatctt ggttctgtga tcaccttggt ttgaggtaaa 16440
agtggttcct ttgatcaagg atggaatttt aagtttatat tcaatcaata atgtattatt 16500
ttgtgattgc aaaattgcct atctagggta taaaaccttt aaaaatttca taataccagt 16560
tcattctcca gttactaatt ccaaaaagcc actgactatg gtgccaatgt ggattctgtt 16620
ctcaaaggaa ggattgtctg tgccctttat tctaatagaa acatcacact gaaaatctaa 16680
gctgaaagaa gccagacttt cctaaataaa taactttcca taaagctcaa acaaggatta 16740
cttttaggag gcactgttaa ggaactgata agtaatgagg ttacttatat aatgatagtc 16800
ccacaagact atctgaggaa aaatcagtac aactcgaaaa cagaacaacc agctaggcag 16860
gaataacagg gctcccaagt caggaggtct atccaacacc cttttctgtt gagggcccca 16920
gacctacata ttgtatacaa acagggaggt gggtgatttt aactctcctg aggtaccttg 16980
gtaaatcttt gtcctgagta agcagtacag tgtacagttt acattttcat ttaaagatac 17040
attagctccc tctaccccct aagactgaca ggcactttgg gggtggggag ggctttggaa 17100
aataacgctt ccatacacta aaagagaaat ttctttaatt aggcttgttg gttccataca 17160
tctactggtg tttctactac ttagtaatat tataatagtc acacaagcat ctttgctctg 17220
tttaggttgt atatttattt taaggcagat gataaaactg tagatcttaa gggatgcttc 17280
tgcttctgag atgatacaaa gaatttagac cataaaacag taggttgcac aagcaataga 17340
atatggccta aagtgttctg acacttagaa gccaagcagt gtaggcttct taagaaatac 17400
cattacaatc accttgctag aaatcaagca ttctggagtg gtcaagcagt gtaacctgta 17460
ctgtaagtta cttttctgct atttttctcc caaagcaagt tctttatgct gatatttcca 17520
gtgttaggaa ctacaaatat taataagttg tcttcactct tttctttacc aaggagggtc 17580
tcttccttca tcttgatctg aaggatgaac aaaggcttga gcagtgcgct ttagaagata 17640
aactgcagca tgaaggcccc cgatgttcac ccagactaca tggacctttc gccacacatg 17700
tcccattcca gataaggcct ggcacacaca aaaaacataa gtcattaggc taccagtctg 17760
attctaaaac aacctaaaat cttcccactt aaatgctatg ggtggtgggt tggaaagttg 17820
actcagaaaa tcacttgctg tttttagaga ggatctgggt tcagtttctg atacattgtg 17880
gcttacaact ataactccag ttctaggggg tccatccaac atcctcttct gttgagggca 17940

ccaaataaat gtattgtgta caaacaggga ggtgagtgat ttaactctcg tgtatagtac 18000
cttggtaaaa catttcttgt cctgagtaag cagtacagct ctgcctgtcc ctggtctaca 18060
gacacggctc atttcccgaa ggcaagctgg atagagattc caatttctct tcttggatcc 18120
catcctataa aagaaggtca agtttaatct attgcaaaag gtaaataggt agtttcttac 18180
atgagacaag aacaaatctt aggtgtgaag cagtcatctt ttacaggcca gagcctctat 18240
tctatgccaa tgaaggaaac tgttagtcca gtgttataga gttagtccag tgtatagttt 18300
tctatcagaa cacttttttt ttaaacaact gcaacttagc ttattgaaga caaaccacga 18360
gtagaaatct gtccaagaag caagtgcttc tcagcctaca atgtggaata ggaccatgta 18420
atggtacagt gagtgaaatg aattatggca tgtttttctg actgagaaga cagtacaata 18480
aaaggtaaac tcatggtatt tatttaaaaa gaatccaatt tctacctttt tccaaatggc 18540
atatctgtta caataatatc cacagaagca gttctcagtg ggaggttgca gatgacctcg 18600
aggggggccc cggtacccag cttttgttcc ctttagtgag ggttaattgc gcgcttggcg 18660
taatcatggt catagctgtt tcctgtgtga aattgttatc cgctcacaat tccacacaac 18720
atacgagccg gaagcataaa gtgtaaagcc tggggtgcct aatgagtgag ctaactcaca 18780
ttaattgcgt tgcgctcact gcccgctttc cagtcgggaa acctgtcgtg ccagctgcat 18840
taatgaatcg gccaacgcgc ggggagaggc ggtttgcgta ttgggcgctc ttccgctcca 18900
attcgcccta tagtgagtcg tattacaatt cactggccgt cgttttacaa cgtcgtgact 18960
gggaaaaccc tggcgttacc caacttaatc gccttgcagc acatccccct ttcgccagct 19020
ggcgtaatag cgaagaggcc cgcaccgatc gcccttccca acagttgcgc agcctgaatg 19080
gcgaatggga cgcgccctgt agcggcgcat taagcgcggc gggtgtggtg gttacgcgca 19140
gcgtgaccgc tacacttgcc agcgccctag cgcccgctcc tttcgctttc ttcccttcct 19200
ttctcgccac gttcgccggc tttccccgtc aagctctaaa tcgggggctc cctttagggt 19260
tccgatttag tgctttacgg cacctcgacc ccaaaaaact tgattagggt gatggttcac 19320
gtagtgggcc atcgccctga tagacggttt ttcgcccttt gacgttggag tccacgttct 19380
ttaatagtgg actcttgttc caaactggaa caacactcaa ccctatctcg gtctattctt 19440
ttgatttata agggattttg ccgatttcgg cctattggtt aaaaaatgag ctgatttaac 19500
aaaaatttaa cgcgaatttt aacaaaatat taacgcttac aatttaggtg gcacttttcg 19560
gggaaatgtg cgcggaaccc ctatttgttt atttttctaa atacattcaa atatgtatcc 19620
gctcatgaga caataaccct gataaatgct tcaataatat tgaaaaagga agagtatgag 19680
tattcaacat ttccgtgtcg cccttattcc cttttttgcg gcattttgcc ttcctgtttt 19740
tgctcaccca gaaacgctgg tgaaagtaaa agatgctgaa gatcagttgg gtgcacgagt 19800
gggttacatc gaactggatc tcaacagcgg taagatcctt gagagttttc gccccgaaga 19860
acgttttcca atgatgagca cttttaaagt tctgctatgt ggcgcggtat tatcccgtat 19920
tgacgccggg caagagcaac tcggtcgccg catacactat tctcagaatg acttggttga 19980
gtactcacca gtcacagaaa agcatcttac ggatggcatg acagtaagag aattatgcag 20040
tgctgccata accatgagtg ataacactgc ggccaactta cttctgacaa cgatcggagg 20100
accgaaggag ctaaccgctt ttttgcacaa catgggggat catgtaactc gccttgatcg 20160
ttgggaaccg gagctgaatg aagccatacc aaacgacgag cgtgacacca cgatgcctgt 20220
agcaatggca acaacgttgc gcaaactatt aactggcgaa ctacttactc tagcttcccg 20280
gcaacaatta atagactgga tggaggcgga taaagttgca ggaccacttc tgcgctcggc 20340
ccttccggct ggctggttta ttgctgataa atctggagcc ggtgagcgtg ggtctcgcgg 20400
tatcattgca gcactggggc cagatggtaa gccctcccgt atcgtagtta tctacacgac 20460
ggggagtcag gcaactatgg atgaacgaaa tagacagatc gctgagatag gtgcctcact 20520
gattaagcat tggtaactgt cagaccaagt ttactcatat atactttaga ttgatttaaa 20580
acttcatttt taatttaaaa ggatctaggt gaagatcctt tttgataatc tcatgaccaa 20640
aatcccttaa cgtgagtttt cgttccactg agcgtcagac cccgtagaaa agatcaaagg 20700
atcttcttga gatccttttt ttctgcgcgt aatctgctgc ttgcaaacaa aaaaaccacc 20760
gctaccagcg gtggtttgtt tgccggatca agagctacca actctttttc cgaaggtaac 20820
tggcttcagc agagcgcaga taccaaatac tgtccttcta gtgtagccgt agttaggcca 20880
ccacttcaag aactctgtag caccgcctac atacctcgct ctgctaatcc tgttaccagt 20940
ggctgctgcc agtggcgata agtcgtgtct taccgggttg gactcaagac gatagttacc 21000
ggataaggcg cagcggtcgg gctgaacggg gggttcgtgc acacagccca gcttggagcg 21060
aacgacctac accgaactga gatacctaca gcgtgagcta tgagaaagcg ccacgcttcc 21120
cgaagggaga aaggcggaca ggtatccggt aagcggcagg gtcggaacag gagagcgcac 21180
gagggagctt ccagggggaa acgcctggta tctttatagt cctgtcgggt ttcgccacct 21240
ctgacttgag cgtcgatttt tgtgatgctc gtcagggggg cggagcctat ggaaaaacgc 21300
cagcaacgcg gcctttttac ggttcctggc cttttgctgg ccttttgctc acatgttctt 21360
tcctgcgtta tcccctgatt ctgtggataa ccgtattacc gcctttgagt gagctgatac 21420
cgctcgccgc agccgaacga ccgagcgcag cgagtcagtg agcgaggaag cggaagagcg 21480
cccaatacgc aaaccgcctc tccccgcgcg ttggccgatt cattaatgca gctggcacga 21540
caggtttccc gactggaaag cgggcagtga gcgcaacgca attaatgtga gttagctcac 21600
tcattaggca ccccaggctt tacactttat gcttccggct cgtatgttgt gtggaattgt 21660
gagcggataa caatttcaca caggaaacag ctatgaccat gattacgcca agcgcgcaat 21720

taaccctcac 21730

<210> 10
<211> 10491
<212> DNA
<213> Artificial Sequence

<220>
<223> Description of Artificial Sequence: basic Rosa
      targeting vector

<400> 10
tttgagtgag ctgataccgc tcgccgcagc cgaacgaccg agcgcagcga gtcagtgagc 60
gaggaagcgg aagagcgccc aatacgcaaa ccgcctctcc ccgcgcgttg gccgattcat 120
taatgcagct ggcacgacag gtttcccgac tggaaagcgg gcagtgagcg caacgcaatt 180
aatgtgagtt agctcactca ttaggcaccc caggctttac actttatgct tccggctcgt 240
atgttgtgtg gaattgtgag cggataacaa tttcacacag gaaacagcta tgaccatgat 300
tacgccaagc gcgcaattaa ccctcactaa agggaacaaa agctgtcgag atctagatat 360
cgatggccat agagttacgc tagggataac agggtaatat agccgcggca ggccctccga 420
gcgtggtgga gccgttctgt gagacagccg ggtacgagtc gtgacgctgg aaggggcaag 480
cgggtggtgg gcaggaatgc ggtccgccct gcagcaaccg gagggggagg gagaagggag 540
cggaaaagtc tccaccggac gcggccatgg ctcggggggg ggggggcagc ggaggagcgc 600
ttccggccga cgtctcgtcg ctgattggct tcttttcctc ccgccgtgtg tgaaaacaca 660
aatggcgtgt tttggttggc gtaaggcgcc tgtcagttaa cggcagccgg agtgcgcagc 720
cgccggcagc ctcgctctgc ccactgggtg gggcgggagg taggtggggt gaggcgagct 780
ggacgtgcgg gcgcggtcgg cctctggcgg ggcgggggag gggagggagg gtcagcgaaa 840
gtagctcgcg cgcgagcggc cgcccaccct ccccttcctc tgggggagtc gttttacccg 900
ccgccggccg ggcctcgtcg tctgattggc tctcggggcc cagaaaactg gcccttgcca 960
ttggctcgtg ttcgtgcaag ttgagtccat ccgccggcca gcgggggcgg cgaggaggcg 1020
ctcccaggtt ccggccctcc cctcggcccc gcgccgcaga gtctggccgc gcgcccctgc 1080
gcaacgtggc aggaagcgcg cgctgggggc ggggacgggc agtagggctg agcggctgcg 1140
gggcgggtgc aagcacgttt ccgacttgag ttgcctcaag aggggcgtgc tgagccagac 1200
ctccatcgcg cactccgggg agtggaggga aggagcgagg gctcagttgg gctgttttgg 1260
aggcaggaag cacttgctct cccaaagtcg ctctgagttg ttatcagtaa gggagctgca 1320
gtggagtagg cggggagaag gccgcaccct tctccggagg ggggagggga gtgttgcaat 1380
acctttctgg gagttctctg ctgcctcctg gcttctgagg accgccctgg gcctgggaga 1440
atcccttccc cctcttccct cgtgatctgc aactccagtc tttctaggta accgatatcc 1500
ctgcaggggt gacctgcacg tctagggcgc agtagtccag ggtttccttg atgatgtcat 1560

acttatcctg tccctttttt ttccacagct cgcggttgag gacaaactct tcgcggtctt 1620
tccagtactc ctgcaggtga ctgactgagt cgacttaatt aaggccatag cggccattta 1680
aatcggccgg cctaggcgcg ccggtaaccg aagttcctat actttctaga gaataggaac 1740
ttcggaatag gaacttctta ggtcaattct accgggtagg ggaggcgctt ttcccaaggc 1800
agtctggagc atgcgcttta gcagccccgc tgggcacttg gcgctacaca agtggcctct 1860
ggcctcgcac acattccaca tccaccggta ggcgccaacc ggctccgttc tttggtggcc 1920
ccttcgcgcc accttctact cctcccctag tcaggaagtt cccccccgcc ccgcagctcg 1980
cgtcgtgcag gacgtgacaa atggaagtag cacgtctcac tagtctcgtg cagatggaca 2040
gcaccgctga gcaatggaag cgggtaggcc tttggggcag cggccaatag cagctttgct 2100
ccttcgcttt ctgggctcag aggctgggaa ggggtgggtc cgggggcggg ctcaggggcg 2160
ggctcagggg cggggcgggc gcccgaaggt cctccggagg cccggcattc tgcacgcttc 2220
aaaagcgcac gtctgccgcg ctgttctcct cttcctcatc tccgggcctt tcgacctgca 2280
gccaatatgg gatcggccat tgaacaagat ggattgcacg caggttctcc ggccgcttgg 2340
gtggagaggc tattcggcta tgactgggca caacagacaa tcggctgctc tgatgccgcc 2400
gtgttccggc tgtcagcgca ggggcgcccg gttctttttg tcaagaccga cctgtccggt 2460
gccctgaatg aactgcagga cgaggcagcg cggctatcgt ggctggccac gacgggcgtt 2520
ccttgcgcag ctgtgctcga cgttgtcact gaagcgggaa gggactggct gctattgggc 2580
gaagtgccgg ggcaggatct cctgtcatct caccttgctc ctgccgagaa agtatccatc 2640
atggctgatg caatgcggcg gctgcatacg cttgatccgg ctacctgccc attcgaccac 2700
caagcgaaac atcgcatcga gcgagcacgt actcggatgg aagccggtct tgtcgatcag 2760
gatgatctgg acgaagagca tcaggggctc gcgccagccg aactgttcgc caggctcaag 2820
gcgcgcatgc ccgacggcga ggatctcgtc gtgacccatg gcgatgcctg cttgccgaat 2880
atcatggtgg aaaatggccg cttttctgga ttcatcgact gtggccggct gggtgtggcg 2940

gaccgctatc aggacatagc gttggctacc cgtgatattg ctgaagagct tggcggcgaa 3000
tgggctgacc gcttcctcgt gctttacggt atcgccgctc ccgattcgca gcgcatcgcc 3060
ttctatcgcc ttcttgacga gttcttctga ggggatcgat ccgctgtaag tctgcagaaa 3120
ttgatgatct attaaacaat aaagatgtcc actaaaatgg aagtttttcc tgtcatactt 3180
tgttaagaag ggtgagaaca gagtacctac attttgaatg gaaggattgg agctacgggg 3240
gtgggggtgg ggtgggatta gataaatgcc tgctctttac tgaaggctct ttactattgc 3300
tttatgataa tgtttcatag ttggatatca taatttaaac aagcaaaacc aaattaaggg 3360
ccagctcatt cctcccactc atgatctata gatctataga tctctcgtgg gatcattgtt 3420
tttctcttga ttcccacttt gtggttctaa gtactgtggt ttccaaatgt gtcagtttca 3480
tagcctgaag aacgagatca gcagcctctg ttccacatac acttcattct cagtattgtt 3540
ttgccaagtt ctaattccat cagaagctga ctctagatcc cgcgccgaag ttcctatact 3600
ttctagagaa taggaacttc ggaataggaa cttcaagctt aagcgctaga agatgggcgg 3660
gagtcttctg ggcaggctta aaggctaacc tggtgtgtgg gcgttgtcct gcaggggaat 3720
tgaacaggtg taaaattgga gggacaagac ttcccacaga ttttcggttt tgtcgggaag 3780
ttttttaata ggggcaaata aggaaaatgg gaggataggt agtcatctgg ggttttatgc 3840
agcaaaacta caggttatta ttgcttgtga tccgcctcgg agtattttcc atcgaggtag 3900
attaaagaca tgctcacccg agttttatac tctcctgctt gagatcctta ctacagtatg 3960
aaattacagt gtcgcgagtt agactatgta agcagaattt taatcatttt taaagagccc 4020
agtacttcat atccatttct cccgctcctt ctgcagcctt atcaaaaggt attttagaac 4080
actcatttta gccccatttt catttattat actggcttat ccaaccccta gacagagcat 4140
tggcattttc cctttcctga tcttagaagt ctgatgactc atgaaaccag acagattagt 4200
tacatacacc acaaatcgag gctgtagctg gggcctcaac actgcagttc ttttataact 4260
ccttagtaca ctttttgttg atcctttgcc ttgatcctta attttcagtg tctatcacct 4320
ctcccgtcag tggtgttcca catttgggcc tattctcagt ccagggagtt ttacaacaat 4380
agatgtattg agaatccaac ctaaagctta actttccact cccatgaatg cctctctcct 4440
ttttctccat ttataaactg agctattaac cattaatggt tccaggtgga tgtctcctcc 4500
ccatattacc tgatgtatct tacatattgc caggctgata ttttaagaca ttaaaaggta 4560
tatttcatta ttgagccaca tggtattgat tactgcttac taaaattttg tcattgtaca 4620
catctgtaaa aggtggttcc ttttggaatg caaagttcag gtgtttgttg tctttcctga 4680
cctaaggtct tgtgagcttg tattttttct atttaagcag tgctttctct tggactggct 4740
tgactcatgg cattctacac gttattgctg gtctaaatgt gattttgcca agcttcttca 4800
ggacctataa ttttgcttga cttgtagcca aacacaagta aaatgattaa gcaacaaatg 4860
tatttgtgaa gcttggtttt taggttgttg tgttgtgtgt gcttgtgctc tataataata 4920
ctatccaggg gctggagagg tggctcggag ttcaagagca cagactgctc ttccagaagt 4980
cctgagttca attcccagca accacatggt ggctcacaac catctgtaat gggatctgat 5040
gccctcttct ggtgtgtctg aagaccacaa gtgtattcac attaaataaa taaatcctcc 5100
ttcttcttct tttttttttt tttaaagaga atactgtctc cagtagaatt tactgaagta 5160
atgaaatact ttgtgtttgt tccaatatgg tagccaataa tcaaattact ctttaagcac 5220
tggaaatgtt accaaggaac taatttttat ttgaagtgta actgtggaca gaggagccat 5280
aactgcagac ttgtgggata cagaagacca atgcagactt taatgtcttt tctcttacac 5340
taagcaataa agaaataaaa attgaacttc tagtatccta tttgtttaaa ctgctagctt 5400
tacttaactt ttgtgcttca tctatacaaa gctgaaagct aagtctgcag ccattactaa 5460
acatgaaagc aagtaatgat aattttggat ttcaaaaatg tagggccaga gtttagccag 5520
ccagtggtgg tgcttgcctt tatgccttta atcccagcac tctggaggca gagacaggca 5580
gatctctgag tttgagccca gcctggtcta cacatcaagt tctatctagg atagccagga 5640
atacacacag aaaccctgtt ggggaggggg gctctgagat ttcataaaat tataattgaa 5700
gcattcccta atgagccact atggatgtgg ctaaatccgt ctacctttct gatgagattt 5760
gggtattatt ttttctgtct ctgctgttgg ttgggtcttt tgacactgtg ggctttcttt 5820
aaagcctcct tcctgccatg tggtctcttg tttgctacta acttcccatg gcttaaatgg 5880
catggctttt tgccttctaa gggcagctgc tgagatttgc agcctgattt ccagggtggg 5940
gttgggaaat ctttcaaaca ctaaaattgt cctttaattt tttttttaaa aatggggtta 6000
tataataaac ctcataaaat agttatgagg agtgaggtgg actaatatta aatgagtccc 6060
tcccctataa aagagctatt aaggcttttt gtcttatact taactttttt tttaaatgtg 6120
gtatctttag aaccaagggt cttagagttt tagtatacag aaactgttgc atcgcttaat 6180
cagattttct agtttcaaat ccagagaatc caaattcttc acagccaaag tcaaattaag 6240
aatttctgac ttttaatgtt aatttgctta ctgtgaatat aaaaatgata gcttttcctg 6300
aggcagggtc tcactatgta tctctgcctg atctgcaaca agatatgtag actaaagttc 6360
tgcctgcttt tgtctcctga atactaaggt taaaatgtag taatactttt ggaacttgca 6420
ggtcagattc ttttataggg gacacactaa gggagcttgg gtgatagttg gtaaaatgtg 6480
tttcaagtga tgaaaacttg aattattatc accgcaacct actttttaaa aaaaaaagcc 6540
aggcctgtta gagcatgctt aagggatccc taggacttgc tgagcacaca agagtagtta 6600
cttggcaggc tcctggtgag agcatatttc aaaaaacaag gcagacaacc aagaaactac 6660
agttaaggtt acctgtcttt aaaccatctg catatacaca gggatattaa aatattccaa 6720

ataatatttc attcaagttt tcccccatca aattgggaca tggatttctc cggtgaatag 6780
gcagagttgg aaactaaaca aatgttggtt ttgtgatttg tgaaattgtt ttcaagtgat 6840
agttaaagcc catgagatac agaacaaagc tgctatttcg aggtctcttg gtttatactc 6900
agaagcactt ctttgggttt ccctgcacta tcctgatcat gtgctaggcc taccttaggc 6960
tgattgttgt tcaaataaac ttaagtttcc tgtcaggtga tgtcatatga tttcatatat 7020
caaggcaaaa catgttatat atgttaaaca tttgtactta atgtgaaagt taggtctttg 7080
tgggtttgat ttttaatttt caaaacctga gctaaataag tcatttttac atgtcttaca 7140
tttggtggaa ttgtataatt gtggtttgca ggcaagactc tctgacctag taaccctacc 7200
tatagagcac tttgctgggt cacaagtcta ggagtcaagc atttcacctt gaagttgaga 7260
cgttttgtta gtgtatacta gtttatatgt tggaggacat gtttatccag aagatattca 7320
ggactatttt tgactgggct aaggaattga ttctgattag cactgttagt gagcattgag 7380
tggcctttag gcttgaattg gagtcacttg tatatctcaa ataatgctgg ccttttttaa 7440
aaagcccttg ttctttatca ccctgttttc tacataattt ttgttcaaag aaatacttgt 7500
ttggatctcc ttttgacaac aatagcatgt tttcaagcca tatttttttt cctttttttt 7560
tttttttttg gtttttcgag acagggtttc tctgtatagc cctggctgtc ctggaactca 7620
ctttgtagac caggctggcc tcgaactcag aaatccgcct gcctctgcct cctgagtgcc 7680
gggattaaag gcgtgcacca ccacgcctgg ctaagttgga tattttgtta tataactata 7740
accaatacta actccactgg gtggattttt aattcagtca gtagtcttaa gtggtcttta 7800
ttggcccttc attaaaatct actgttcact ctaacagagg ctgttggtac tagtggcact 7860
taagcaactt cctacggata tactagcaga ttaagggtca gggatagaaa ctagtctagc 7920
gttttgtata cctaccagct ttatactacc ttgttctgat agaaatattt caggacatct 7980
agcacccaat tcgccctata gtgagtcgta ttacaattca ctggccgtcg ttttacaacg 8040
tcgtgactgg gaaaaccctg gcgttaccca acttaatcgc cttgcagcac atcccccttt 8100
cgccagctgg cgtaatagcg aagaggcccg caccgatcgc ccttcccaac agttgcgcag 8160
cctgaatggc gaatggacgc gccctgtagc ggcgcattaa gcgcggcggg tgtggtggtt 8220
acgcgcagcg tgaccgctac acttgccagc gccctagcgc ccgctccttt cgctttcttc 8280
ccttcctttc tcgccacgtt cgccggcttt ccccgtcaag ctctaaatcg ggggctccct 8340
ttagggttcc gatttagtgc tttacggcac ctcgacccca aaaaacttga ttagggtgat 8400
ggttcacgta gtgggccatc gccctgatag acggtttttc gccctttgac gttggagtcc 8460
acgttcttta atagtggact cttgttccaa actggaacaa cactcaaccc tatctcggtc 8520
tattcttttg atttataagg gattttgccg atttcggcct attggttaaa aaatgagctg 8580
atttaacaaa aatttaacgc gaattttaac aaaatattaa cgcttacaat ttaggtggca 8640
cttttcgggg aaatgtgcgc ggaaccccta tttgtttatt tttctaaata cattcaaata 8700
tgtatccgct catgagacaa taaccctgat aaatgcttca ataatattga aaaaggaaga 8760
gtatgagtat tcaacatttc cgtgtcgccc ttattccctt ttttgcggca ttttgccttc 8820
ctgtttttgc tcacccagaa acgctggtga aagtaaaaga tgctgaagat cagttgggtg 8880
cacgagtggg ttacatcgaa ctggatctca acagcggtaa gatccttgag agttttcgcc 8940
ccgaagaacg ttttccaatg atgagcactt ttaaagttct gctatgtggc gcggtattat 9000
cccgtattga cgccgggcaa gagcaactcg gtcgccgcat acactattct cagaatgact 9060
tggttgagta ctcaccagtc acagaaaagc atcttacgga tggcatgaca gtaagagaat 9120
tatgcagtgc tgccataacc atgagtgata acactgcggc caacttactt ctgacaacga 9180
tcggaggacc gaaggagcta accgcttttt tgcacaacat gggggatcat gtaactcgcc 9240

ttgatcgttg ggaaccggag ctgaatgaag ccataccaaa cgacgagcgt gacaccacga 9300
tgcctgtagc aatggcaaca acgttgcgca aactattaac tggcgaacta cttactctag 9360
cttcccggca acaattaata gactggatgg aggcggataa agttgcagga ccacttctgc 9420
gctcggccct tccggctggc tggtttattg ctgataaatc tggagccggt gagcgtgggt 9480
ctcgcggtat cattgcagca ctggggccag atggtaagcc ctcccgtatc gtagttatct 9540
acacgacggg gagtcaggca actatggatg aacgaaatag acagatcgct gagataggtg 9600
cctcactgat taagcattgg taactgtcag accaagttta ctcatatata ctttagattg 9660
atttaaaact tcatttttaa tttaaaagga tctaggtgaa gatccttttt gataatctca 9720
tgaccaaaat cccttaacgt gagttttcgt tccactgagc gtcagacccc gtagaaaaga 9780
tcaaaggatc ttcttgagat cctttttttc tgcgcgtaat ctgctgcttg caaacaaaaa 9840
aaccaccgct accagcggtg gtttgtttgc cggatcaaga gctaccaact ctttttccga 9900
aggtaactgg cttcagcaga gcgcagatac caaatactgt ccttctagtg tagccgtagt 9960
taggccacca cttcaagaac tctgtagcac cgcctacata cctcgctctg ctaatcctgt 10020
taccagtggc tgctgccagt ggcgataagt cgtgtcttac cgggttggac tcaagacgat 10080
agttaccgga taaggcgcag cggtcgggct gaacgggggg ttcgtgcaca cagcccagct 10140
tggagcgaac gacctacacc gaactgagat acctacagcg tgagctatga gaaagcgcca 10200
cgcttcccga agggagaaag gcggacaggt atccggtaag cggcagggtc ggaacaggag 10260
agcgcacgag ggagcttcca gggggaaacg cctggtatct ttatagtcct gtcgggtttc 10320
gccacctctg acttgagcgt cgatttttgt gatgctcgtc aggggggcgg agcctatgga 10380
aaaacgccag caacgcggcc tttttacggt tcctggcctt ttgctggcct tttgctcac 10440

atgttctttc ctgcgttatc ccctgattct gtggataacc gtattaccgc c          10491

<210> 11
<211> 15200
<212> DNA
<213> Artificial Sequence

<220>
<223> Description of Artificial Sequence: Rosa26
      targeting vector

<400> 11
ttacgctagg gataacaggg taatatagcc gcggcaggcc ctccgagcgt ggtggagccg 60
ttctgtgaga cagccgggta cgagtcgtga cgctggaagg ggcaagcggg tggtgggcag 120
gaatgcggtc cgccctgcag caaccggagg gggagggaga agggagcgga aaagtctcca 180
ccggacgcgg ccatggctcg gggggggggg ggcagcggag gagcgcttcc ggccgacgtc 240
tcgtcgctga ttggcttctt ttcctcccgc cgtgtgtgaa aacacaaatg gcgtgttttg 300
gttggcgtaa ggcgcctgtc agttaacggc agccggagtg cgcagccgcc ggcagcctcg 360
ctctgcccac tgggtggggc gggaggtagg tggggtgagg cgagctggac gtgcgggcgc 420
ggtcggcctc tggcggggcg ggggagggga gggagggtca gcgaaagtag ctcgcgcgcg 480
agcggccgcc caccctcccc ttcctctggg ggagtcgttt tacccgccgc cggccgggcc 540
tcgtcgtctg attggctctc ggggcccaga aaactggccc ttgccattgg ctcgtgttcg 600
tgcaagttga gtccatccgc cggccagcgg gggcggcgag gaggcgctcc caggttccgg 660
ccctcccctc ggccccgcgc cgcagagtct ggccgcgcgc ccctgcgcaa cgtggcagga 720
agcgcgcgct gggggcgggg acgggcagta gggctgagcg gctgcggggc gggtgcaagc 780
acgtttccga cttgagttgc ctcaagaggg gcgtgctgag ccagacctcc atcgcgcact 840
ccggggagtg gagggaagga gcgagggctc agttgggctg ttttggaggc aggaagcact 900
tgctctccca aagtcgctct gagttgttat cagtaaggga gctgcagtgg agtaggcggg 960
gagaaggccg cacccttctc cggagggggg aggggagtgt tgcaatacct ttctgggagt 1020
tctctgctgc ctcctggctt ctgaggaccg ccctgggcct gggagaatcc cttccccctc 1080
ttccctcgtg atctgcaact ccagtctttc taggtaaccg atatccctgc aggggtgacc 1140
tgcacgtcta gggcgcagta gtccagggtt tccttgatga tgtcatactt atcctgtccc 1200
ttttttttcc acagctcgcg gttgaggaca aactcttcgc ggtctttcca gtactcctgc 1260
aggtgactga ctgagtcgac gccaccatgg agaagttcct attccgaagt tcctattctt 1320
caaatagtat aggaacttcg ctcgagctca agcttcgaat tctgcagtcg acggtaccgc 1380
gggcccggga tccaccggtc gccaccatgg cccagtccaa gcacggcctg accaaggaga 1440
tgaccatgaa gtaccgcatg gagggctgcg tggacggcca caagttcgtg atcaccggcg 1500
agggcatcgg ctaccccttc aagggcaagc aggccatcaa cctgtgcgtg gtggagggcg 1560
gccccttgcc cttcgccgag gacatcttgt ccgccgcctt catgtacggc aaccgcgtgt 1620
tcaccgagta cccccaggac atcgtcgact acttcaagaa ctcctgcccc gccggctaca 1680
cctgggaccg ctccttcctg ttcgaggacg gcgccgtgtg catctgcaac gccgacatca 1740
ccgtgagcgt ggaggagaac tgcatgtacc acgagtccaa gttctacggc gtgaacttcc 1800
ccgccgacgg ccccgtgatg aagaagatga ccgacaactg ggagccctcc tgcgagaaga 1860
tcatccccgt gcccaagcag ggcatcttga agggcgacgt gagcatgtac ctgctgctga 1920
aggacggtgg ccgcttgcgc tgccagttcg acaccgtgta caaggccaag tccgtgcccc 1980
gcaagatgcc cgactggcac ttcatccagc acaagctgac ccgcgaggac cgcagcgacg 2040
ccaagaacca gaagtggcac ctgaccgagc acgccatcgc ctccggctcc gccttgccct 2100
gagcggccga ccggttcgag atccaggcgc ggatcaataa aagatcatta ttttcaatag 2160
atctgtgtgt tggttttttg tgtgccttgg gggaggggga ggccagaatg aggcgcggcc 2220
aaggggggagg gggaggccag aatgaccttg gggagggggg aggccagaat gaccttgggg 2280
gagggggagg ccagaatgag gcgcgtatgc aggtcaattc taccgggtag gggaggcgct 2340
tttcccaagg cagtctggag catgcgcttt agcagccccg ctggcacttg gcgctacaca 2400
agtggcctct ggcctcgcac acattccaca tccaccggta gcgccaaccg gctccgttct 2460
ttggtggccc cttcgcgcca ccttctactc ctcccctagt caggaagttc ccccccgccc 2520
cgcagctcgc gtcgtgcagg acgtgacaaa tggaagtagc acgtctcact agtctcgtgc 2580
agatggacag caccgctgag caatggaagc gggtaggcct ttggggcagc ggccaatagc 2640
agctttgctc cttcgctttc tgggctcaga ggctgggaag gggtgggtcc gggggcgggc 2700
tcaggggcgg gctcaggggc ggggcgggcg cgaaggtcct cccgaggccc ggcattctcg 2760
cacgcttcaa aagcgcacgt ctgccgcgct gttctcctct tcctcatctc cgggcctttc 2820
gacgatccag ccgccaccat gaaaaagcct gaactcaccg cgacgtctgt cgagaagttt 2880
ctgatcgaaa agttcgacag cgtctccgac ctgatgcagc tctcggaggg cgaagaatct 2940
cgtgctttca gcttcgatgt aggagggcgt ggatatgtcc tgcgggtaaa tagctgcgcc 3000

gatggtttct acaaagatcg ttatgtttat cggcactttg catcggccgc gctcccgatt 3060
ccggaagtgc ttgacattgg ggaattcagc gagagcctga cctattgcat ctcccgccgt 3120
gcacagggtg tcacgttgca agacctgcct gaaaccgaac tgcccgctgt tctgcagccg 3180
gtcgcggagg ccatggatgc catcgctgcg gccgatctta gccagacgag cgggttcggc 3240
ccattcggac cgcaaggaat cggtcaatac actacatggc gtgatttcat atgcgcgatt 3300
gctgatcccc atgtgtatca ctggcaaact gtgatggacg acaccgtcag tgcgtccgtc 3360
gcgcaggctc tcgatgagct gatgctttgg gccgaggact gccccgaagt ccggcacctc 3420
gtgcacgcgg atttcggctc caacaatgtc ctgacggaca atggccgcat aacagcggtc 3480
attgactgga gcgaggcgat gttcggggat tcccaatacg aggtcgccaa catcttcttc 3540
tggaggccgt ggttggcttg tatggagcag cagacgcgct acttcgagcg gaggcatccg 3600
gagcttgcag gatcgccgcg gctccgggcg tatatgctcc gcattggtct tgaccaactc 3660
tatcagagct tggttgacgg caatttcgat gatgcagctt gggcgcaggg tcgatgcgac 3720
gcaatcgtcc gatccggagc cgggactgtc gggcgtacac aaatcgcccg cagaagcgcg 3780
gccgtctgga ccgatggctg tgtagaagta ctcgccgata gtggaaaccg acgccccagc 3840
actcgtccga gggcaaagga atagtcgatg cagaaattga tgatctatta aacaataaag 3900
atgtccacta aaatggaagt ttttcctgtc atactttgtt aagaagggtg agaacagagt 3960
acctacattt tgaatggaag gattggagct acgggggtgg gggtggggtg ggattagata 4020
aatgcctgct ctttactgaa ggctctttac tattgcttta tgataatgtt tcatagttgg 4080
atatcataat ttaaacaagc aaaaccaaat taagggccag ctcattcctc ccactcatga 4140
tctatagatc tatagatctc tcgtgggatc attgtttttc tcttgattcc cactttgtgg 4200
ttctaagtac tgtggtttcc aaatgtgtca gtttcatagc ctgaagaacg agatcagcag 4260
cctctgttcc acatacactt cattctcagt attgttttgc caagttctaa ttccatcaga 4320
agctgactct agatcctgca ttaattaagg ccatagcggc catttgggct agcttgcatg 4380
cctgcaggtt ttcgacattg attattgact agttattaat agtaatcaat tacggggtca 4440
ttagttcata gcccatatat ggagttccgc gttacataac ttacggtaaa tggcccgcct 4500
ggctgaccgc ccaacgaccc ccgcccattg acgtcaataa tgacgtatgt tcccatagta 4560
acgccaatag ggactttcca ttgacgtcaa tgggtggact atttacggta aactgcccac 4620
ttggcagtac atcaagtgta tcatatgcca agtacgcccc ctattgacgt caatgacggt 4680
aaatggcccg cctggcatta tgcccagtac atgaccttat gggactttcc tacttggcag 4740
tacatctacg tattagtcat cgctattacc atgggtcgag gtgagcccca cgttctgctt 4800
cactctcccc atctcccccc cctccccacc cccaattttg tatttattta ttttttaatt 4860
attttgtgca gcgatggggg cggggggggg gggggcgcgc gccaggcggg gcggggcggg 4920
gcgaggggcg gggcggggcg aggcggagag gtgcggcggc agccaatcag agcggcgcgc 4980
tccgaaagtt tccttttatg gcgaggcggc ggcggcggcg gccctataaa aagcgaagcg 5040
cgcggcgggc gggagtcgct gcgttgcctt cgccccgtgc cccgctccgc gccgcctcgc 5100
gccgcccgcc ccggctctga ctgaccgcgt tactcccaca ggtgagcggg cgggacggcc 5160
cttctcctcc gggctgtaat tagcgcttgg tttaatgacg gctcgtttct tttctgtggc 5220
tgcgtgaaag ccttaaaggg ctccgggagg gccctttgtg cgggggggag cggctcgggg 5280
ggtgcgtgcg tgtgtgtgtg cgtggggagc gccgcgtgcg gcccgcgctg cccggcggct 5340
gtgagcgctg cgggcgcggc gcggggcttt gtgcgctccg cgtgtgcgcg aggggagcgc 5400
ggccgggggc ggtgccccgc ggtgcggggg ggctgcgagg ggaacaaagg ctgcgtgcgg 5460
ggtgtgtgcg tgggggggtg agcagggggt gtgggcgcgg cggtcgggct gtaacccccc 5520
cctgcacccc cctccccgag ttgctgagca cggcccggct tcgggtgcgg ggctccgtgc 5580
ggggcgtggc gcggggctcg ccgtgccggg cggggggtgg cggcaggtgg gggtgccggg 5640
cggggcgggg ccgcctcggg ccggggaggg ctcgggggag gggcgcggcg gccccggagc 5700
gccggcggct gtcgaggcgc ggcgagccgc agccattgcc ttttatggta atcgtgcgag 5760
agggcgcagg gacttccttt gtcccaaatc tggcggagcc gaaatctggg aggcgccgcc 5820
gcaccccctc tagcgggcgc gggcgaagcg gtgcggcgcc ggcaggaagg aaatgggcgg 5880
ggagggcctt cgtgcgtcgc cgcgccgccg tccccttctc catctccagc ctcggggctg 5940
ccgcaggggg acggctgcct tcggggggga cggggcaggg cggggttcgg cttctggcgt 6000
gtgaccggcg gctctagtaa gcgttggggt gagtactccc tctcaaaagc gggcatgact 6060
tctgcgctaa gattgtcagt ttccaaaaac gaggaggatt tgatattcac ctggcccgcg 6120
gtgatgcctt tgagggtggc cgcgtccatc tggtcagaaa agacaatctt tttgttgtca 6180
agcttgaggt gtggcaggct tgagatctgg ccatacactt gagtgacatt gacatccact 6240
ttgcctttct ctccacaggt gtccactccc agggcggccc tagagcctct gctaaccatg 6300
ttcatgcctt cttctttttc ctacagctcc tgggcaacgt gctggttgtt gtgctgtctc 6360
atcattttgg caaagaatta cccgccgcca ccatggctcc caagaagaag aggaaggtga 6420
tgagtcaatt tgatatatta tgtaaaacac cacctaaggt cctggttcgt cagtttgtgg 6480
aaaggtttga aagaccttca ggggaaaaaa tagcatcatg tgctgctgaa ctaacctatt 6540
tatgttggat gattactcat aacggaacag caatcaagag agccacattc atgagctata 6600
atactatcat aagcaattcg ctgagtttcg atattgtcaa caaatcactc cagtttaaat 6660
acaagacgca aaaagcaaca attctggaag cctcattaaa gaaattaatt cctgcttggg 6720
aatttacaat tattccttac aatggacaaa aacatcaatc tgatatcact gatattgtaa 6780

gtagtttgca attacagttc gaatcatcgg aagaagcaga taagggaaat agccacagta 6840
aaaaaatgct taaagcactt ctaagtgagg gtgaaagcat ctgggagatc actgagaaaa 6900
tactaaattc gtttgagtat acctcgagat ttacaaaaac aaaaacttta taccaattcc 6960
tcttcctagc tactttcatc aattgtggaa gattcagcga tattaagaac gttgatccga 7020
aatcatttaa attagtccaa aataagtatc tgggagtaat aatccagtgt ttagtgacag 7080
agacaaagac aagcgttagt aggcacatat acttctttag cgcaaggggt aggatcgatc 7140
cacttgtata tttggatgaa tttttgagga attctgaacc agtcctaaaa cgagtaaata 7200
ggaccggcaa ttcttcaagc aacaaacagg aataccaatt attaaaagat aacttagtca 7260
gatcgtacaa caaggctttg aagaaaaatg cgccttatcc aatctttgct ataaagaatg 7320
gcccaaaatc tcacattgga agacatttga tgacctcatt tctgtcaatg aagggcctaa 7380
cggagttgac taatgttgtg ggaaattgga gcgataagcg tgcttctgcc gtggccagga 7440
caacgtatac tcatcagata acagcaatac ctgatcacta cttcgcacta gtttctcggt 7500
actatgcata tgatccaata tcaaaggaaa tgatagcatt gaaggatgag actaatccaa 7560
ttgaggagtg gcagcatata gaacagctaa agggtagtgc tgaaggaagc atacgatacc 7620
ccgcatggaa tgggataata tcacaggagg tactagacta cctttcatcc tacataaata 7680
gacgcatata atgatctaga gtcgagttag gccgcgtcga cctcgagatc caggcgcgga 7740
tcaataaaag atcattattt tcaatagatc tgtgtgttgg ttttttgtgt gccttggggg 7800
aggggggaggc cagaatgagg cgcggccaag gggagggggg aggccagaat gaccttgggg 7860
gagggggagg ccagaatgac cttgggggag ggggaggcca gaatgaggcg cgccggtaac 7920
cgaagttcct atactttcta gagaatagga acttcggaat aggaacttca agcttaagcg 7980
ctagaagatg ggcgggagtc ttctgggcag gcttaaaggc taacctggtg tgtgggcgtt 8040
gtcctgcagg ggaattgaac aggtgtaaaa ttggagggac aagacttccc acagattttc 8100
ggttttgtcg ggaagttttt taataggggc aaataaggaa aatgggagga taggtagtca 8160
tctggggttt tatgcagcaa aactacaggt tattattgct tgtgatccgc ctcggagtat 8220
tttccatcga ggtagattaa agacatgctc acccgagttt tatactctcc tgcttgagat 8280
ccttactaca gtatgaaatt acagtgtcgc gagttagact atgtaagcag aattttaatc 8340
atttttaaag agcccagtac ttcatatcca tttctcccgc tccttctgca gccttatcaa 8400
aaggtatttt agaacactca ttttagcccc attttcattt attatactgg cttatccaac 8460
ccctagacag agcattggca ttttcccttt cctgatctta gaagtctgat gactcatgaa 8520
accagacaga ttagttacat acaccacaaa tcgaggctgt agctggggcc tcaacactgc 8580
agttctttta taactcctta gtacactttt tgttgatcct ttgccttgat ccttaatttt 8640
cagtgtctat cacctctccc gtcagtggtg ttccacattt gggcctattc tcagtccagg 8700
gagttttaca acaatagatg tattgagaat ccaacctaaa gcttaacttt ccactcccat 8760
gaatgcctct ctcctttttc tccatttata aactgagcta ttaaccatta atggttccag 8820
gtggatgtct cctccccata ttacctgatg tatcttacat attgccaggc tgatatttta 8880
agacattaaa aggtatattt cattattgag ccacatggta ttgattactg cttactaaaa 8940
ttttgtcatt gtacacatct gtaaaaggtg gttccttttg gaatgcaaag ttcaggtgtt 9000
tgttgtcttt cctgacctaa ggtcttgtga gcttgtattt tttctattta agcagtgctt 9060
tctcttggac tggcttgact catggcattc tacacgttat tgctggtcta aatgtgattt 9120
tgccaagctt cttcaggacc tataattttg cttgacttgt agccaaacac aagtaaaatg 9180
attaagcaac aaatgtattt gtgaagcttg gtttttaggt tgttgtgttg tgtgtgcttg 9240
tgctctataa taatactatc caggggctgg agaggtggct cggagttcaa gagcacagac 9300
tgctcttcca gaagtcctga gttcaattcc cagcaaccac atggtggctc acaaccatct 9360
gtaatgggat ctgatgccct cttctggtgt gtctgaagac cacaagtgta ttcacattaa 9420
ataaataaat cctccttctt cttctttttt ttttttttaa agagaatact gtctccagta 9480
gaatttactg aagtaatgaa atactttgtg tttgttccaa tatggtagcc aataatcaaa 9540
ttactcttta agcactggaa atgttaccaa ggaactaatt tttatttgaa gtgtaactgt 9600
ggacagagga gccataactg cagacttgtg ggatacagaa gaccaatgca gactttaatg 9660
tcttttctct tacactaagc aataaagaaa taaaaattga acttctagta tcctatttgt 9720
ttaaactgct agctttactt aacttttgtg cttcatctat acaaagctga aagctaagtc 9780
tgcagccatt actaaacatg aaagcaagta atgataattt tggatttcaa aatgtagggg 9840
ccagagttta gccagccagt ggtggtgctt gcctttatgc ctttaatccc agcactctgg 9900
aggcagagac aggcagatct ctgagtttga gcccagcctg gtctacacat caagttctat 9960
ctaggatagc caggaataca cacagaaacc ctgttgggga ggggggctct gagatttcat 10020
aaaattataa ttgaagcatt ccctaatgag ccactatgga tgtggctaaa tccgtctacc 10080
tttctgatga gatttgggta ttattttttc tgtctctgct gttggttggg tcttttgaca 10140
ctgtgggctt tctttaaagc ctccttcctg ccatgtggtc tcttgtttgc tactaacttc 10200
ccatggctta aatggcatgg ctttttgcct tctaagggca gctgctgaga tttgcagcct 10260
gatttccagg gtggggttgg gaaatctttc aaacactaaa attgtccttt aatttttttt 10320
ttaaaaaatg ggttatataa taaacctcat aaaatagtta tgaggagtga ggtggactaa 10380
tattaaatga gtccctcccc tataaaagag ctattaaggc tttttgtctt atacttaact 10440
ttttttttaa atgtggtatc tttagaacca agggtcttag agttttagta tacagaaact 10500
gttgcatcgc ttaatcagat tttctagttt caaatccaga gaatccaaat tcttcacagc 10560

caaagtcaaa ttaagaattt ctgactttta atgttaattt gcttactgtg aatataaaaa 10620
tgatagcttt tcctgaggca gggtctcact atgtatctct gcctgatctg caacaagata 10680
tgtagactaa agttctgcct gcttttgtct cctgaatact aaggttaaaa tgtagtaata 10740
cttttggaac ttgcaggtca gattctttta taggggacac actaagggag cttgggtgat 10800
agttggtaaa atgtgtttca agtgatgaaa acttgaatta ttatcaccgc aacctacttt 10860
ttaaaaaaaa aagccaggcc tgttagagca tgcttaaggg atccctagga cttgctgagc 10920
acacaagagt agttacttgg caggctcctg gtgagagcat atttcaaaaa acaaggcaga 10980
caaccaagaa actacagtta aggttacctg tctttaaacc atctgcatat acacagggat 11040
attaaaatat tccaaataat atttcattca agttttcccc catcaaattg ggacatggat 11100
ttctccggtg aataggcaga gttggaaact aaacaaatgt tggttttgtg atttgtgaaa 11160
ttgttttcaa gtgatagtta aagcccatga gatacagaac aaagctgcta tttcgaggtc 11220
tcttggttta tactcagaag cacttctttg ggtttccctg cactatcctg atcatgtgct 11280
aggcctacct taggctgatt gttgttcaaa taaacttaag tttcctgtca ggtgatgtca 11340
tatgatttca tatatcaagg caaaacatgt tatatatgtt aaacatttgt acttaatgtg 11400
aaagttaggt ctttgtgggt ttgattttta attttcaaaa cctgagctaa ataagtcatt 11460
tttacatgtc ttacatttgg tggaattgta taattgtggt ttgcaggcaa gactctctga 11520
cctagtaacc ctacctatag agcactttgc tgggtcacaa gtctaggagt caagcatttc 11580
accttgaagt tgagacgttt tgttagtgta tactagttta tatgttggag gacatgttta 11640
tccagaagat attcaggact atttttgact gggctaagga attgattctg attagcactg 11700
ttagtgagca ttgagtggcc tttaggcttg aattggagtc acttgtatat ctcaaataat 11760
gctggccttt tttaaaaagc ccttgttctt tatcaccctg ttttctacat aatttttgtt 11820
caaagaaata cttgtttgga tctccttttg acaacaatag catgttttca agccatattt 11880
tttttccttt tttttttttt ttttggtttt tcgagacagg gtttctctgt atagccctgg 11940
ctgtcctgga actcactttg tagaccaggc tggcctcgaa ctcagaaatc cgcctgcctc 12000
tgcctcctga gtgccgggat taaaggcgtg caccaccacg cctggctaag ttggatattt 12060
tgttatataa ctataaccaa tactaactcc actgggtgga tttttaattc agtcagtagt 12120
cttaagtggt ctttattggc ccttcattaa aatctactgt tcactctaac agaggctgtt 12180
ggtactagtg gcacttaagc aacttcctac ggatatacta gcagattaag ggtcagggat 12240
agaaactagt ctagcgtttt gtatacctac cagctttata ctaccttgtt ctgatagaaa 12300
tatttcagga catctagcac ccaattcgcc ctatagtgag tcgtattaca attcactggc 12360
cgtcgtttta caacgtcgtg actgggaaaa ccctggcgtt acccaactta atcgccttgc 12420
agcacatccc cctttcgcca gctggcgtaa tagcgaagag gcccgcaccg atcgcccttc 12480
ccaacagttg cgcagcctga atggcgaatg ggacgcgccc tgtagcggcg cattaagcgc 12540
ggcgggtgtg gtggttacgc gcagcgtgac cgctacactt gccagcgccc tagcgcccgc 12600
tcctttcgct ttcttccctt cctttctcgc cacgttcgcc ggctttcccc gtcaagctct 12660
aaatcggggg ctccctttag ggttccgatt tagtgcttta cggcacctcg accccaaaaa 12720
acttgattag ggtgatggtt cacgtagtgg gccatcgccc tgatagacgg tttttcgccc 12780
tttgacgttg gagtccacgt tctttaatag tggactcttg ttccaaactg gaacaacact 12840
caaccctatc tcggtctatt cttttgattt ataagggatt ttgccgattt cggcctattg 12900
gttaaaaaat gagctgattt aacaaaaatt taacgcgaat tttaacaaaa tattaacgct 12960
tacaatttag gtggcacttt tcggggaaat gtgcgcggaa cccctatttg tttatttttc 13020
taaatacatt caaatatgta tccgctcatg agacaataac cctgataaat gcttcaataa 13080
tattgaaaaa ggaagagtat gagtattcaa catttccgtg tcgcccttat tccctttttt 13140
gcggcatttt gccttcctgt ttttgctcac ccagaaacgc tggtgaaagt aaaagatgct 13200
gaagatcagt tgggtgcacg agtgggttac atcgaactgg atctcaacag cggtaagatc 13260
cttgagagtt ttcgccccga agaacgtttt ccaatgatga gcacttttaa agttctgcta 13320
tgtggcgcgg tattatcccg tattgacgcc gggcaagagc aactcggtcg ccgcatacac 13380
tattctcaga atgacttggt tgagtactca ccagtcacag aaaagcatct tacggatggc 13440
atgacagtaa gagaattatg cagtgctgcc ataaccatga gtgataacac tgcggccaac 13500
ttacttctga caacgatcgg aggaccgaag gagctaaccg cttttttgca caacatgggg 13560
gatcatgtaa ctcgccttga tcgttgggaa ccggagctga atgaagccat accaaacgac 13620
gagcgtgaca ccacgatgcc tgtagcaatg gcaacaacgt tgcgcaaact attaactggc 13680
gaactactta ctctagcttc ccggcaacaa ttaatagact ggatggaggc ggataaagtt 13740
gcaggaccac ttctgcgctc ggcccttccg gctggctggt ttattgctga taaatctgga 13800
gccggtgagc gtgggtctcg cggtatcatt gcagcactgg ggccagatgg taagccctcc 13860
cgtatcgtag ttatctacac gacggggagt caggcaacta tggatgaacg aaatagacag 13920
atcgctgaga taggtgcctc actgattaag cattggtaac tgtcagacca agtttactca 13980
tatatacttt agattgattt aaaacttcat ttttaattta aaaggatcta ggtgaagatc 14040
ctttttgata atctcatgac caaaatccct taacgtgagt tttcgttcca ctgagcgtca 14100
gaccccgtag aaaagatcaa aggatcttct tgagatcctt tttttctgcg cgtaatctgc 14160
tgcttgcaaa caaaaaaacc accgctacca gcggtggttt gtttgccgga tcaagagcta 14220
ccaactcttt ttccgaaggt aactggcttc agcagagcgc agataccaaa tactgtcctt 14280
ctagtgtagc cgtagttagg ccaccacttc aagaactctg tagcaccgcc tacatacctc 14340

gctctgctaa tcctgttacc agtggctgct gccagtggcg ataagtcgtg tcttaccggg 14400
ttggactcaa gacgatagtt accggataag gcgcagcggt cgggctgaac ggggggttcg 14460
tgcacacagc ccagcttgga gcgaacgacc tacaccgaac tgagatacct acagcgtgag 14520
ctatgagaaa gcgccacgct tcccgaaggg agaaaggcgg acaggtatcc ggtaagcggc 14580
agggtcggaa caggagagcg cacgagggag cttccagggg gaaacgcctg gtatctttat 14640
agtcctgtcg ggtttcgcca cctctgactt gagcgtcgat ttttgtgatg ctcgtcaggg 14700
gggcggagcc tatggaaaaa cgccagcaac gcggcctttt tacggttcct ggccttttgc 14760
tggccttttg ctcacatgtt ctttcctgcg ttatcccctg attctgtgga taaccgtatt 14820
accgcctttg agtgagctga taccgctcgc cgcagccgaa cgaccgagcg cagcgagtca 14880
gtgagcgagg aagcggaaga gcgcccaata cgcaaaccgc ctctccccgc gcgttggccg 14940
attcattaat gcagctggca cgacaggttt cccgactgga aagcgggcag tgagcgcaac 15000
gcaattaatg tgagttagct cactcattag gcaccccagg ctttacactt tatgcttccg 15060
gctcgtatgt tgtgtggaat tgtgagcgga taacaatttc acacaggaaa cagctatgac 15120
catgattacg ccaagcgcgc aattaaccct cactaaaggg aacaaaagct gtcgagatct 15180
agatatcgat ggccatagag 15200

<210> 12
<211> 5070
<212> DNA
<213> Artificial Sequence

<220>
<223> Description of Artificial Sequence: Exchange
      vector

<400> 12
gggtaactaa gtaaggatcg agatccaggc gcggatcaat aaaagatcat tattttcaat 60
agatctgtgt gttggttttt tgtgtgcctt ggggggaggg gaggccagaa tgaggcgcgg 120
ccaaggggga gggggaggcc agaatgacct tgggggaggg ggaggccaga atgaccttgg 180
gggaggggga ggccagaatg aggcgcggat ccggagaagt tcctattccg aagttcctat 240
tcttcaaata gtataggaac ttcgctcgag ggatcggcca ttgaacaaga tggattgcac 300
gcaggttctc cggccgcttg ggtggagagg ctattcggct atgactgggc acaacagaca 360
atcggctgct ctgatgccgc cgtgttccgg ctgtcagcgc aggggcgccc ggttcttttt 420
gtcaagaccg acctgtccgg tgccctgaat gaactgcagg acgaggcagc gcggctatcg 480
tggctggcca cgacgggcgt tccttgcgca gctgtgctcg acgttgtcac tgaagcggga 540
agggactggc tgctattggg cgaagtgccg gggcaggatc tcctgtcatc tcaccttgct 600
cctgccgaga aagtatccat catggctgat gcaatgcggc ggctgcatac gcttgatccg 660
gctacctgcc cattcgacca ccaagcgaaa catcgcatcg agcgagcacg tactcggatg 720
gaagccggtc ttgtcgatca ggatgatctg gacgaagagc atcaggggct cgcgccagcc 780
gaactgttcg ccaggctcaa ggcgcgcatg cccgacggcg aggatctcgt cgtgacccat 840
ggcgatgcct gcttgccgaa tatcatggtg gaaaatggcc gcttttctgg attcatcgac 900
tgtggccggc tgggtgtggc ggaccgctat caggacatag cgttggctac ccgtgatatt 960
gctgaagagc ttggcggcga atgggctgac cgcttcctcg tgctttacgg tatcgccgct 1020
cccgattcgc agcgcatcgc cttctatcgc cttcttgacg agttcttctg aggggatcga 1080
tccgctgtaa gtctgcagaa attgatgatc tattaaacaa taaagatgtc cactaaaatg 1140
gaagtttttc ctgtcatact ttgttaagaa gggtgagaac agagtaccta cattttgaat 1200
ggaaggattg gagctacggg ggtgggggtg gggtgggatt agataaatgc ctgctcttta 1260
ctgaaggctc tttactattg ctttatgata atgtttcata gttggatatc ataatttaaa 1320
caagcaaaac caaattaagg gccagctcat tcctcccact catgatctat agatctatag 1380
atctctcgtg ggatcattgt ttttctcttg attcccactt tgtggttcta agtactgtgg 1440
tttccaaatg tgtcagtttc atagcctgaa gaacgagatc agcagcctct gttccacata 1500
cacttcattc tcagtattgt tttgccaagt tctaattcca tcagaagctg actctagatg 1560
gcgcgtatgc attaattaag gccatagcgg ccatttatcg gccggccata acttcgtata 1620
atgtatgcta tacgaagtta tggatcctca cagtaggtgg catcgttcct ttctgactgc 1680
ccgccccccg catgccgtcc cgcgatattg agctccgaac ctctcgccct gccgccgccg 1740
gtgctccgtc gccgccgcgc cgccatggaa ttcgaacgct gacgtcatca acccgctcca 1800
aggaatcgcg ggcccagtgt cactaggcgg gaacacccag cgcgcgtgcg ccctggcagg 1860
aagatggctg tgagggacag gggagtggcg ccctgcaata tttgcatgtc gctatgtgtt 1920
ctgggaaatc accataaacg tgaaatgtct ttggatttgg gaatcttata agttctgtat 1980
gagaccactc tttcccactg gcttctttct tcagtttcct tagcactggg aaattgagga 2040
aagaagccgg ttttttctag agactcgaga taacttcgta taatgtatgc tatacgaagt 2100
tatggcgcgt tgacattgat tattgactag aaatcggccg gcctaggcgc gccggtaacc 2160

gaagttccta tactttctag agaataggaa cttcggaata ggaacttcaa gccggtaccc 2220
agcttttgtt ccctttagtg agggttaatt tcgagcttgg cgtaatcatg gtcatagctg 2280
tttcctgtgt gaaattgtta tccgctcaca attccacaca acatacgagc cggaagcata 2340
aagtgtaaag cctggggtgc ctaatgagtg agctaactca cattaattgc gttgcgctca 2400
ctgcccgctt tccagtcggg aaacctgtcg tgccagctgc attaatgaat cggccaacgc 2460
gcggggagag gcggtttgcg tattgggcgc tcttccgctt cctcgctcac tgactcgctg 2520
cgctcggtcg ttcggctgcg gcgagcggta tcagctcact caaaggcggt aatacggtta 2580
tccacagaat caggggataa cgcaggaaag aacatgtgag caaaaggcca gcaaaaggcc 2640
aggaaccgta aaaaggccgc gttgctggcg tttttccata ggctccgccc ccctgacgag 2700
catcacaaaa atcgacgctc aagtcagagg tggcgaaacc cgacaggact ataaagatac 2760
caggcgtttc cccctggaag ctccctcgtg cgctctcctg ttccgaccct gccgcttacc 2820
ggatacctgt ccgcctttct cccttcggga agcgtggcgc tttctcatag ctcacgctgt 2880
aggtatctca gttcggtgta ggtcgttcgc tccaagctgg gctgtgtgca cgaacccccc 2940
gttcagcccg accgctgcgc cttatccggt aactatcgtc ttgagtccaa cccggtaaga 3000
cacgacttat cgccactggc agcagccact ggtaacagga ttagcagagc gaggtatgta 3060
ggcggtgcta cagagttctt gaagtggtgg cctaactacg gctacactag aaggacagta 3120
tttggtatct gcgctctgct gaagccagtt accttcggaa aaagagttgg tagctcttga 3180
tccggcaaac aaaccaccgc tggtagcggt ggtttttttg tttgcaagca gcagattacg 3240
cgcagaaaaa aaggatctca agaagatcct ttgatctttt ctacggggtc tgacgctcag 3300
tggaacgaaa actcacgtta agggattttg gtcatgagat tatcaaaaag gatcttcacc 3360
tagatccttt taaattaaaa atgaagtttt aaatcaatct aaagtatata tgagtaaact 3420
tggtctgaca gttaccaatg cttaatcagt gaggcaccta tctcagcgat ctgtctattt 3480
cgttcatcca tagttgcctg actccccgtc gtgtagataa ctacgatacg ggagggctta 3540
ccatctggcc ccagtgctgc aatgataccg cgagacccac gctcaccggc tccagattta 3600
tcagcaataa accagccagc cggaagggcc gagcgcagaa gtggtcctgc aactttatcc 3660
gcctccatcc agtctattaa ttgttgccgg gaagctagag taagtagttc gccagttaat 3720
agtttgcgca acgttgttgc cattgctaca ggcatcgtgg tgtcacgctc gtcgtttggt 3780
atggcttcat tcagctccgg ttcccaacga tcaaggcgag ttacatgatc ccccatgttg 3840
tgcaaaaaag cggttagctc cttcggtcct ccgatcgttg tcagaagtaa gttggccgca 3900
gtgttatcac tcatggttat ggcagcactg cataattctc ttactgtcat gccatccgta 3960
agatgctttt ctgtgactgg tgagtactca accaagtcat tctgagaata gtgtatgcgg 4020
cgaccgagtt gctcttgccc ggcgtcaata cgggataata ccgcgccaca tagcagaact 4080
ttaaaagtgc tcatcattgg aaaacgttct tcggggcgaa aactctcaag gatcttaccg 4140
ctgttgagat ccagttcgat gtaacccact cgtgcaccca actgatcttc agcatctttt 4200
actttcacca gcgtttctgg gtgagcaaaa acaggaaggc aaaatgccgc aaaaaaggga 4260
ataagggcga cacggaaatg ttgaatactc atactcttcc tttttcaata ttattgaagc 4320
atttatcagg gttattgtct catgagcgga tacatatttg aatgtattta gaaaaataaa 4380
caaatagggg ttccgcgcac atttccccga aaagtgccac ctaaattgta agcgttaata 4440
ttttgttaaa attcgcgtta aatttttgtt aaatcagctc attttttaac caataggccg 4500
aaatcggcaa aatcccttat aaatcaaaag aatagaccga gatagggttg agtgttgttc 4560
cagtttggaa caagagtcca ctattaaaga acgtggactc caacgtcaaa gggcgaaaaa 4620
ccgtctatca gggcgatggc ccactacgtg aaccatcacc ctaatcaagt tttttggggt 4680
cgaggtgccg taaagcacta aatcggaacc ctaaagggag cccccgattt agagcttgac 4740
ggggaaagcc ggcgaacgtg gcgagaaagg aagggaagaa agcgaaagga gcgggcgcta 4800
gggcgctggc aagtgtagcg gtcacgctgc gcgtaaccac cacacccgcc gcgcttaatg 4860
cgccgctaca gggcgcgtcc cattcgccat tcaggctgcg caactgttgg gaagggcgat 4920
cggtgcgggc ctcttcgcta ttacgccagc tggcgaaagg gggatgtgct gcaaggcgat 4980
taagttgggt aacgccaggg ttttcccagt cacgacgttg taaaacgacg gccagtgaat 5040
tgtaatacga ctcactatag ggcgaattgg 5070

<210> 13
<211> 47
<212> DNA
<213> Artificial Sequence

<220>
<223> Description of Artificial Sequence: DNA encoding
      an shRNA directed against  the CDH-1 gene

<400> 13
tgagaagtct cccagtcagt tcaagagact gactgggaga cttctca 47

<210> 14
<211> 47
<212> DNA
<213> Artificial Sequence

<220>
<223> Description of Artificial Sequence: DNA encoding
      an shRNA directed against the p53 gene

<400> 14
gactccagtg gtaatctact tcaagagagt agattaccac tggagtc                47


<210> 15
<211> 47
<212> DNA
<213> Artificial Sequence

<220>
<223> Description of Artificial Sequence: DNA encoding
      an shRNA directed against the CDC20 gene

<400> 15
cggcaggact ccgggccgat tcaagagatc ggcccggagt cctgccg                47


<210> 16
<211> 47
<212> DNA
<213> Artificial Sequence

<220>
<223> Description of Artificial Sequence: DNA encoding
      an shRNA directed against the CYLD gene

<400> 16
cctcatgcag ttctctttgt tcaagagaca aagagaactg catgagg                47


<210> 17
<211> 50
<212> DNA
<213> Artificial Sequence

<220>
<223> Description of Artificial Sequence: DNA encoding
      an shRNA directed against the RAS-GAP gene

<400> 17
aagatgaagc cactccctat ttcaagagaa aatagggagt ggcttcatct             50


<210> 18
<211> 41
<212> DNA
<213> Artificial Sequence

<220>
<223> Description of Artificial Sequence: DNA encoding
      an shRNA directed against the tubulin gene

<400> 18

gacagagcca agtggactca cagagtccac ttggctctgt c                    41

<210> 19
<211> 42
<212> DNA
<213> Artificial Sequence

<220>
<223> Description of Artificial Sequence: DNA encoding
      an shRNA directed against the lamin gene

<400> 19
ctggacttcc agaagaacat tcgtgttctt ctggaagtcc ag                   42

<210> 20
<211> 47
<212> DNA
<213> Artificial Sequence

<220>
<223> Description of Artificial Sequence: DNA encoding
      an shRNA directed against the gene for the
      ubiquitin carboxyl-terminal hydrolase 12

<400> 20
gagattggtc cagaacagtt tcaagagaac tgttctggac caatctc              47

<210> 21
<211> 47
<212> DNA
<213> Artificial Sequence

<220>
<223> Description of Artificial Sequence: DNA encoding
      an shRNA directed against the gene for  the
      ubiquitin carboxyl-terminal hydrolase 12

<400> 21
gcccttccga tcatggtagt tcaagagact accatgatcg gaagggc              47

<210> 22
<211> 47
<212> DNA
<213> Artificial Sequence

<220>
<223> Description of Artificial Sequence: DNA encoding
      an shRNA directed against the gene for the
      ubiquitin carboxyl-terminal hydrolase 12

<400> 22
tctttagaat tcttaagtat tcaagagata cttaagaatt ctaaaga              47

<210> 23
<211> 47
<212> DNA
<213> Artificial Sequence

<220>
<223> Description of Artificial Sequence: DNA encoding
      an shRNA directed against the gene of the
      ubiquitin carboxyl-terminal hydrolase

<400> 23
cattagctat atcaacatgt tcaagagaca tgttgatata gctaatg                47


<210> 24
<211> 47
<212> DNA
<213> Artificial Sequence

<220>
<223> Description of Artificial Sequence: DNA encoding
      an shRNA directed against the gene for the
      ubiquitin carboxyl-terminal hydrolase11

<400> 24
accacaaacg gcggaacgat tcaagagatc gttccgccgt ttgtggt                47


<210> 25
<211> 47
<212> DNA
<213> Artificial Sequence

<220>
<223> Description of Artificial Sequence: DNA encoding
      an shRNA directed against the gene for the
      ubiquitin carboxyl-terminal hydrolase 11

<400> 25
gagggtcttg gaggtcttct tcaagagaga agacctccaa gaccctc                47


<210> 26
<211> 47
<212> DNA
<213> Artificial Sequence

<220>
<223> Description of Artificial Sequence: DNA encoding
      an shRNA directed against the gene for the
      ubiquitin carboxyl-terminal hydrolase 11

<400> 26
gtccatgccc agccgtacat tcaagagatg tacggctggg catggac                47


<210> 27
<211> 47
<212> DNA
<213> Artificial Sequence

<220>
<223> Description of Artificial Sequence: DNA encoding
      an shRNA directed against the gene for the
      ubiquitin carboxyl-terminal hydrolase 11

<400> 27
gctggacacc ctcgtggagt tcaagagact ccacgagggt gtccagc                47

<210> 28
<211> 47
<212> DNA
<213> Artificial Sequence

<220>
<223> Description of Artificial Sequence: DNA encoding
      an shRNA directed against the gene for the
      ubiquitin carboxyl-terminal hydrolase 10

<400> 28
gaatatcaga gaattgagtt tcaagagaac tcaattctct gatattc                47


<210> 29
<211> 47
<212> DNA
<213> Artificial Sequence

<220>
<223> Description of Artificial Sequence: DNA encoding
      an shRNA directed against the gene for the
      ubiquitin carboxyl-terminal hydrolase 10

<400> 29
tggacttcat gaggaaatgt tcaagagaca tttcctcatg aagtcca                47


<210> 30
<211> 47
<212> DNA
<213> Artificial Sequence

<220>
<223> Description of Artificial Sequence: DNA encoding
      an shRNA directed against the  gene for the
      ubiquitin carboxyl-terminal hydrolase 10

<400> 30
tattgaatat cctgtggact tcaagagagt ccacaggata ttcaata                47


<210> 31
<211> 47
<212> DNA
<213> Artificial Sequence

<220>
<223> Description of Artificial Sequence: DNA encoding
      an shRNA directed against the gene for the
      ubiquitin carboxyl-terminal hydrolase 10

<400> 31
ttgtactgag agaaactgct tcaagagagc agtttctctc agtacaa                47


<210> 32
<211> 47
<212> DNA
<213> Artificial Sequence

<220>
<223> Description of Artificial Sequence: DNA encoding
      an shRNA directed against the HAUSP gene

<400> 32
gatcaatgat aggtttgaat tcaagagatt caaacctatc attgatc                47


<210> 33
<211> 47
<212> DNA
<213> Artificial Sequence

<220>
<223> Description of Artificial Sequence: DNA encoding
      an shRNA directed against the HAUSP gene

<400> 33
ggagtttgag aagtttaaat tcaagagatt taaacttctc aaactcc                47


<210> 34
<211> 47
<212> DNA
<213> Artificial Sequence

<220>
<223> Description of Artificial Sequence: DNA encoding
      an shRNA directed against the HAUSP gene

<400> 34
gaactcctcg cttgctgagt tcaagagact cagcaagcga ggagttc                47


<210> 35
<211> 47
<212> DNA
<213> Artificial Sequence

<220>
<223> Description of Artificial Sequence: DNA encoding
      an shRNA directed against the HAUSP gene

<400> 35
ccgaatttaa cagagagaat tcaagagatt ctctctgtta aattcgg                47


<210> 36
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 36
gacagcagaa gaatgcagat tcaagagatc tgcattcttc tgctgtc                47


<210> 37
<211> 47

<212> DNA
<213> Artificial Sequence

<220>
<223> Description of Artificial Sequence: DNA encoding
      an shRNA directed against the gene for the
      ubiquitin carboxyl-terminal hydrolase 8

<400> 37
ataaagctca acgagaacct tcaagagagg ttctcgttga gctttat                47


<210> 38
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 38
ggtgaagtgg cagaagaatt tcaagagaat tcttctgcca cttcacc                47


<210> 39
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 39
gtattgcagt aatcatcact tcaagagagt gatgattact gcaatac                47


<210> 40
<211> 47
<212> DNA
<213> Artificial Sequence

<220>
<223> Description of Artificial Sequence: DNA encoding
      an shRNA directed against the FLJ10785 gene

<400> 40
gatatggggt tccatgtcat tcaagagatg acatggaacc ccatatc                47


<210> 41
<211> 47
<212> DNA
<213> Artificial Sequence

<220>
<223> Description of Artificial Sequence: DNA encoding
      an shRNA directed against the FLJ10785 gene

<400> 41

ggagacatgg ttcttagtgt tcaagagaca ctaagaacca tgtctcc 47

<210> 42
<211> 47
<212> DNA
<213> Artificial Sequence

<220>
<223> Description of Artificial Sequence: DNA encoding
      an shRNA directed against the FLJ10785 gene

<400> 42
agcaccaagt tcgtctcagt tcaagagact gagacgaact tggtgct 47

<210> 43
<211> 47
<212> DNA
<213> Artificial Sequence

<220>
<223> Description of Artificial Sequence: DNA encoding
      an shRNA directed against the FLJ10785 gene

<400> 43
gatgcaacac tgaaagaact tcaagagagt tctttcagtg ttgcatc 47

<210> 44
<211> 47
<212> DNA
<213> Artificial Sequence

<220>
<223> Description of Artificial Sequence: DNA encoding
      an shRNA directed against the KIAA0710 gene

<400> 44
gtcaatggca gtgatgatat tcaagagata tcatcactgc cattgac 47

<210> 45
<211> 47
<212> DNA
<213> Artificial Sequence

<220>
<223> Description of Artificial Sequence: DNA encoding
      an shRNA directed against the KIAA0710 gene

<400> 45
cctgctagct gcctgtggct tcaagagagc cacaggcagc tagcagg 47

<210> 46
<211> 47
<212> DNA
<213> Artificial Sequence

<220>
<223> Description of Artificial Sequence: DNA encoding
      an shRNA directed against the KIAA0710 gene

<400> 46
ccacctttgc cagaaggagt tcaagagact ccttctggca aaggtgg                47

<210> 47
<211> 47
<212> DNA
<213> Artificial Sequence

<220>
<223> Description of Artificial Sequence: DNA encoding
      an shRNA directed against the KIAA0710 gene

<400> 47
ccctattgag gcaagtgtct tcaagagaga cacttgcctc aataggg                47

<210> 48
<211> 47
<212> DNA
<213> Artificial Sequence

<220>
<223> Description of Artificial Sequence: DNA encoding
      an shRNA directed against the genes
      FLJ12552/FLJ14256

<400> 48
gaaggaaaac ttgctgacgt tcaagagacg tcagcaagtt ttccttc                47

<210> 49
<211> 47
<212> DNA
<213> Artificial Sequence

<220>
<223> Description of Artificial Sequence: DNA encoding
      an shRNA directed against the FLJ12552/FLJ14256
      genes

<400> 49
ctcacctggg tccatgagat tcaagagatc tcatggaccc aggtgag                47

<210> 50
<211> 47
<212> DNA
<213> Artificial Sequence

<220>
<223> Description of Artificial Sequence: DNA encoding
      an shRNA directed against the FLJ12552/FLJ14256
      genes

<400> 50
gctgtcttac cgtgtggtct tcaagagaga ccacacggta agacagc                47

<210> 51
<211> 47
<212> DNA

<213> Artificial Sequence

<220>
<223> Description of Artificial Sequence: DNA encoding
      an shRNA directed against the FLJ12552/FLJ14256
      genes

<400> 51
cctggaccgc atgtatgact tcaagagagt catacatgcg gtccagg                47


<210> 52
<211> 47
<212> DNA
<213> Artificial Sequence

<220>
<223> Description of Artificial Sequence: DNA encoding
      an shRNA directed against the KIAA1203 gene

<400> 52
gtcaatggca gtgatgatat tcaagagata tcatcactgc cattgac                47


<210> 53
<211> 47
<212> DNA
<213> Artificial Sequence

<220>
<223> Description of Artificial Sequence: DNA encoding
      an shRNA directed against the KIAA1203 gene

<400> 53
cctgctagct gcctgtggct tcaagagagc cacaggcagc tagcagg                47


<210> 54
<211> 47
<212> DNA
<213> Artificial Sequence

<220>
<223> Description of Artificial Sequence: DNA encoding
      an shRNA directed against the KIAA1203 gene

<400> 54
ccacctttgc cagaaggagt tcaagagact ccttctggca aaggtgg                47


<210> 55
<211> 47
<212> DNA
<213> Artificial Sequence

<220>
<223> Description of Artificial Sequence: DNA encoding
      an shRNA directed against the KIAA1203 gene

<400> 55
ccctattgag gcaagtgtct tcaagagaga cacttgcctc aataggg                47

<210> 56
<211> 47
<212> DNA
<213> Artificial Sequence

<220>
<223> Description of Artificial Sequence: DNA encoding
      an shRNA directed against the FLJ23277 gene

<400> 56
ggaaatccga attgcttggt tcaagagacc aagcaattcg gatttcc                47


<210> 57
<211> 47
<212> DNA
<213> Artificial Sequence

<220>
<223> Description of Artificial Sequence: DNA encoding
      an shRNA directed against the FLJ23277 gene

<400> 57
cacatttctt caagtgtggt tcaagagacc acacttgaag aaatgtg                47


<210> 58
<211> 47
<212> DNA
<213> Artificial Sequence

<220>
<223> Description of Artificial Sequence: DNA encoding
      an shRNA directed against the FLJ23277 gene

<400> 58
cagcaggatg ctcaagaatt tcaagagaat tcttgagcat cctgctg                47


<210> 59
<211> 47
<212> DNA
<213> Artificial Sequence

<220>
<223> Description of Artificial Sequence: DNA encoding
      an shRNA directed against the FLJ23277 gene

<400> 59
gctgaatacc tacattggct tcaagagagc caatgtaggt attcagc                47


<210> 60
<211> 47
<212> DNA
<213> Artificial Sequence

<220>
<223> Description of Artificial Sequence: DNA encoding
      an shRNA directed against the FLJ14914 (similar to
      UBP4) gene

<400> 60

gggcttgtgc ctggccttgt tcaagagaca aggccaggca caagccc 47

<210> 61
<211> 47
<212> DNA
<213> Artificial Sequence

<220>
<223> Description of Artificial Sequence: DNA encoding
      an shRNA directed against the FLJ14914 (similar to
      UBP4) gene

<400> 61
gccttgtcct gccaagaagt tcaagagact tcttggcagg acaaggc 47

<210> 62
<211> 47
<212> DNA
<213> Artificial Sequence

<220>
<223> Description of Artificial Sequence: DNA encoding
      an shRNA directed against the FLJ14914 (similar to
      UBP4) gene

<400> 62
gattgaagcc aagggaacgt tcaagagacg ttcccttggc ttcaatc 47

<210> 63
<211> 47
<212> DNA
<213> Artificial Sequence

<220>
<223> Description of Artificial Sequence: DNA encoding
      an shRNA directed against the FLJ14914 (similar to
      UBP4) gene

<400> 63
tggcgcctgc tccccatctt tcaagagaag atggggagca ggcgcca 47

<210> 64
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 64
gaaccagcag gctctgtggt tcaagagacc acagagcctg ctggttc 47

<210> 65
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 65
ggaagcataa ttatctgcct tcaagagagg cagataatta tgcttcc                47


<210> 66
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 66
agaagaagat gcttttcact tcaagagagt gaaaagcatc ttcttct                47


<210> 67
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 67
cttgcagagg aggaacccat tcaagagatg ggttcctcct ctgcaag                47


<210> 68
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L3

<400> 68
gcaaacaatc agcaatgcct tcaagagagg cattgctgat tgtttgc                47


<210> 69
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L3

<400> 69

ttggactgat tcatgctatt tcaagagaat agcatgaatc agtccaa 47

<210> 70
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L3

<400> 70
ctggcaattc gttgatgtat tcaagagata catcaacgaa ttgccag 47

<210> 71
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L3

<400> 71
ttagatgggc ggaagccatt tcaagagaat ggcttccgcc catctaa 47

<210> 72
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L1

<400> 72
gaggagtctc tgggctcggt tcaagagacc gagcccagag actcctc 47

<210> 73
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L1

<400> 73
gagctgaagg gacaagaagt tcaagagact tcttgtccct tcagctc 47

<210> 74
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L1

<400> 74
tgtcgggtag atgacaaggt tcaagagacc ttgtcatcta cccgaca                47


<210> 75
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L1

<400> 75
cacagctgtt cttctgttct tcaagagaga acagaagaac agctgtg                47


<210> 76
<211> 47
<212> DNA
<213> Artificial Sequence

<220>
<223> Description of Artificial Sequence: DNA encoding
      an shRNA directed against the KIAA1891/FLJ25263
      genes

<400> 76
gtggaagcct ttacagatct tcaagagaga tctgtaaagg cttccac                47


<210> 77
<211> 47
<212> DNA
<213> Artificial Sequence

<220>
<223> Description of Artificial Sequence: DNA encoding
      an shRNA directed against the KIAA1891/FLJ25236
      genes

<400> 77
caacagctgc cttcatctgt tcaagagaca gatgaaggca gctgttg                47


<210> 78
<211> 47
<212> DNA
<213> Artificial Sequence

<220>
<223> Description of Artificial Sequence: DNA encoding
      an shRNA directed against the KIAA1891/FLJ25263
      genes

<400> 78

ccataggcag tcctcctaat tcaagagatt aggaggactg cctatgg 47

<210> 79
<211> 47
<212> DNA
<213> Artificial Sequence

<220>
<223> Description of Artificial Sequence: DNA encoding
      an shRNA directed against the KIAA1891/FLJ25263
      genes

<400> 79
tgtatcactg ccactggttt tcaagagaaa ccagtggcag tgataca 47

<210> 80
<211> 47
<212> DNA
<213> Artificial Sequence

<220>
<223> Description of Artificial Sequence: DNA encoding
      an shRNA directed against the FLJ14528 (similar to
      UBP8) gene

<400> 80
catgttgggc agctgcagct tcaagagagc tgcagctgcc caacatg 47

<210> 81
<211> 47
<212> DNA
<213> Artificial Sequence

<220>
<223> Description of Artificial Sequence: DNA encoding
      an shRNA directed against the FLJ14528 (similar to
      UBP8) gene

<400> 81
cacaactgga gacctgaagt tcaagagact tcaggtctcc agttgtg 47

<210> 82
<211> 47
<212> DNA
<213> Artificial Sequence

<220>
<223> Description of Artificial Sequence: DNA encoding
      an shRNA directed against the FLJ14528 (similar to
      UBP8) gene

<400> 82
gtatgcctcc aagaaagagt tcaagagact ctttcttgga ggcatac 47

<210> 83
<211> 47
<212> DNA
<213> Artificial Sequence

<220>
<223> Description of Artificial Sequence: DNA encoding
      an shRNA directed against the FLJ14528 (similar to
      UBP8) gene

<400> 83
cttcacagta catttctctt tcaagagaag agaaatgtac tgtgaag                47


<210> 84
<211> 47
<212> DNA
<213> Artificial Sequence

<220>
<223> Description of Artificial Sequence: DNA encoding
      an shRNA directed against the gene for the U4/U6
      TRI snRNP 65 kDa protein

<400> 84
gtactttcaa ggccggggtt tcaagagaac cccggccttg aaagtac                47


<210> 85
<211> 47
<212> DNA
<213> Artificial Sequence

<220>
<223> Description of Artificial Sequence: DNA encoding
      an shRNA directed against the gene for the U4/U6
      TRI snRNP 65 kDa protein

<400> 85
cttggacaag caagccaaat tcaagagatt tggcttgctt gtccaag                47


<210> 86
<211> 47
<212> DNA
<213> Artificial Sequence

<220>
<223> Description of Artificial Sequence: DNA encoding
      an shRNA directed against the gene for the U4/U6
      TRI snRNP 65 kDa protein

<400> 86
gactattgtg actgatgttt tcaagagaaa catcagtcac aatagtc                47


<210> 87
<211> 47
<212> DNA
<213> Artificial Sequence

<220>
<223> Description of Artificial Sequence: DNA encoding
      an shRNA directed against the gene for the U4/U6
      TRI snRNP 65kDa protein

<400> 87

ggagaacttt ctgaagcgct tcaagagagc gcttcagaaa gttctcc 47

<210> 88
<211> 47
<212> DNA
<213> Artificial Sequence

<220>
<223> Description of Artificial Sequence: DNA encoding
      an shRNA for the XM_089437 gene

<400> 88
gacgagagaa accttcacct tcaagagagg tgaaggtttc tctcgtc 47

<210> 89
<211> 47
<212> DNA
<213> Artificial Sequence

<220>
<223> Description of Artificial Sequence: DNA encoding
      an shRNA directed against the XM_089437 gene

<400> 89
acattattct acattctttt tcaagagaaa agaatgtaga ataatgt 47

<210> 90
<211> 47
<212> DNA
<213> Artificial Sequence

<220>
<223> Description of Artificial Sequence: DNA encoding
      an shRNA directed against the XM_089437 gene

<400> 90
agattcgcaa atggatgtat tcaagagata catccatttg cgaatct 47

<210> 91
<211> 47
<212> DNA
<213> Artificial Sequence

<220>
<223> Description of Artificial Sequence: DNA encoding
      an shRNA directed against the XM_089437 gene

<400> 91
cattcccacc atgagtctgt tcaagagaca gactcatggt gggaatg 47

<210> 92
<211> 47
<212> DNA
<213> Artificial Sequence

<220>
<223> Description of Artificial Sequence: DNA encoding
      an shRNA directed against the KIAA1453 gene

<400> 92
gatcgcccga cacttccgct tcaagagagc ggaagtgtcg ggcgatc                47

<210> 93
<211> 47
<212> DNA
<213> Artificial Sequence

<220>
<223> Description of Artificial Sequence: DNA encoding
      an shRNA directed against the KIAA1453 gene

<400> 93
ccagcaggcc tacgtgctgt tcaagagaca gcacgtaggc ctgctgg                47

<210> 94
<211> 47
<212> DNA
<213> Artificial Sequence

<220>
<223> Description of Artificial Sequence: DNA encoding
      an shRNA directed against the KIAA1453 gene

<400> 94
gccagctcct ccacagcact tcaagagagt gctgtggagg agctggc                47

<210> 95
<211> 47
<212> DNA
<213> Artificial Sequence

<220>
<223> Description of Artificial Sequence: DNA encoding
      an shRNA directed against the KIAA1453 gene

<400> 95
cgccgccaag tggagcagat tcaagagatc tgctccactt ggcggcg                47

<210> 96
<211> 47
<212> DNA
<213> Artificial Sequence

<220>
<223> Description of Artificial Sequence: DNA encoding
      an shRNA directed against the FLJ12697 gene

<400> 96
gaagatgccc atgaattcct tcaagagagg aattcatggg catcttc                47

<210> 97
<211> 47
<212> DNA
<213> Artificial Sequence

<220>

<223> Description of Artificial Sequence: DNA encoding
      an shRNA directed against the FLJ12697 gene

<400> 97
caaacaggct gcgccaggct tcaagagagc ctggcgcagc ctgtttg                47


<210> 98
<211> 47
<212> DNA
<213> Artificial Sequence

<220>
<223> Description of Artificial Sequence: DNA encoding
      an shRNA directed against the FLJ12697 gene

<400> 98
acggcctagc gcctgatggt tcaagagacc atcaggcgct aggccgt                47


<210> 99
<211> 47
<212> DNA
<213> Artificial Sequence

<220>
<223> Description of Artificial Sequence: DNA encoding
      an shRNA directed against the FLJ12697 gene

<400> 99
ctgtaacctc tctgatcggt tcaagagacc gatcagagag gttacag                47


<210> 100
<211> 47
<212> DNA
<213> Artificial Sequence

<220>
<223> Description of Artificial Sequence: DNA encoding
      an shRNA directed against the gene for the
      ubiquitin specific protease (USP18)

<400> 100
tctgtcagtc catcctggct tcaagagagc caggatggac tgacaga                47


<210> 101
<211> 47
<212> DNA
<213> Artificial Sequence

<220>
<223> Description of Artificial Sequence: DNA encoding
      an shRNA directed against the gene for the
      ubiquitin specific protease (USP18)

<400> 101
tgaagcgaga gtcttgtgat tcaagagatc acaagactct cgcttca                47


<210> 102
<211> 47

<212> DNA
<213> Artificial Sequence

<220>
<223> Description of Artificial Sequence: DNA encoding
      an shRNA directed against the gene for the
      ubiquitin specific protease (USP18)

<400> 102
gatggagtgc taatggaaat tcaagagatt tccattagca ctccatc                47


<210> 103
<211> 47
<212> DNA
<213> Artificial Sequence

<220>
<223> Description of Artificial Sequence: DNA encoding
      an shRNA directed against the gene for the
      ubiquitin specific protease (USP18)

<400> 103
ccttcagaga ttgacacgct tcaagagagc gtgtcaatct ctgaagg                47


<210> 104
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 104
cctgaccacg ttccgactgt tcaagagaca gtcggaacgt ggtcagg                47


<210> 105
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 105
gagttccttc gctgcctgat tcaagagatc aggcagcgaa ggaactc                47


<210> 106
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 106
gactgccttg ctgccttctt tcaagagaag aaggcagcaa ggcagtc                47

<210> 107
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 107
cgccgagggc tacgtactct tcaagagaga gtacgtagcc ctcggcg                47

<210> 108
<211> 47
<212> DNA
<213> Artificial Sequence

<220>
<223> Description of Artificial Sequence: DNA encoding
      an shRNA directed against the gene for the
      ubiquitin carboxyl-terminal hydrolase 24

<400> 108
ggcgagaaga aaggactgtt tcaagagaac agtcctttct tctcgcc                47

<210> 109
<211> 47
<212> DNA
<213> Artificial Sequence

<220>
<223> Description of Artificial Sequence: DNA encoding
      an shRNA directed against the gene for the
      ubiquitin carboxyl terminal hydrolase 24

<400> 109
ggacgagaat tgataaagat tcaagagatc tttatcaatt ctcgtcc                47

<210> 110
<211> 47
<212> DNA
<213> Artificial Sequence

<220>
<223> Description of Artificial Sequence: DNA encoding
      an shRNA directed against the gene for the
      ubiquitin carboxyl-terminal hydrolase 24

<400> 110
gcacgagaat ttgggaatct tcaagagaga ttcccaaatt ctcgtgc                47

<210> 111
<211> 47

<212> DNA
<213> Artificial Sequence

<220>
<223> Description of Artificial Sequence: DNA encoding
      an shRNA directed against the gene for the
      ubiquitin carboxyl-terminal hydrolase 24

<400> 111
ctacttcatg aaatattggt tcaagagacc aatatttcat gaagtag                47


<210> 112
<211> 47
<212> DNA
<213> Artificial Sequence

<220>
<223> Description of Artificial Sequence: DNA encoding
      an shRNA directed against the KIAA1594 gene

<400> 112
gataacagct tcttgtctat tcaagagata gacaagaagc tgttatc                47


<210> 113
<211> 47
<212> DNA
<213> Artificial Sequence

<220>
<223> Description of Artificial Sequence: DNA encoding
      an shRNA directed against the KIAA1594 gene

<400> 113
gagaatagga catcagggct tcaagagagc cctgatgtcc tattctc                47


<210> 114
<211> 47
<212> DNA
<213> Artificial Sequence

<220>
<223> Description of Artificial Sequence: DNA encoding
      an shRNA directed against the KIAA1594 gene

<400> 114
cttggaagac tgaacctgtt tcaagagaac aggttcagtc ttccaag                47


<210> 115
<211> 47
<212> DNA
<213> Artificial Sequence

<220>
<223> Description of Artificial Sequence: DNA encoding
      an shRNA directed against the KIAA1594 gene

<400> 115
caactccttt gtggatgcat tcaagagatg catccacaaa ggagttg                47

<210> 116
<211> 47
<212> DNA
<213> Artificial Sequence

<220>
<223> Description of Artificial Sequence: DNA encoding
      an shRNA directed against the KIAA1350 gene

<400> 116
gatgttgtct ccaaatgcat tcaagagatg catttggaga caacatc                47


<210> 117
<211> 47
<212> DNA
<213> Artificial Sequence

<220>
<223> Description of Artificial Sequence: DNA encoding
      an shRNA directed against the KIAA1350 gene

<400> 117
cgtggggact gtacctccct tcaagagagg gaggtacagt ccccacg                47


<210> 118
<211> 47
<212> DNA
<213> Artificial Sequence

<220>
<223> Description of Artificial Sequence: DNA encoding
      an shRNA direcetd against the KIAA1350 gene

<400> 118
gtacagcttc agaaccaagt tcaagagact tggttctgaa gctgtac                47


<210> 119
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 119
gatgatcttc agagagcaat tcaagagatt gctctctgaa gatcatc                47


<210> 120
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 120
ggaacatcgg aatttgcctt tcaagagaag gcaaattccg atgttcc                47

<210> 121
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 121
gagctagtga gggactcttt tcaagagaaa gagtccctca ctagctc                47

<210> 122
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 122
gcagggttct ttaaggcaat tcaagagatt gccttaaaga accctgc                47

<210> 123
<211> 47
<212> DNA
<213> Artificial Sequence

<220>
<223> Description of Artificial Sequence: DNA encoding
      an shRNA directed against the gene for the
      ubiquitin carboxyl-terminal hydrolase 16

<400> 123
tcgatgattc ctctgaaact tcaagagagt ttcagaggaa tcatcga                47

<210> 124
<211> 47
<212> DNA
<213> Artificial Sequence

<220>
<223> Description of Artificial Sequence: DNA encoding
      an shRNA directed against the gene for the
      ubiquitin carboxyl-terminal hydrolase 16

<400> 124
gataatggaa atattgaact tcaagagagt tcaatatttc cattatc                47

<210> 125
<211> 47

<212> DNA
<213> Artificial Sequence

<220>
<223> Description of Artificial Sequence: DNA encoding
      an shRNA directed against the gene for the
      ubiquitin carboxyl-terminal hydrolase 16

<400> 125
gttcttcatt taaatgatat tcaagagata tcatttaaat gaagaac                47


<210> 126
<211> 47
<212> DNA
<213> Artificial Sequence

<220>
<223> Description of Artificial Sequence: DNA encoding
      an shRNA directed against the gene for the
      ubiquitin carboxyl-terminal hydrolase 16

<400> 126
gttaacaaac acataaagtt tcaagagaac tttatgtgtt tgttaac                47


<210> 127
<211> 47
<212> DNA
<213> Artificial Sequence

<220>
<223> Description of Artificial Sequence: DNA encoding
      an shRNA directed against the USP9X gene

<400> 127
gttagagaag attcttcgtt tcaagagaac gaagaatctt ctctaac                47


<210> 128
<211> 47
<212> DNA
<213> Artificial Sequence

<220>
<223> Description of Artificial Sequence: DNA encoding
      an shRNA directed against the USP9X gene

<400> 128
gttgattgga caattaaact tcaagagagt ttaattgtcc aatcaac                47


<210> 129
<211> 47
<212> DNA
<213> Artificial Sequence

<220>
<223> Description of Artificial Sequence: DNA encoding
      an shRNA directed against the USP9X gene

<400> 129
ggttgatacc gtaaagcgct tcaagagagc gctttacggt atcaacc                47

<210> 130
<211> 47
<212> DNA
<213> Artificial Sequence

<220>
<223> Description of Artificial Sequence: DNA encoding
      an shRNA directed against the USP9X gene

<400> 130
gcaatgaaac gtccaatggt tcaagagacc attggacgtt tcattgc                47


<210> 131
<211> 47
<212> DNA
<213> Artificial Sequence

<220>
<223> Description of Artificial Sequence: DNA encoding
      an shRNA directed against the USP9Y gene

<400> 131
agctagagaa aattcttcgt tcaagagacg aagaattttc tctagct                47


<210> 132
<211> 47
<212> DNA
<213> Artificial Sequence

<220>
<223> Description of Artificial Sequence: DNA encoding
      an shRNA directed against the USP9Y gene

<400> 132
gatcctatat gatggatgat tcaagagatc atccatcata taggatc                47


<210> 133
<211> 47
<212> DNA
<213> Artificial Sequence

<220>
<223> Description of Artificial Sequence: DNA encoding
      an shRNA directed against the USP9Y gene

<400> 133
gttcttcttg tcagtgaaat tcaagagatt tcactgacaa gaagaac                47


<210> 134
<211> 47
<212> DNA
<213> Artificial Sequence

<220>
<223> Description of Artificial Sequence: DNA encoding
      an shRNA directed against the USP9Y gene

<400> 134
cttgagcttg agtgaccact tcaagagagt ggtcactcaa gctcaag                47

<210> 135
<211> 47
<212> DNA
<213> Artificial Sequence

<220>
<223> Description of Artificial Sequence: DNA encoding
      an shRNA direcetd against the gene for the
      ubiquitin carboxyl-terminal hydrolase 5

<400> 135
gaccggccag cgagtctact tcaagagagt agactcgctg gccggtc                47

<210> 136
<211> 47
<212> DNA
<213> Artificial Sequence

<220>
<223> Description of Artificial Sequence: DNA encoding
      an shRNA directed against the gene for the
      ubiquitin carboxyl-terminal hydrolase 5

<400> 136
ggacctgggc tacatctact tcaagagagt agatgtagcc caggtcc                47

<210> 137
<211> 47
<212> DNA
<213> Artificial Sequence

<220>
<223> Description of Artificial Sequence: DNA encoding
      an shRNA directed against the gene for the
      ubiquitin carboxyl-terminal hydrolase 5

<400> 137
ctctgtggtc caggtgctct tcaagagaga gcacctggac cacagag                47

<210> 138
<211> 47
<212> DNA
<213> Artificial Sequence

<220>
<223> Description of Artificial Sequence: DNA encoding
      an shRNA directed against the gene for the
      ubiquitin carboxyl-terminal hydrolase 5

<400> 138
gaccacacga tttgcctcat tcaagagatg aggcaaatcg tgtggtc                47

<210> 139
<211> 47
<212> DNA

<213> Artificial Sequence

<220>
<223> Description of Artificial Sequence: DNA encoding
      an shRNA directed against the gene for the
      ubiquitin carboxyl-terminal hydrolase 26

<400> 139
tggcttgttt attgaaggat tcaagagatc cttcaataaa caagcca                47


<210> 140
<211> 47
<212> DNA
<213> Artificial Sequence

<220>
<223> Description of Artificial Sequence: DNA encoding
      an shRNA directed against the gene for the
      ubiquitin carboxyl-terminal hydrolase 26

<400> 140
gtgaatttgg ggaagataat tcaagagatt atcttcccca aattcac                47


<210> 141
<211> 47
<212> DNA
<213> Artificial Sequence

<220>
<223> Description of Artificial Sequence: DNA encoding
      an shRNA directed against the gene for the
      ubiquitin carboxyl- terminal hydrolase 26

<400> 141
cgctatagct tgaatgagtt tcaagagaac tcattcaagc tatagcg                47


<210> 142
<211> 47
<212> DNA
<213> Artificial Sequence

<220>
<223> Description of Artificial Sequence: DNA encoding
      an shRNA directed against the gene for the
      ubiquitin carboxyl- terminal hydrolase 26

<400> 142
gatatcctgg ctccacacat tcaagagatg tgtggagcca ggatatc                47


<210> 143
<211> 47
<212> DNA
<213> Artificial Sequence

<220>
<223> Description of Artificial Sequence: DNA encoding
      an shRNA directed against the KIAA1097 gene

<400> 143

gagccagtcg gatgtagatt tcaagagaat ctacatccga ctggctc                47

<210> 144
<211> 47
<212> DNA
<213> Artificial Sequence

<220>
<223> Description of Artificial Sequence: DNA encoding
      an shRNA directed against the KIAA1097 gene

<400> 144
gtaaattctg aaggcgaatt tcaagagaat tcgccttcag aatttac                47

<210> 145
<211> 47
<212> DNA
<213> Artificial Sequence

<220>
<223> Description of Artificial Sequence: DNA encoding
      an shRNA directed against the KIAA1097 gene

<400> 145
gccctcctaa atcaggcaat tcaagagatt gcctgattta ggagggc                47

<210> 146
<211> 47
<212> DNA
<213> Artificial Sequence

<220>
<223> Description of Artificial Sequence: DNA encoding
      an shRNA directed against the KIAA1097 gene

<400> 146
gttgagaaat ggagtgaagt tcaagagact tcactccatt tctcaac                47

<210> 147
<211> 47
<212> DNA
<213> Artificial Sequence

<220>
<223> Description of Artificial Sequence: DNA encoding
      an shRNA directed against the gene for the
      ubiquitin specific protease (USP22) gene

<400> 147
gcttggaaaa tgcaaggcgt tcaagagacg ccttgcattt tccaagc                47

<210> 148
<211> 47
<212> DNA
<213> Artificial Sequence

<220>
<223> Description of Artificial Sequence: DNA encoding

an shRNA directed against the gene for the
      ubiquitin specific protease (USP22) gene

<400> 148
ctgcatcata gaccagatct tcaagagaga tctggtctat gatgcag                47

<210> 149
<211> 47
<212> DNA
<213> Artificial Sequence

<220>
<223> Description of Artificial Sequence: DNA encoding
      an shRNA directed against the gene for the
      ubiquitin specific protease (USP22) gene

<400> 149
gatcaccacg tatgtgtcct tcaagagagg acacatacgt ggtgatc                47

<210> 150
<211> 47
<212> DNA
<213> Artificial Sequence

<220>
<223> Description of Artificial Sequence: DNA encoding
      an shRNA directed against the gene for the
      ubiquitin specific protease 22 (USP22) gene

<400> 150
tgacaacaag tattccctgt tcaagagaca gggaatactt gttgtca                47

<210> 151
<211> 47
<212> DNA
<213> Artificial Sequence

<220>
<223> Description of Artificial Sequence: DNA encoding
      an shRNA directed against the gene for the
      ubiquitin specific processing protease 29

<400> 151
gaaatataag acagattcct tcaagagagg aatctgtctt atatttc                47

<210> 152
<211> 47
<212> DNA
<213> Artificial Sequence

<220>
<223> Description of Artificial Sequence: DNA encoding
      an shRNA directed against the gene for the
      ubiquitin specific processing protease 29

<400> 152
cccatcaagt ttagaggatt tcaagagaat cctctaaact tgatggg                47

<210> 153
<211> 47
<212> DNA
<213> Artificial Sequence

<220>
<223> Description of Artificial Sequence: DNA encoding
      an shRNA directed against the gene for the
      ubiquitin specific processing protease 29

<400> 153
ggtgtcccat gggaatatat tcaagagata tattcccatg ggacacc                47


<210> 154
<211> 47
<212> DNA
<213> Artificial Sequence

<220>
<223> Description of Artificial Sequence: DNA encoding
      an shRNA directed against the gene for the
      ubiquitin specific processing protease 29

<400> 154
gaatgccgac ctacaaagat tcaagagatc tttgtaggtc ggcattc                47


<210> 155
<211> 47
<212> DNA
<213> Artificial Sequence

<220>
<223> Description of Artificial Sequence: DNA encoding
      an shRNA directed against the CYLD gene

<400> 155
cagttatatt ctgtgatgtt tcaagagaac atcacagaat ataactg                47


<210> 156
<211> 47
<212> DNA
<213> Artificial Sequence

<220>
<223> Description of Artificial Sequence: DNA encoding
      an shRNA directed against the CYLD gene

<400> 156
gaggtgttgg ggacaaaggt tcaagagacc tttgtcccca acacctc                47


<210> 157
<211> 47
<212> DNA
<213> Artificial Sequence

<220>
<223> Description of Artificial Sequence: DNA encoding
      an shRNA directed against the CYLD gene

<400> 157
gtgggctcat tggctgaagt tcaagagact tcagccaatg agcccac                47

<210> 158
<211> 47
<212> DNA
<213> Artificial Sequence

<220>
<223> Description of Artificial Sequence: DNA encoding
      an shRNA directed against the CYLD gene

<400> 158
gagctactga ggacagaaat tcaagagatt tctgtcctca gtagctc                47

<210> 159
<211> 47
<212> DNA
<213> Artificial Sequence

<220>
<223> Description of Artificial Sequence: DNA encoding
      an shRNA directed against the gene for the
      ubiquitin carboxyl-terminal hydrolase 2

<400> 159
tcagcaggat gctcaggagt tcaagagact cctgagcatc ctgctga                47

<210> 160
<211> 47
<212> DNA
<213> Artificial Sequence

<220>
<223> Description of Artificial Sequence: DNA encoding
      an shRNA directed against the gene for  the
      ubiquitin carboxyl-terminal hydrolase 2

<400> 160
gaagttctcc atccagaggt tcaagagacc tctggatgga gaacttc                47

<210> 161
<211> 47
<212> DNA
<213> Artificial Sequence

<220>
<223> Description of Artificial Sequence: DNA encoding
      an shRNA directed against the gene for the
      ubiquitin carboxyl-terminal hydrolase 2

<400> 161
gccggtcccc accagcagct tcaagagagc tgctggtggg gaccggc                47

<210> 162
<211> 47
<212> DNA
<213> Artificial Sequence

<220>
<223> Description of Artificial Sequence: DNA encoding
      an shRNA directed against the gene for the
      ubiquitin carboxyl-terminal hydrolase 2

<400> 162
cactcgggag ttgagagatt tcaagagaat ctctcaactc ccgagtg                47


<210> 163
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 163
gcccttgggt ctgtttgact tcaagagagt caaacagacc caagggc                47


<210> 164
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 164
ctcaacacta aacagcaagt tcaagagact tgctgtttag tgttgag                47


<210> 165
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 165
gatttcattg gacagcatat tcaagagata tgctgtccaa tgaaatc                47


<210> 166
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 166

catggggcac caactaattt tcaagagaaa ttagttggtg ccccatg 47

<210> 167
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 167
ggtgtctctg cgggattgtt tcaagagaac aatcccgcag agacacc 47

<210> 168
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 168
agttcagtag gtgtagactt tcaagagaag tctacaccta ctgaact 47

<210> 169
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 169
gagttcctga agctcctcat tcaagagatg aggagcttca ggaactc 47

<210> 170
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 170
ggatttgctg ggggcaaggt tcaagagacc ttgcccccag caaatcc 47

<210> 171
<211> 47
<212> DNA
<213> Artificial Sequence

<220>
<223> Description of Artificial Sequence: DNA encoding
      an shRNA directed against the UBP-32.7 gene

<400> 171
ctcagaaagc caacattcat tcaagagatg aatgttggct ttctgag                47


<210> 172
<211> 47
<212> DNA
<213> Artificial Sequence

<220>
<223> Description of Artificial Sequence: DNA encoding
      an shRNA directed against the UBP-32.7 gene

<400> 172
cgcattgtaa taagaaggtt tcaagagaac cttcttatta caatgcg                47


<210> 173
<211> 47
<212> DNA
<213> Artificial Sequence

<220>
<223> Description of Artificial Sequence: DNA encoding
      an shRNA directed against the UBP-32.7 gene

<400> 173
gggaggaaaa tgcagaaatt tcaagagaat ttctgcattt tcctccc                47


<210> 174
<211> 47
<212> DNA
<213> Artificial Sequence

<220>
<223> Description of Artificial Sequence: DNA encoding
      an shRNA directed against the UBP-32.7 gene

<400> 174
ttacaaattt aggaaatact tcaagagagt atttcctaaa tttgtaa                47


<210> 175
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 175
gttatgaatt gatatgcagt tcaagagact gcatatcaat tcataac                47

<210> 176
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 176
gtgataacac aactaatggt tcaagagacc attagttgtg ttatcac                47


<210> 177
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 177
gtagaggaga gttctgaaat tcaagagatt tcagaactct cctctac                47


<210> 178
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 178
gcctctaatc ctgataaggt tcaagagacc ttatcaggat tagaggc                47


<210> 179
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 179
gatgatcttc aggctgccat tcaagagatg gcagcctgaa gatcatc                47


<210> 180
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 180
gtatggacaa gagcgttggt tcaagagacc aacgctcttg tccatac                47


<210> 181
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 181
cgaacccttc tggaacagtt tcaagagaac tgttccagaa gggttcg                47


<210> 182
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 182
gtggcatgaa gattatagtt tcaagagaac tataatcttc atgccac                47


<210> 183
<211> 47
<212> DNA
<213> Artificial Sequence

<220>
<223> Description of Artificial Sequence: DNA encoding
      an shRNA directed against the ubiquitin
      carboxyl-terminal hydrolase 14

<400> 183
ggtgaacaag gacagtatct tcaagagaga tactgtcctt gttcacc                47


<210> 184
<211> 47
<212> DNA
<213> Artificial Sequence

<220>
<223> Description of Artificial Sequence: DNA encoding
      an shRNA directed against the gene for the
      ubiquitin carboxyl-terminal hydrolase 14

<400> 184

gcaatagagg atgattctgt tcaagagaca gaatcatcct ctattgc                47

<210> 185
<211> 47
<212> DNA
<213> Artificial Sequence

<220>
<223> Description of Artificial Sequence: DNA encoding
      an shRNA directed against the gene for the
      ubiquitin carboxyl-terminal hydrolase 14

<400> 185
tctgtgaatg ccaaagttct tcaagagaga actttggcat tcacaga                47

<210> 186
<211> 47
<212> DNA
<213> Artificial Sequence

<220>
<223> Description of Artificial Sequence: DNA encoding
      an shRNA directed against the gene for the
      ubiquitin carboxyl-terminal hydrolase 14

<400> 186
cacaccaggg aaggtctagt tcaagagact agaccttccc tggtgtg                47

<210> 187
<211> 47
<212> DNA
<213> Artificial Sequence

<220>
<223> Description of Artificial Sequence: DNA encoding
      an shRNA directed against the DUB1 gene

<400> 187
gcaggaagat gcccatgaat tcaagagatt catgggcatc ttcctgc                47

<210> 188
<211> 47
<212> DNA
<213> Artificial Sequence

<220>
<223> Description of Artificial Sequence: DNA encoding
      an shRNA directed agains the DUB1 gene

<400> 188
gaatgtgcaa tatcctgagt tcaagagact caggatattg cacattc                47

<210> 189
<211> 47
<212> DNA
<213> Artificial Sequence

<220>

<223> Description of Artificial Sequence: DNA encoding
      an shRNA directed against the DUB1 gene

<400> 189
tggatgatgc caaggtcact tcaagagagt gaccttggca tcatcca                47


<210> 190
<211> 47
<212> DNA
<213> Artificial Sequence

<220>
<223> Description of Artificial Sequence: DNA encoding
      an shRNA directed against the DUB1 gene

<400> 190
gctccgtgct aaacctctct tcaagagaga gaggtttagc acggagc                47


<210> 191
<211> 47
<212> DNA
<213> Artificial Sequence

<220>
<223> Description of Artificial Sequence: DNA encoding
      an shRNA directed against the gene for the mouse
      USP27 homolog

<400> 191
gcctccacct caacagaggt tcaagagacc tctgttgagg tggaggc                47


<210> 192
<211> 47
<212> DNA
<213> Artificial Sequence

<220>
<223> Description of Artificial Sequence: DNA encoding
      an shRNA directed against the gene for the mouse
      USP27 homolog

<400> 192
ctgcatcata gaccaaatct tcaagagaga tttggtctat gatgcag                47


<210> 193
<211> 47
<212> DNA
<213> Artificial Sequence

<220>
<223> Description of Artificial Sequence: DNA encoding
      an shRNA directed against the gene for the mouse
      USP27 homolog

<400> 193
gatcactaca tacatttcct tcaagagagg aaatgtatgt agtgatc                47


<210> 194

<211> 47
<212> DNA
<213> Artificial Sequence

<220>
<223> Description of Artificial Sequence: DNA encoding
      an shRNA directed against the gene for the mouse
      USP27 homolog

<400> 194
gtaaagagag cagaatgaat tcaagagatt cattctgctc tctttac                47


<210> 195
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 195
cgcggggcgc agtggtatct tcaagagaga taccactgcg ccccgcg                47


<210> 196
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 196
cagaaggcag tggggaagat tcaagagatc ttccccactg ccttctg                47


<210> 197
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 197
gcctgggaga atcacaggtt tcaagagaac ctgtgattct cccaggc                47


<210> 198
<211> 47
<212> DNA
<213> Artificial Sequence

<220>
<223> Description of Artificial Sequence: DNA encoding
      an shRNA directed against the gene for the

ubiquitin carboxyl-terminal hydrolase 4

<400> 198
accagacaag gaaataccct tcaagagagg gtatttcctt gtctggt                47


<210> 199
<211> 47
<212> DNA
<213> Artificial Sequence

<220>
<223> Description of Artificial Sequence: DNA encoding
      an shRNA directed against the TRE-2 gene

<400> 199
cacatccacc acatcgacct tcaagagagg tcgatgtggt ggatgtg                47


<210> 200
<211> 47
<212> DNA
<213> Artificial Sequence

<220>
<223> Description of Artificial Sequence: DNA encoding
      an shRNA directed against the TRE-2 gene

<400> 200
gtcacaaccc aagaccatgt tcaagagaca tggtcttggg ttgtgac                47


<210> 201
<211> 47
<212> DNA
<213> Artificial Sequence

<220>
<223> Description of Artificial Sequence: DNA encoding
      an shRNA directed against the TRE-2 gene

<400> 201
ctcaacagga caaatcccat tcaagagatg ggatttgtcc tgttgag                47


<210> 202
<211> 47
<212> DNA
<213> Artificial Sequence

<220>
<223> Description of Artificial Sequence: DNA encoding
      an shRNA directed against the TRE-2 gene

<400> 202
tagatcaatt attgtggatt tcaagagaat ccacaataat tgatcta                47


<210> 203
<211> 47
<212> DNA
<213> Artificial Sequence

<220>
<223> Description of Artificial Sequence: DNA encoding
      an shRNA directed against the gene for the
      ubiquitin carboxyl-terminal hydrolase 15 (UNPH-2)

<400> 203
ggaacacctt attgatgaat tcaagagatt catcaataag gtgttcc                47


<210> 204
<211> 47
<212> DNA
<213> Artificial Sequence

<220>
<223> Description of Artificial Sequence: DNA encoding
      an shRNA directed against the gene for the
      ubiquitin carboxyl-terminal hydrolase 15 (UNPH-2)

<400> 204
ctttaacaga aattgtctct tcaagagaga gacaatttct gttaaag                47


<210> 205
<211> 47
<212> DNA
<213> Artificial Sequence

<220>
<223> Description of Artificial Sequence: DNA encoding
      an shRNA directed agaainst the gene for the
      ubiquitin carboxyl-terminal hydrolase 15 (UNPH-2)

<400> 205
cctatgcagt acaaagtggt tcaagagacc actttgtact gcatagg                47


<210> 206
<211> 47
<212> DNA
<213> Artificial Sequence

<220>
<223> Description of Artificial Sequence: DNA encoding
      an shRNA directed against the gene for the
      ubiquitin carboxyl-terminal hydrolase 15 (UNPH-2)

<400> 206
gatcttttct tgctttggat tcaagagatc caaagcaaga aaagatc                47


<210> 207
<211> 47
<212> DNA
<213> Artificial Sequence

<220>
<223> Description of Artificial Sequence: DNA encoding
      an shRNA directed against the KIAA1372 gene

<400> 207
cagcatcctt caggccttat tcaagagata aggcctgaag gatgctg                47

<210> 208
<211> 47
<212> DNA
<213> Artificial Sequence

<220>
<223> Description of Artificial Sequence: DNA encoding
      an shRNA directed against the KIAA1372 gene

<400> 208
gatagtgact cggatctgct tcaagagagc agatccgagt cactatc                47


<210> 208
<211> 47
<212> DNA
<213> Artificial Sequence

<220>
<223> Description of Artificial Sequence: DNA encoding
      an shRNA directed against the KIAA1372 gene

<400> 209
gacatcacag cccgggagtt tcaagagaac tcccgggctg tgatgtc                47


<210> 210
<211> 47
<212> DNA
<213> Artificial Sequence

<220>
<223> Description of Artificial Sequence: DNA encoding
      an shRNA directed against the KIAA1372 gene

<400> 210
ggacacagcc tatgtgctgt tcaagagaca gcacataggc tgtgtcc                47


<210> 211
<211> 47
<212> DNA
<213> Artificial Sequence

<220>
<223> Description of Artificial Sequence: DNA encoding
      an shRNA directed against the gene for the BRCA1
      associated protein-1

<400> 211
gtggaggaga tctacgacct tcaagagagg tcgtagatct cctccac                47


<210> 212
<211> 47
<212> DNA
<213> Artificial Sequence

<220>
<223> Description of Artificial Sequence: DNA encoding
      an shRNA directed against the gene for the BRCA1
      associated protein-1

<400> 212
ctcttgtgca actcatgcct tcaagagagg catgagttgc acaagag                47

<210> 213
<211> 47
<212> DNA
<213> Artificial Sequence

<220>
<223> Description of Artificial Sequence: DNA encoding
      an shRNA directed against the gene for the BRCA1
      associated protein-1

<400> 213
acagggcccc tgcagcctct tcaagagaga ggctgcaggg gccctgt                47

<210> 214
<211> 47
<212> DNA
<213> Artificial Sequence

<220>
<223> Description of Artificial Sequence: DNA encoding
      an shRNA directed against the gene for the BRCA1
      associated protein-1

<400> 214
gaagacctgg cggcaggtgt tcaagagaca cctgccgcca ggtcttc                47

## Description

### Introduction

The invention provides a method for targeted transgenesis of short hairpin RNA expression cassettes using recombinase mediated cassette exchange. Suitable nucleotide acid sequences and vectors for the targeted transgenesis and recombinase mediated transgenesis are provided.

### Background of the Invention

The generation of transgenic mice by nuclear injection of purified DNA into fertilized eggs is a widely used approach for studying gene or promoter function *in vivo.* However, the level and pattern of expression often varies strongly depending on copy number, configuration, and integration site of the transgene. In addition, founder mice occasionally do not transmit the transgene. Thus, a number of different founders need to be generated and tested in order to identify a useful strain, which is a laborious and time-consuming undertaking (Bradley et. al., Nature Genet., 14:121-123 (1996); Jasin et al., Proc. Natl. Acad. Sci. USA, 93:8804-8808 (1996); Dobie et al., Trends Genet., 13:127-130 (1997); Garrick et al., Nature Genet., 18:56-59 (1998), Al-Shawi et al., Mol. Cell. Boil. 10:1192-1198 (1990)).

To overcome these limitations, homologous recombination in embryonic stem cells has been used to produce mice carrying a single copy of the transgene integrated into a predetermined site of the genome (Shaw-White et al., Transgenic Res.; (1):1-13 (1993); Bronson et al., Proc. Natl. Acad. Sci. USA, 93(17:9067-72 (1996); Hatada et al., J. Biol., Chem., 274(2):948-55 (1999); Vivian et al., Biotechniques, 27(1):154-62 (1999); Evans et al., Physiol. Genomics, Mar. 13, 2(2):67-75 (2000); Cvetkovic et al., J. boil. Chem., 275(2):1073-8 (2000); Guillot et al., Physiol. Genomics, Mar. 13, (2):77-83 (2000); Magness et al., Blood, 95(11):3568-77 (2000); Misra et al., BMC Biotechnol., 1(1):12 (2001); Minami et al., Blood, 100(12):4019-25 (2002); Tang et al., Genesis, 32(3):199-202 (2002)). In these studies, the ubiquitous Hprt locus was more or less successfully used for 'targeted transgenesis'. Insertion of a lacZ gene under the control of the polyoma enhancer/HSV thymidine kinase promoter into the third exon of Hprt resulted in variable β-galactosidase expression that was both orientation and cell-type dependent (Shaw-White et al., Transgenic Res.; (1):1-13 (1993)). Although transgenes under the control of the human and the chicken β-actin gene promoter resulted in widespread expression when inserted into the Hprt locus, the level of transcripts varied strongly in different tissues (Bronson et al., Proc. Natl. Acad. Sci. USA, 93(17:9067-72 (1996)). Unexpectedly, expression of these transgenes, but not of the endogenous Hprt gene appeared to be low or undetectable in kidney and liver (Bronson et al., Proc. Natl. Acad. Sci. USA, 93(17:9067-72 (1996)). Hatada et al. demonstrated that the HPRT locus suppresses the activity of both, the haptoglobin gene promoter as well as the herpes simplex thymidine kinase promoter in several tissues of mice (Hatada et al., J. Biol., Chem., 274(2):948-55 (1999)). Likewise, a human eNOS promoter-LacZ reporter gene placed in the Hprt locus was found to be inactive in hepatic vessels that otherwise express the endogenous eNOS gene (Guillot et al., Physiol. Genomics, Mar. 13, (2):77-83 (2000). Finally, since the HPRT gene is on the X chromosome, transgene expression at this locus is subjected to random X-inactivation. The expression of the transgene in all cells of the female, therefore, requires the generation of homozygotes.

Application PCT/EP04/00065 reports on a particular autosomal locus, namely Rosa 26 that allows strong and predictable expression of transgenes inserted through homologous recombination. This chromosomal locus was found useful in the context of the "targeted transgenesis" approach for the efficient generation of transgenic organisms (such as mice) with a predictable transgene expression pattern. The "targeted transgenesis" method provided in said application comprises consecutive experimental steps. A gene expression cassette comprising a suitable promoter (e.g. a ubiquitous or tissue specific promoter, either inducible or constitutive) functionally linked to a gene of interest is created; subsequently a vector for the targeted insertion of the above mentioned gene expression cassette into the Rosa26 locus is generated; the insertion of the above mentioned gene expression cassette into the Rosa26 locus through homologous recombination or site specific recombination in embryonic stem cells follows; finally transgenic mice are generated by the injection of such genetically modified ES cells into blastocysts.

Previously, he rosa26 locus had been identified by random insertion of retroviral sequences and a β-galactosidase-neomycin resistance fusion gene into the genome of mouse embryonic stem cells (Zambrowicz et al., Proc. Natl. Acad. Sci. USA, 94, 3789-94 (1997)). The rosa26 promoter appeared to mediate ubiquitous expression of promoter-less genes both in embryos and adult mice (Kisseberth et al., Dev. Biol., 214:128-138 (1999); Zambrowicz et al., Proc. Natl. Acad. Sci. USA, 94, 3789-94 (1997)), albeit at different levels in different organs (Vooijs et al., EMBO reports, 21:292-297 (2001)).

Moreover, WO 99/53017 describes a process for making transgenic animals which ubiquitously express a heterlogous gene, wherein the heterologous gene is under the control of a ubiquitously expressed endogenous promoter, e.g. that of the mouse Rosa26 locus. R. Dacquin et al., Dev. Dynamics 224 :245-251 (2002) and K. A. Moses et al., Genesis 31:176-180 (2001) utilize the transgenic mouse strain R26R obtained according to WO 99/53017 for the expression of heterlogous genes. WO 02/098217 describes a method of targeting promoter-less selection cassettes into transcriptionally active loci, such as the Rosa26 locus.

Finally, WO 03/020743 (published March 13, 2003) describes the expression of transgenes *in vivo* by targeting protected transgene cassettes into predetermined loci (e.g. the Rosa26 locus), such that the introduced tissue specific exogenous promoter has at least some tissue specific activity. The protected transgene cassette contains (from 5' to 3' direction) a transcriptional stop signal, the exogenous tissue specific promoter and the gene of interest. The presence of a transcriptional stop signal is vital for the method of WO 03/020743 as therewith the expression pattern is determined primarily by the nature of the tissue specific exogenous promoter.

RNA interference (RNAi) has been discovered some years ago as a tool for inhibition of gene expression (Fire, A. et al., Nature 391, 806-811 (1998)). It based on the introduction of double stranded RNA (dsRNA) molecules into cells, whereby one strand is complementary to the coding region of a target gene. Through pairing of the specific mRNA with the introduced RNA molecule, the mRNA is degraded by a cellular mechanism. Since long dsRNA provokes an interferon response in mammalian cells, the technology was initially restricted to organisms or cells showing no interferon response (Bass, B.L., Nature 411, 428-429 (2001)). The finding that *short* (<30 bp) *interfering RNAs* (siRNA) circumvent the interferon response extended the application to mammalian cells (Elbashir, S.M. et al., Nature 411, 494-498 (2001)).

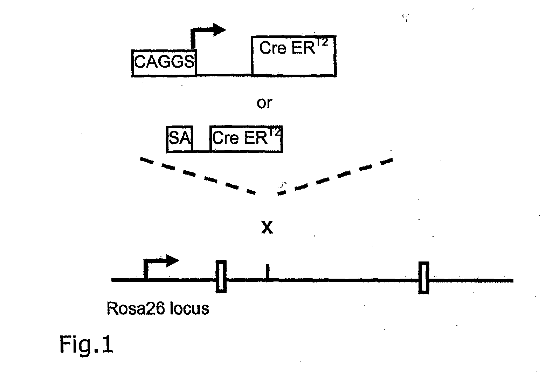

Although RNAi in mice has been in principle demonstrated, the current technology does not allow performing systematic gene function analysis *in vivo*. So far the inhibition of gene expression has been achieved by injection of *purified* siRNA into the tail vain of mice (McCaffrey, A.P. et al., Nature 418, 38-39 (2002); Lewis, D.H. et al., Nature Genet. 32, 107-108 (2002)). Using this approach, gene inhibition is restricted to specific organs and persists only a few days. A further improvement of the siRNA technology is based on the intracellular transcription of short hairpin RNA (shRNA) molecules using gene expression vectors (see Fig. 1; Brummelkamp, T.R. et al., Science 296, 550-553 (2002); Paddison, P.J. et al, Genes Dev. 16, 948-958 (2002); Yu, J.Y. et al., Proc. Natl. Acad. Sci. USA 99, 6047-6052 (2002); Sui, G. et al., Proc. Natl. Acad. Sci. USA 99, 5515-5520 (2002); Paul, C.P. et al., Nature Biotechnol. 20, 505-508 (2002); Xia, H. et al., Nat. Biotechnol. 10, 1006-10 (2002); Jacque, J.M. et al., Nature 418(6896):435-8 (2002)). The activity of shRNA in mice has been demonstrated by McCaffrey et al., 2002 through injection of shRNA expression vectors into the tail vain. Again, gene inhibition was temporally and spatially restricted. Although these results demonstrate that the mechanism of shRNA mediated gene silencing is functional in mice, they do not clarify whether constitutive RNAi can be achieved in transgenic animals. Brummelkamp, T.R. et al., Science 296, 550-553 (2002), Paddison, P.J. et al., Genes Dev. 16, 948-958 (2002), Hemann, M.T. et al., Nat. Genet. 33(3):396-400 (2003); and Devroe, E. et al., BMC Biotechnol. 2(1):15 (2002) have shown the long-term inhibition of gene expression through stable integration of shRNA vectors in cultivated cell lines. These experiments included random integration of shRNA transgenes and screening for clones with appropriate siRNA expression, which is not applicable for testing of a large number of different shRNA transgenes in mice. Finally, several reports have demonstrated shRNA-mediated gene silencing in transgenic mice and rats (Hasuwa, H. et al., FEBS Lett. 532(1-2):227-30 (2002); Carmell, M.A. et al., Nat. Struct. Biol. 10(2):91-2 (2003); Rubinson, D.A. et al., Nat. Genet. 33(3):401-6 (2003); Kunath, T. et al., Nat. Biotechnol. (Apr. 7 2003)). However, these experiments again included random integration of shRNA transgenes resulting in variable levels and patterns of shRNA expression. Thus, testing of ES cell clones or mouse lines with appropriate shRNA expression had been required, which is a laborious and time-consuming undertaking.

The *in vivo* validation of genes by RNAi mediated gene repression in a large scale setting requires the expression of siRNA at sufficiently high levels and with a predictable pattern in multiple organs. Targeted transgenesis provides the only approach to achieve reproducible expression of transgenes in the living organism (e.g. mammalians such as mice). It has been, however, questionable whether a single copy of a siRNA expression vector integrated into the genome would result in sufficiently high levels of siRNA required for RNAi-mediated gene inhibition in multiple organs of the living organism.

Most siRNA expression vectors are based on polymerase III dependent (Pol III) promoters (U6 or H1) that allow the production of transcripts carrying only a few non-homologous bases at their 3' ends. It has been shown that the presence of non-homologous RNA at the ends of the shRNA stretches lower the efficiency of RNAi mediated gene silencing (Xia et al., Nat. Biotechnol. 10, 1006-10 (2002)). However, it has been unpredictable for a person skilled in the art which genomic region within the living organism promotes an appropriate expression pattern of the Pol III promoter driven shRNA constructs required for RNAi-mediated gene inhibition in multiple organs of the living organism.

For the temporal control of RNAi mediated gene silencing in transgenic cells lines and living organism, a tight system for inducible siRNA expression is needed. Inducible gene expression systems based on the tetracycline dependent repressor are known. It is, however, not obvious whether a stably integrated, single copy configuration of these systems can be created that allows inducible RNAi in multiple organs without background activity.

Two types of procedures have been described for targeted integration of transgenes into defined loci of the embryonic stem (ES) cell genome. One is based on homologous recombination (HR) in embryonic stem cells, and the other on site-specific recombination. In the first case, the efficiency is limited by the low frequency of HR. In contrast, site-specific recombination has emerged as a powerful tool for the targeted insertion of transgenes into the eukaryotic genome.
Site-specific recombinases such as Flp and Cre mediate recombination between two copies of their target sequence termed FRT and loxP, respectively. The use of two incompatible target sequences, for example FRT in combination with F3 (Schlake & Bode, Biochemistry, 1994 Nov. 1, 33(43):12746-51) as well as inverted recognition target sites (Feng et al., J. Mol. Biol. 292(4):779-85 (1999)) allows the insertion of DNA segments into a predefined chromosomal locus carrying target sequences in a similar configuration. This exchange system is called recombinase mediated cassette exchange (RMCE; Bode & Baer, Curr Opin Biotechnol. 2001 Oct;12(5):473-80). In contrast to approaches using a single recombination site the targeting product is stable even under the permanent influence of the recombinase unless it is exposed to an exchange plasmid (Seibler & Bode (1997) Biochemistry 36, 1740- 1747.).
So far, only few examples of successful RMCE in ES have been described (Feng et al., J Mol Biol. 1999 Oct 1;292(4):779-85; Seibler et al., Biochemistry. 1998 May 5;37(18):6229-34; Kolb, Anal Biochem. 2001 Mar15;290(2):260-71; Belteki et al., Nat Biotechnol. 2003 Mar;21(3):321-4.; Cesari et al., 2004, Genesis, 38:87-92.). In these experiments, random integration of the exchange vector as well as incomplete recombination frequently produced unwanted transgene configurations. The efficiency of RMCE appeared to vary strongly depending on the choice of recombination sites, the selection strategy, and the chromosomal target. The criteria for efficient RMCE at a given locus are therefore not defined and unpredictable for a person skilled in the art.

The only example of efficient (>90%) RMCE at a defined locus used the tissue-specific β-casein gene as chromosomal target (Kolb, Anal Biochem. 2001 Mar15;290(2):260-71). However, a HPRT gene was required to exclude random integration or incomplete recombination of the exchange vector. The application of this strategy is therefore limited to HPRT-negative ES cells. In addition, the cell type specific activity of the β-Casein locus may not be suitable for the expression of transgenes in multiple tissues. Taken together, a general strategy for efficient RMCE at a ubiquitously active locus has never been achieved.

### Summary of the Invention

It was surprisingly found that RMCE can be effectively be performed at ubiquitously active loci with high efficiency. The invention provides:
(1) a method for generating transgenic eukaryotic cells having an ubiquitous locus modified by an expression cassette comprising a short hairpin RNA construct operatively linked to a promoter or an inactive precursor thereof, which method comprises introducing the expression cassette into the ubiquitous locus of eukaryotic cells by recombinase mediated cassette exchange;
(2) the method of (1) above, which comprises
   (a) introducing a functional DNA sequence into the Rosa26 locus of starting eukaryotic cells by homologous recombination with a targeting vector comprising flanking DNA sequences homologous to the ubiquitous locus and an acceptor DNA, which integrates into the genome of the starting cell, the acceptor DNA comprising two mutally incompatible first recombinase recognition sites (RRSs), and
   (b) effecting recombinase mediated cassette exchange of the recombination product of step (a) having an RMCE target site with an exchange vector comprising a donor DNA, which comprises the expression cassette flanked by the same two mutually incompatible first RRSs contained in the acceptor DNA, by utilizing a recombinase which catalyzes recombination between the RRSs of the acceptor and donor DNA;
(3) the method of (1) or (2) above, wherein the transgenic eukaryotic cells are derived from mouse and the ubiquitous locus is a Rosa26 locus, and
   (i) the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and/or
   (ii) the RRSs of the targeting and exchange vectors are F3/Frt and the targeting vectors encodes the recombinase Flp or a muötant thereof, preferably Flp^{e}; and/or
   (iii) the targeting vector comprises a negative selection marker; and/or
   (iv) the exchange vector comprises a promoter-less positive selection marker; and/or
   (v) the promoter of the expression cassette is a H1 or H6;

   most preferably the targeting vector has the sequence shown in SEQ ID NO:11 and the exchange vector has the sequence shown in SEQ ID NO:12 or a variant thereof with modification in the short hairpin RNA construct;
(4) an exchange vector as defined in (1) to (3) above;
(5) a eukaryotic cells having a modified ubiquitous locus obtainable by the method of (1), (2) and (3) above;
(6) a method for preparing a transgenenic multi-cell organism having a modified ubiquitous locus which comprises utilizing the method as defined in (1) and (3) above;
(7) the method of (6) above, wherein the transgenenic multi-cell organism is a non-human mammal and said method comprises modifying an ES cell as defined in (3) above;
(8) a transgenic multi-cell organism and non-human mammal obtainable by the above defined methods (6) and (7), respectively; and
(9) the use of the eukaryotic cell of (5) above, the transgenic multi-cell organism of
(8) above, or the transgenic non-human mammal of (8) above for gene function studies, drug development, as disease model, etc.

The method of the invention offers several advantages over the current technology of pronuclear injection. In particular, the targeting vector allows insertion of a single copy of a gene expression cassette, thus avoiding modulation of transgene expression by the arrangement of multiple copies. By choosing the autosomal Rosa26 locus as insertion site, the expression pattern of the inserted transgene in the non-human animal is predictable; random X-inactivation and/or modulation by chromosomal position effects are avoided. This also eliminates the need to generate and analyse multiple transgenic strains for any given transgene. Finally, the Rosa26 targeting vector for the site-specific integration can be used for multiple gene expression cassettes. Moreover, the RMCE strategy provides for more flexibility for consitutive and inducible gene knock-down, RNA mediated gene silencing in transgene animals and living organs.

### Description of the Figures

Figure 1: Targeted insertion of CreER and CAGGS-Cre-ER into the Rosa26 locus. A cassette comprising a Cre-ER operationally linked to a CAGGS promoter or a cassette comprising a splice acceptor site (SA) linked to a Cre-ER are inserted into the Rosa26 locus via homologous recombination. A perpendicular dash marks the insertion point within the Rosa26 locus and the rectangular boxes delinate the starting and end points of the Rosa26 transcript.

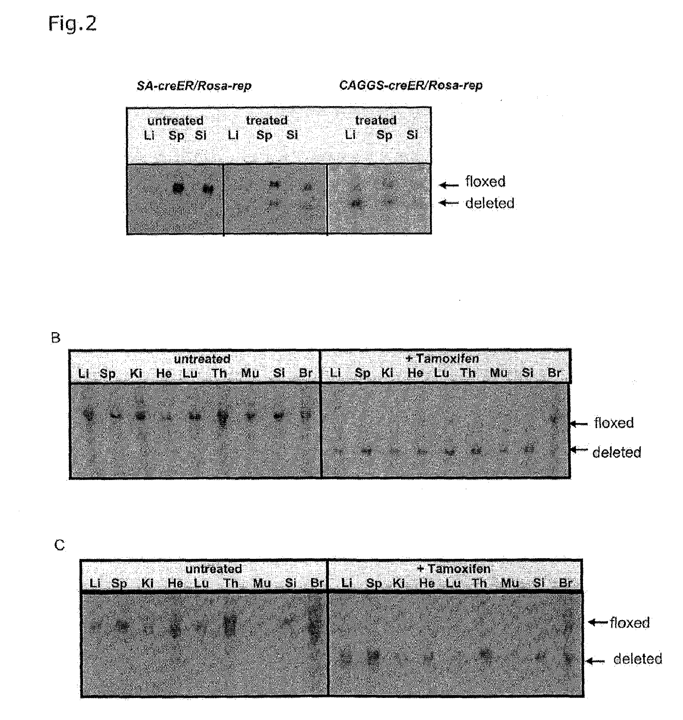

Figure 2: Southern Blot analysis of the inducible recombination of the Rosa (reporter). (A) Genomic DNA was isolated from liver (Li) spleen (Sp) and small intestine (Si) of transgenic mice carrying the SA-creER/Rosa-rep insert or the CAGGS-creER/Rosa-rep insert. To induce the Cre-ER recombinase the mice were treated with Tamoxifen (treated). As a control, a group of mice with the SA-creER/Rosa-rep insert was left untreated (untreated). Presence of the reporter band (floxed) and deletion (deleted) of it upon an induced recombination event are indicated. (B) Transgenic mice carrying at one Rosa26 locus a loxP flanked DNA polymerase β gene segment (pol^{*βflox*}) and at the other a SA-creER/Rosa-rep were treated with Tamoxifen (treated). A control group of mice was left untreated (untreated). Genomic DNA from liver (Li), spleen (Sp), kidney (Ki). heart (He), lung (Lu), thymus (Th), muscle (Mu), small intestine (Si) and brain (Br) was analysed for presence of pol^{*βflox*}. In a non-recombination event the pol^{*βflox*} band remained (floxed), in a recombination event deletion occurred (deleted). (C) As (B), but mice carried instead of the SA-creER/Rosa-rep the CAGGS-creER/Rosa-rep insert.

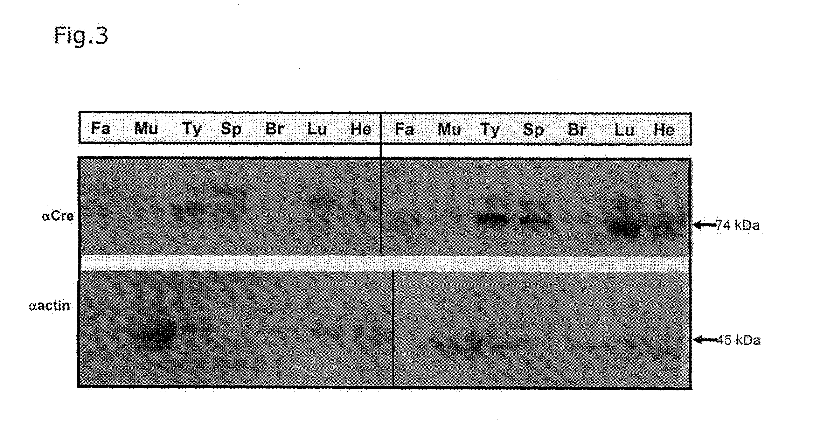

Figure 3: Western Blot analysis of recombinase and α-actin expression. Proteins were extracted from rosa(SA-CreER^{T2}) and rosa (CAGGS-CreER^{T2}) mice and analyzed as described in the "Materials and Method" section. The positions of bands representing CreER^{T2} and actin are indicated. FA: fat tissue, Ty: Thymus; Sp: spleen, Br: Brain, Lu: lung, He: heart.

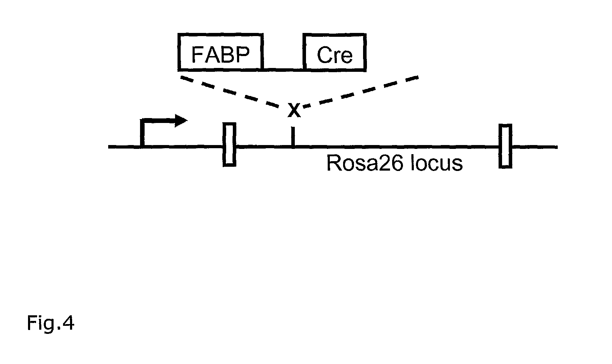

Figure 4: Fabp-Cre targeting vector. An expression cassette, in which the Cre recombinase is expressed under the control of the Fabpl^{4x at -132} promoter is inserted into the Rosa26 targeting vector. This vector was used to insert the Fabp-Cre cassette into the Rosa26 locus by homologous recombination in ES cells.

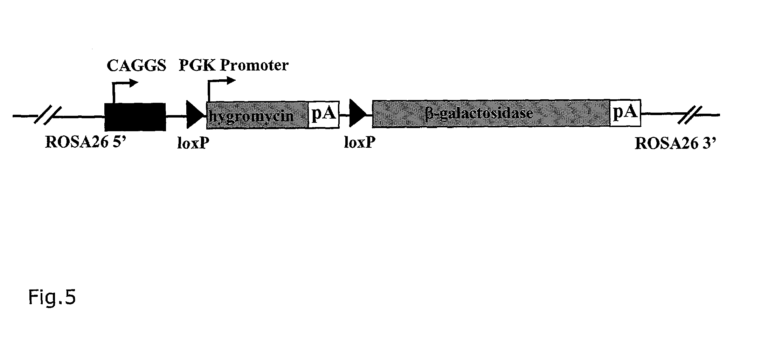

Figure 5: ROSA26 locus of the Cre reporter mice carrying a Cre substrate reporter construct. A recombination substrate (Seq ID NO:9) has been inserted in the ROSA26 locus. The substrate consists of a CAGGS promoter followed by a cassette consisting of the hygromycin resistance gene driven by a PGK promoter and flanked by loxP recombination sites. This cassette is followed by the coding region for beta-galactosidase, which is only expressed when the hygromycin resistance gene has been deleted by recombination.

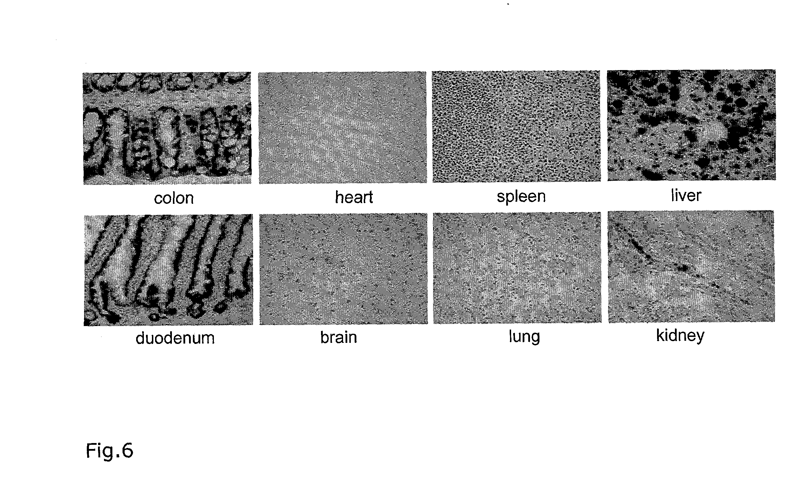

Figure 6: *In situ* detection of beta-galactosidase in cryosections of different tissues of Fabp-Cre/reporter substrate double transgenic mice. Mouse tissues were embedded in OCT, frozen and cut into microsections. The sections were stained for beta-galactosidase activity (indicated by the blue color) by X-gal staining, counterstained with Nuclear Fast Red Solution, dehydrated, mounted and photographed.

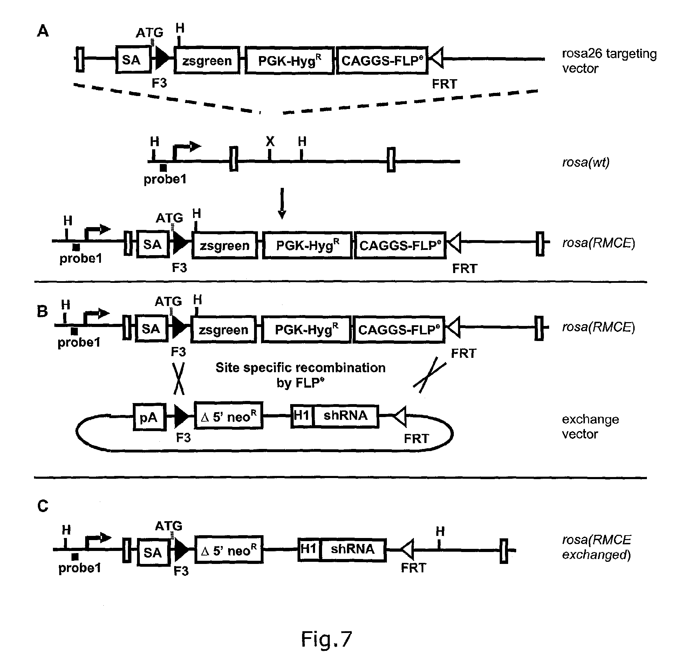

Figure 7: RMCE targeting system for rosa26. A) Insertion of the RMCE target into the rosa26 locus. A cassette comprising zsgreen, PGK-Hyg, and CAGGS-FLP is inserted into the Rosa26 locus via homologous recombination in ES cells. The FRT and F3 sites are oriented in opposite direction to each other. A perpendicular dash with 'X' marks the insertion point within the rosa26 locus. B) Exchange vector carrying FRT and F3 sites together with a truncated neo^{R} gene for positive selection of RMCE and a shRNA expression cassette under the control of the U6 promoter for targeted integration into the Rosa26 locus. The polyA signal is included to prevent expression of the truncated neo^{R} gene at sites of random integration. C) Configuration of the targeted Rosa26 locus following. X: XbaI, H: HindIII.

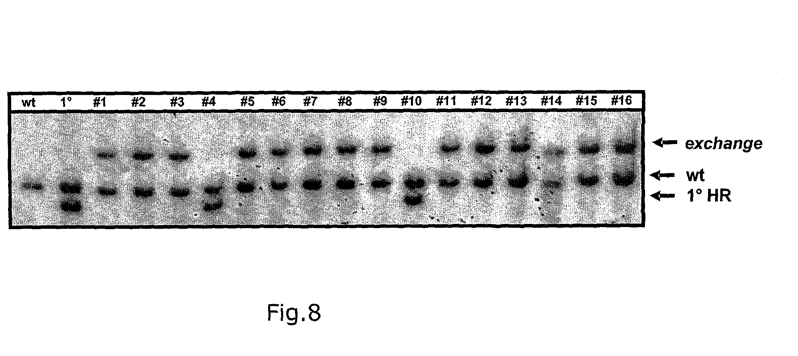

Figure 8: Southern blot analysis of genomic DNA from rosa(RMCE) targeted ES cells transfected with the exchange vector. *rosa(RMCE exchanged)* alleles. The sizes of the wt, Rosa26 targeted (1° HR) and RMCE alleles (exchange) are 4.4 kb, 3.9 kb and 5.8 kb, respectively. In clones #1 - 3, 5 - 9, and 11 - 16 successful RMCE had occurred. Genomic DNA was digested with HindIII and analyzed using probe 1.

### Detailed Descripion of the Invention

The term "living organisms" according to the present invention relates to multi-cell organisms which can be vertebrates such as mammals (e.g. non-human animals such as rodents including mice and rats; and humans) or non-mammals (e.g. fish) or can be invertebrates such as insects or worms, or can be plants (higher plants, algi or fungi). Most preferred living organisms are mice and fish.

"Eukaryotic cells" and "starting eukaryotic cells" according to the present invention include cells isolated (derived) from the above defined living organisms and cultured in *vitro*. These cells can be transformed (immortalized) or untransformed (directly derived from living organisms; primary cell culture). The term "eukaryotic cells" also includes mono-cellular eukaryotic cells such as yeasts, etc.

It is preferred in the method (1) of the present invention that the eukaryotic cells are derived from a multi-cell organism including vertebrates, invertebrates and plants, preferably is a vertebrate cell, more preferably is derived from a mammal, including rodents such as mouse, rat, etc., or a fish such as zebrafish.

In the method (1) of the invention it is preferred that the functional DNA sequence comprises a gene encoding a protein/peptide of interest (i.e. is a expressible and translatable DNA sequence), more preferably said functional DNA sequence is a gene expression cassette (a) comprising a gene of interest operatively linked to a promoter, or (b) is a DNA sequence which can be converted into such gene expression cassette (i.e. into an operatively linked "promoter-gene of interest" construct, e.g. by subsequent modification reactions after its integration). The gene of interest within the gene expression cassette can be any gene coding for a certain protein/peptide of interest, including, but not limited to, recombinases, reporter genes, receptors, signaling molecules, transcription factors, pharmaceutically active proteins and peptides, drug target candidates, disease causing gene products, toxins, etc.

The promoter of the gene expression cassette (which is a heterologous promoter relative to the Rosa26 locus) preferably is a ubiquitous or tissue specific promoter, either constitutive or inducible. The ubiquitous promoter in the vector according to the invention is preferably selected from polymerases I, II and III dependent promoters, preferably is a polymerase II or III dependent promoter including, but not limited to, a CMV promoter, a CAGGS promoter, a snRNA promoter such as U6, a RNAse P RNA promoter such as H1, a tRNA promoter, a 7SL RNA promoter, a 5 S rRNA promoter, etc. Particularly preferred ubiquitous promoters are CAGGS, hCMV, PGK. Preferred tissue specific promoters are FABP (Saam & Gordon, J. Biol. Chem., 274:38071-38082 (1999)), Lck (Orban et al., Proc. Natl. Acad. Sci. USA, 89:6861-5 (1992)), CamKII (Tsien et al., Cell 87: 1317-1326 (1996)), CD19 (Rickert et al., Nucleic Acids Res. 25:1317-1318 (1997)), Keratin (Li et al., Development, 128:675-88 (201)), Albumin (Postic & Magnuson, Genesis, 26:149-150 (2000)), aP2 (Barlow et al., Nucleic Acids Res., 25 (1997)), Insulin (Ray et al., Int. J. Pancreatol. 25:157-63 (1999)), MCK (Brüning et al., Molecular Cell 2:559-569 (1998)), MyHC (Agak et al., J. Clin. Invest., 100:169-179 (1997), WAP (Utomo et al., Nat. Biotechnol. 17:1091-1096 (1999)), Col2A (Ovchinnikov et al., Genesis, 26:145-146 (2000)); preferred inducible promoter systes are Mx (Kühn et al. Scinence, 269:1427-1429 (1995)), tet (Urlinger et al., Proc. Natl. Acad. Sci. USA, 97:7963-8 (2000)), Trex (Feng and Erikson, Human Gene Therapy, 10:419-27). Suitable inducible promoters are the above-mentioned promoters containing an operator sequence including, but not limited to, tet, Gal4, lac, etc.

The targeting vector, recombination vector, functional DNA sequence or gene expression cassette may further comprises one ore more additional functional sequences including but not limited to (selectable) marker genes (such as the neomycin phosphotransferase gene of *E. coli* transposon, etc.), recombinase recognition sites (which in case of the recombination vector differ from the first recombinase recognition sites and which include IoxP, FRT, variants thereof, etc.), poly A signals (such as synthetic polyadenylation sites, or the polyadenylation site of human growth hormones, etc.), splice acceptor sequences (such as a splice acceptor of adenovirus, etc.), introns, tags for protein detection, enhancers, selection markers, etc.

In a preferred embodiment methods (1) to (3) of the invention comprise homologous recombination. It is then preferred that the DNA sequences homologous to the Rosa26 locus are 0.2 to 20 kB, preferably 1 to 10 kB long. In a particularly preferred embodiment of the method (2) the eukaryotic cells are derived from mouse, the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and the promoter is a CAGGS-promoter, most preferably the targeting vector has the sequence shown in SEQ ID NO:7.

As set forth above, methods (1) to (3) of the invention comprise recombinase mediated cassette exchange (RMCE). The insertion of transgenes or DNA segments into the genome can be mediated by site specific recombination (Fukushige & Sauer, Proc. Natl. Acad. Sci. USA 89(17):7905-9 (1992)). A site specific recombinase like cre or FLP recombines two recognition target sites like IoxP or FRT, respectively. The use of two incompatible recognition target sites (F3 or F5, Schlake & Bode, Biochemistry, 1994 Nov. 1, 33(43): 12746-51) or inverted recognition target sites (Feng et al., J. Mol. Biol. 292(4):779-85 (1999)) allows the insertion of DNA segments flanked by two incompatible or inverted target sites. This exchange system has been called recombinase mediated cassette exchange (RMCE). In a preferred embodiment a FLP based RMCE system is inserted into the Rosa26 locus. Said recombinase mediated recombination preferably comprises the steps:
(a1) introducing into the starting cells an acceptor DNA which integrates into the genome of the starting cell, the acceptor DNA comprising two mutally incompatible first RRSs, and introducing into the therewith obtained cell
(a2) a donor DNA comprising the same two mutually incompatible first RRSs contained in the acceptor DNA by utilizing a recombination vector as defined above; and
(a3) the recombinase which catalyzes recombination between the RRSs of the acceptor and donor

In said recombinase mediated recombination method it is preferred that
(i) the RRS are loxP or FRT sites or variants thereof (such as single mutant recognition sited lox66 and lox71 (Albert et al., The Plant J. 7:649-659 (1995)); and/or
(ii) the acceptor DNA comprises a negatively selectable marker (e.g. herpes simplex virus thymidin kinase gene, etc.) and or
(iii) the donor DNA comprises an inactive positive selection marker (e.g. neomycin phosphotransferase, etc.).

For further selectable markers it is referred to U.S. Patents Nos. 5,487,932 and 5,464,763 which are hereby incorporated in their entirety.

The ubiquitous promoter in the vector according to the invention is preferably selected from polymerase I, II and III dependent promoters, preferably is a polymerase II or III dependent promoter including, but not limited to, a CMV promoter, a CAGGS promoter, a snRNA promoter such as U6, a RNAse P RNA promoter such as H1, a tRNA promoter, a 7SL RNA promoter, a 5 S rRNA promoter, etc.
The ubiquitous promoter can be a constitutive promoter, or can be an inducible promoter. Suitable inducible promoters are the above-mentioned polymerase I, II and III dependent promoters containing an operator sequence including, but not limited to, tet, Gal4, lac, etc.

The expression vector of the invention is suitable for the following particularly preferred approaches (for constitutive and inducible expression):
A. a Pol III dependent promoter (constitutive U6, H1 or the like) driven shRNA construct (to be integrated into a ubiquitously active Pol II dependent locus (see Fig. 2);
B. a Pol III dependent promoter (inducible U6, H1 or the like) driven shRNA construct (to be integrated into a ubiquitously active Pol II dependent locus (Fig 3 and 4)); or
C. a polymerase II (Pol II) dependent promoter (inducible CMV or the like) driven shRNA construct (to be integrated into a ubiquitously active Pol II dependent locus (Fig. 5 and 6)).

The ShRNA within the vector of the invention preferably comprises
(I) at least one DNA segment A-B-C wherein
   A is a 15 to 35, preferably 19 to 29 bp DNA sequence being at least 95%, preferably 100% complementary to the gene to be knocked down (e.g. firefly luciferase, p53, etc.);
   B is a spacer DNA sequence having 5 to 9 bp forming the loop of the expressed RNA hair pin molecule, and
   C is a 15 to 35, preferably 19 to 29 bp DNA sequence being at least 85% complementary to the sequence A; and
(II) a stop and/or polyadenylation sequence.

Suitable shRNA sequences for the knock down of a given target gene are well known in the art (see e.g. the particular shRNA sequences mentioned in Tables 1 and 2 below) or can readily be determined by the skilled artesian.

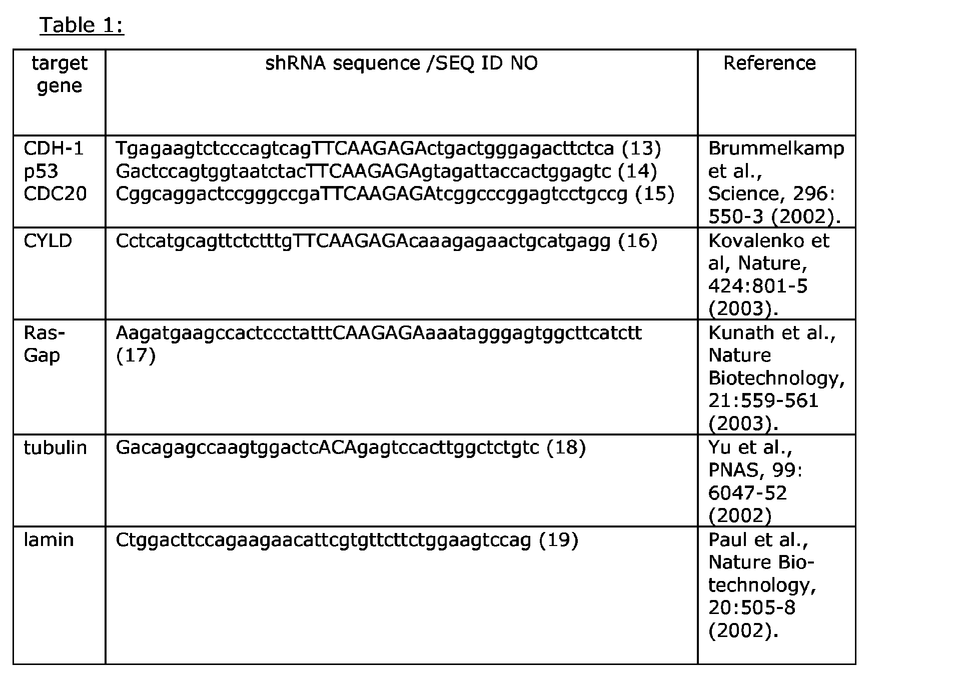

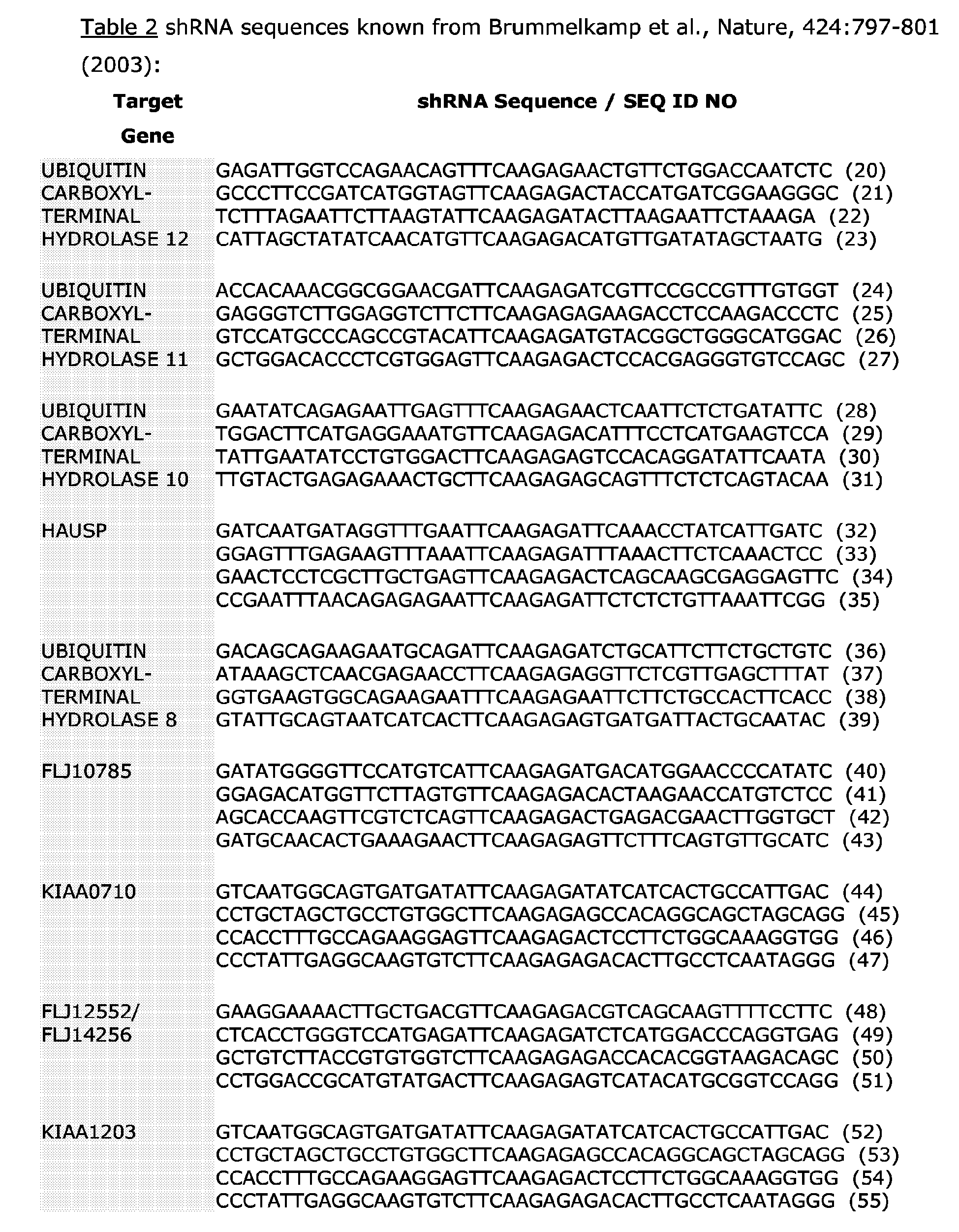

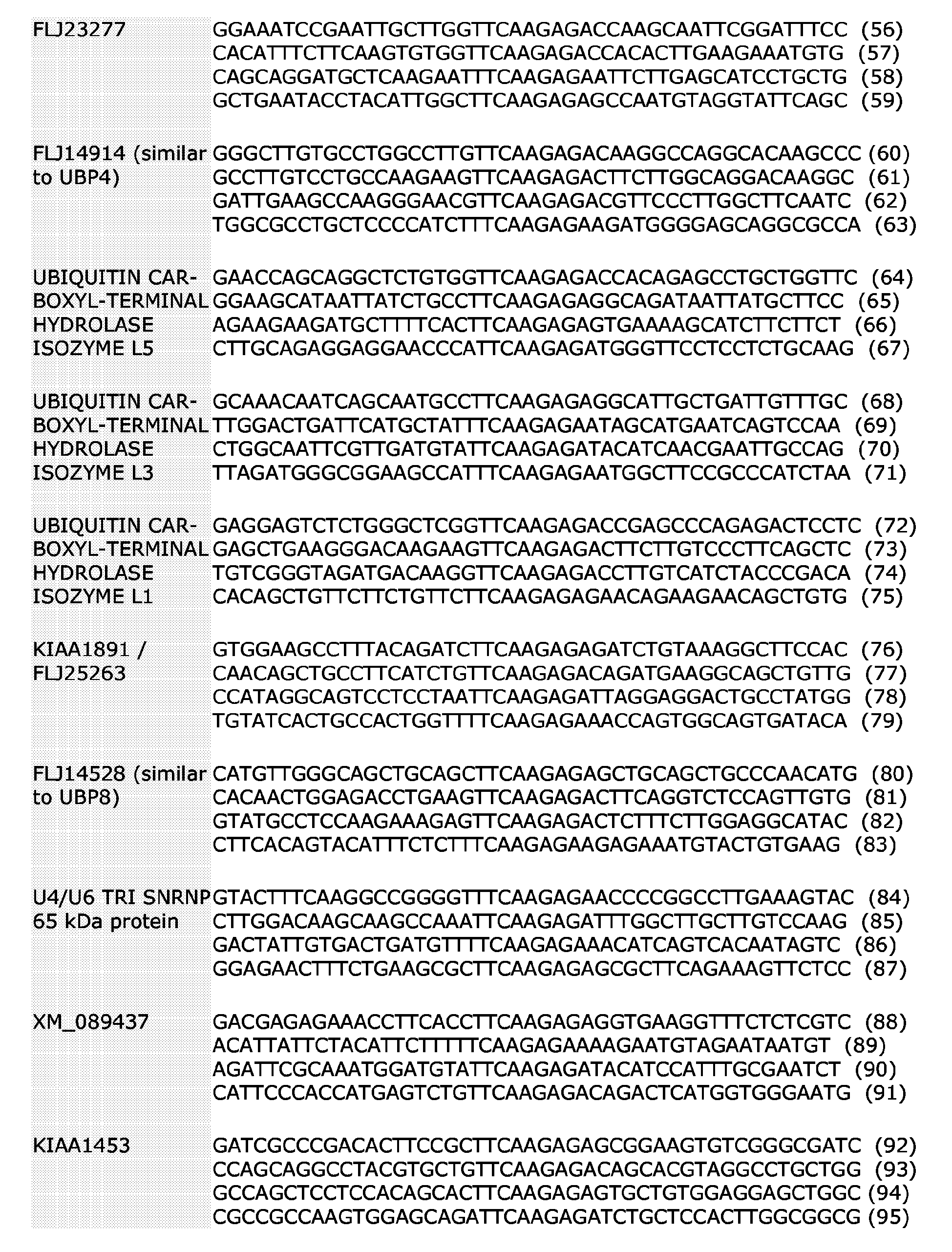

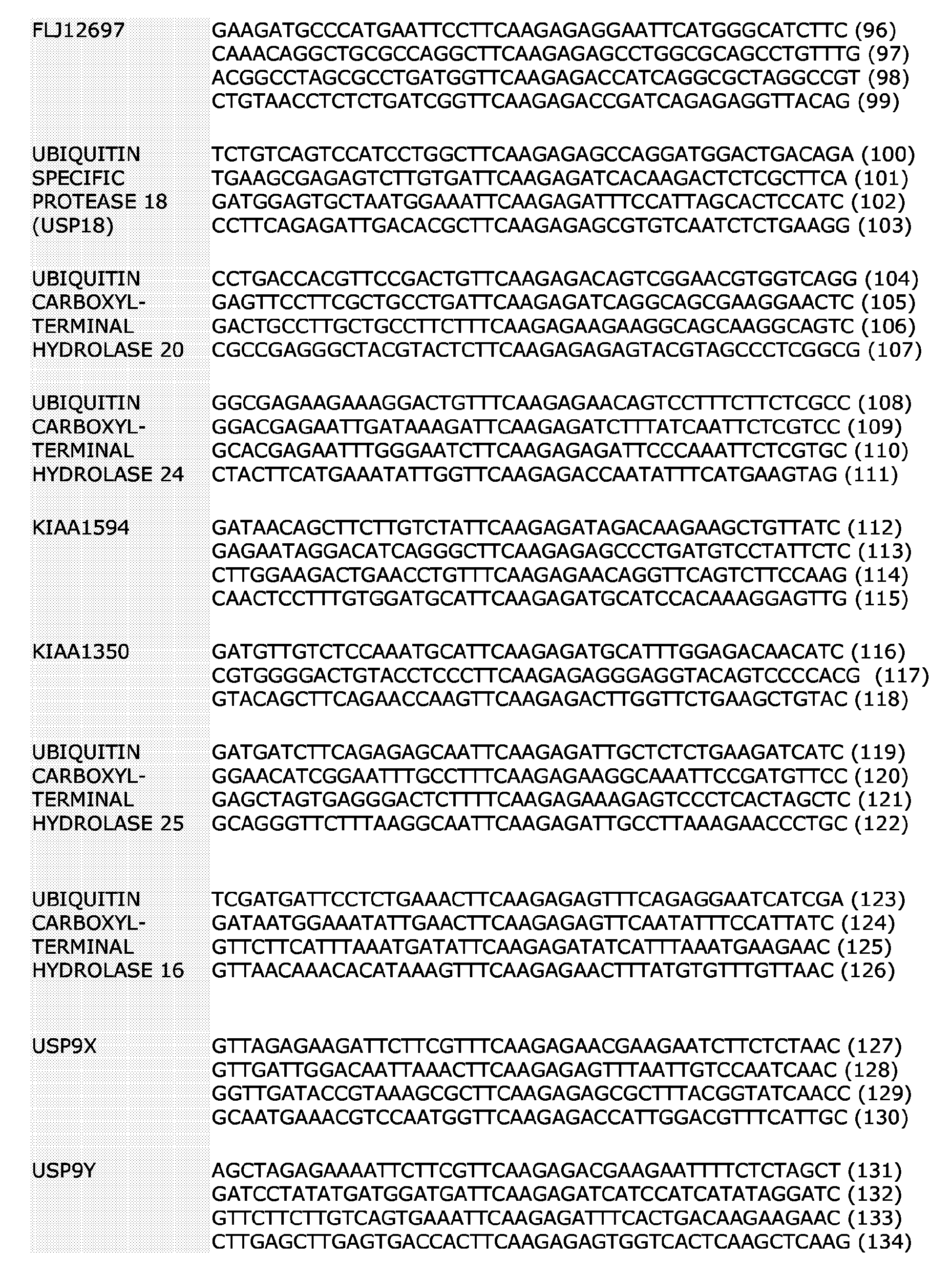

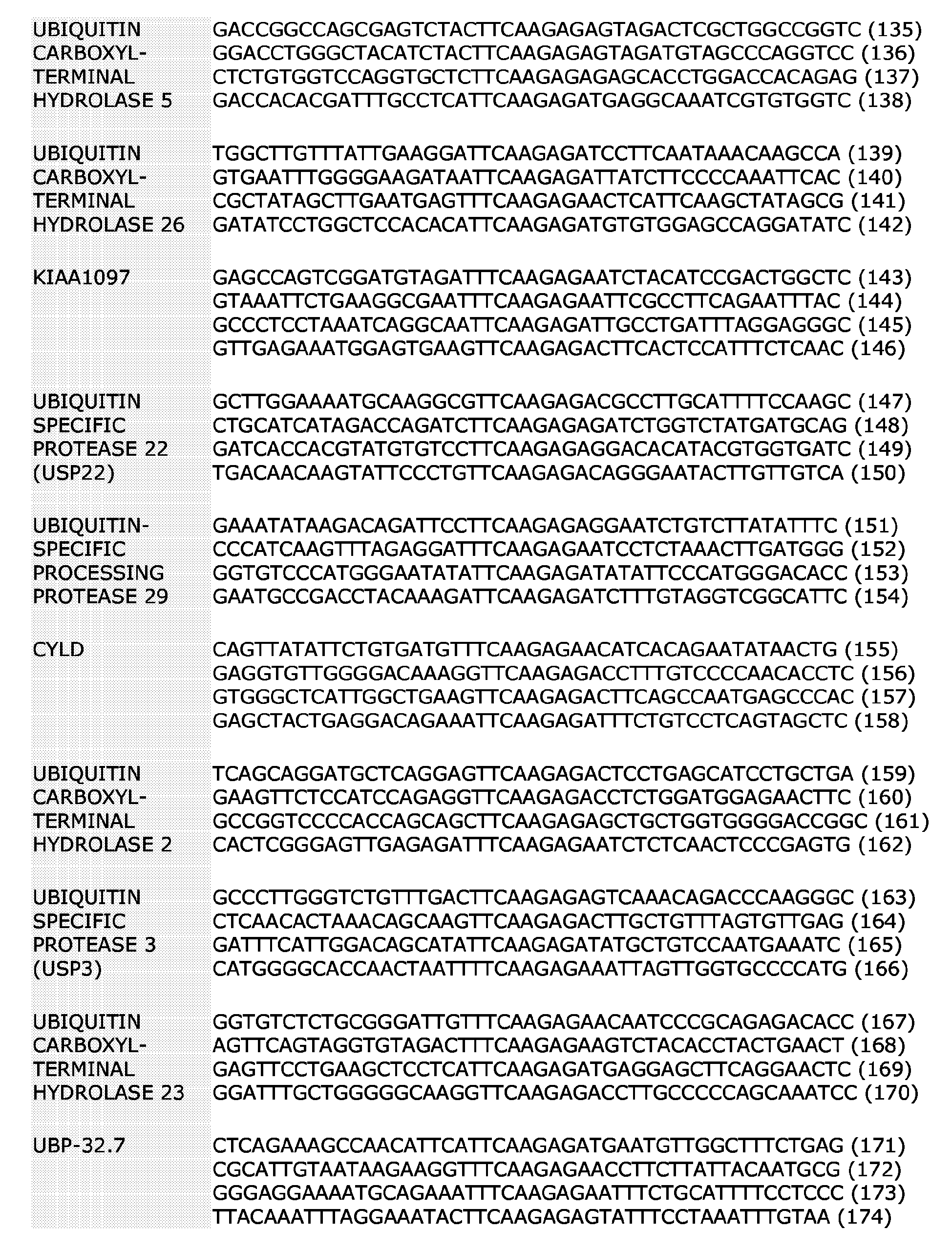

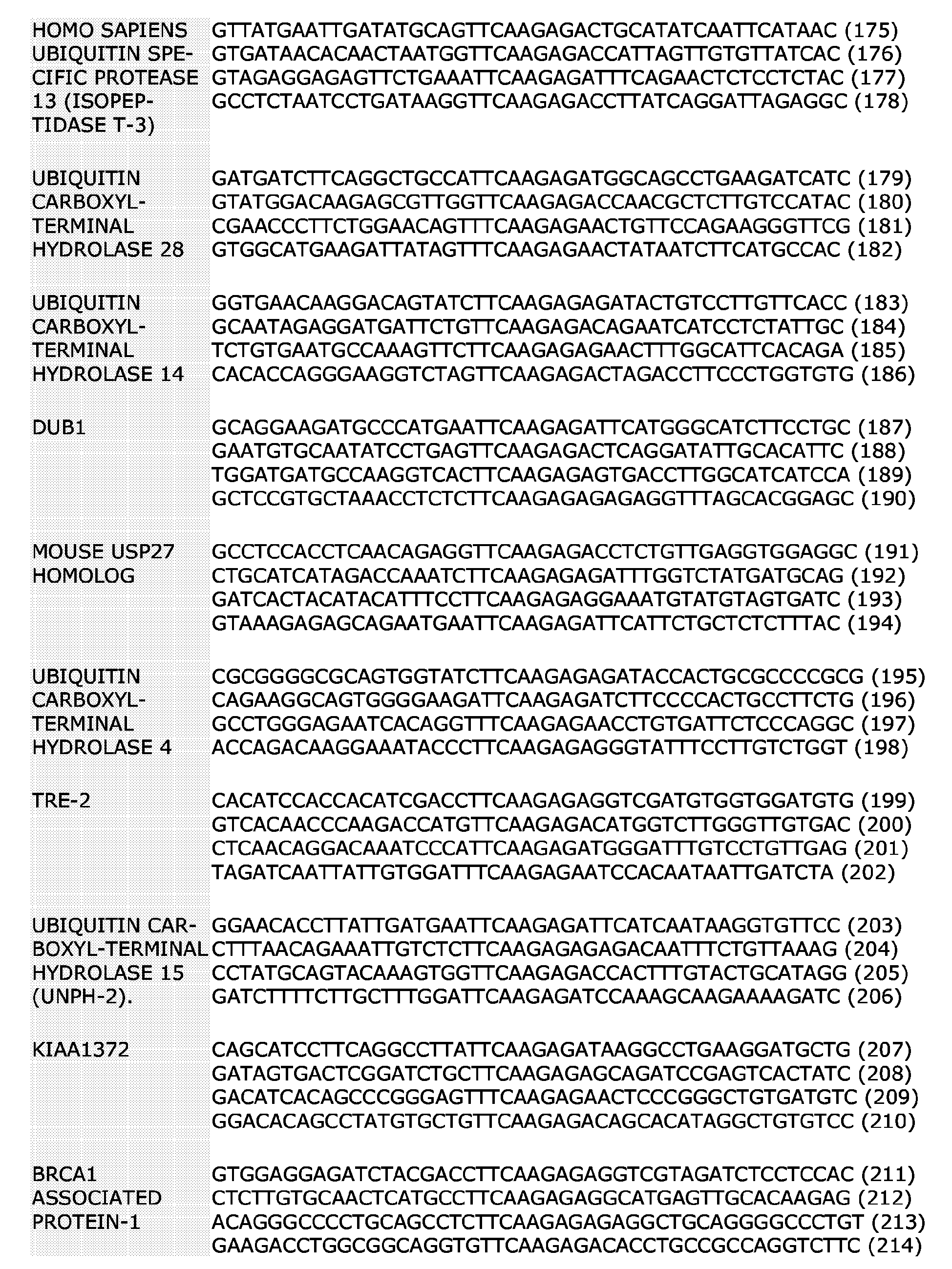

A preferred embodiment of the method (1) or (2) of the invention concerns the following steps:
1. Generation of the short hairpin DNA containing the antisense- and sense-strand of the coding region of a gene (e.g. firefly luciferase; p53). Antisense and sense-strand are separated by a spacer of 5 to 9 bp.
2. Generation of constructs for the expression of the above mentioned shRNA under the control of a constitutive or inducible promoter (Pol II or Pol III dependent).
3. Insertion of the mentioned expression constructs into an exchange vector and subsequent insertion of the exchange vector into a ubiquitously expressed locus in ES cells by RMCE.
4. Analysis of the constitutive and inducible inhibition of gene expression (e.g. firefly luciferase; p53) in ES cells (e.g. through Western blot analysis).
5. Generation of mice using the mentioned ES cells and analysis of the inhibition of gene expression in several tissues (e.g. firefly luciferase; p53; e.g. through Western blot analysis).

The vector according to embodiment (4) of the invention is suitable for stable or transient integration. Said vector is suitable for gene transfer.

The technology of the present application provides for the following advantages:
(i) A stable and body wide inhibition of gene expression by generating transgenic animals (such as mice).
(ii) A reversible inhibition of gene expression using the inducible constructs.

We showed that high efficient (>90%) RMCE at the ubiquitously expressed Rosa26 locus. The following features where combined in the RMCE strategy of the invention:
1. We utilized Flp mediated RMCE using a wild type Flp target site (FRT) in combination with an inverted F3 site. The F3 sequence was generated by systematic mutagenesis of the 8 bp spacer localized between the Flp binding elements (Schlake & Bode (1994) Biochemistry 33, 12746- 12751.). The F3/F3 couple is recombined by FLP with the same efficiency as two wild type recombinase recognition sites (RRS) whereas recombination of a FRT/F3 pair is not catalyzed (Seibler & Bode (1997) Biochemistry 36, 1740- 1747.). This characteristic contrasts other pairs of wild type and mutant RRS such as loxp/lox511 that exhibit a residual recombination capacity (Lauth et al., 2002, Nucleic Acids Res. 30:e115).
2. We included a constitutive FLP^{e} expression cassette on the targeting vector to provide sufficient recombinase activity until successful RMCE of the exchange vector. Thus, incomplete recombination intermediates should be avoided.
3. The positive selection marker along with a splice acceptor site on the exchange vector lacks a functional promoter. Thus, expression of the selection marker should only be mediated by the endogenous Rosa26 promoter following successful RMCE, but not through random integration of the exchange vector.
4. The fluorescent protein expression cassette on the targeting vector should allow for the detection of RMCE in early embryos, avoiding long term culture in medium containing antibiotics.

The methods (1) to (3) may further (besides step (a) and (b) defined above) comprise one or more of the steps (c) isolating the eukaryotic cells, preferably the ES cells having the desired fuctional exchange cassette integrated into the Rosa26 locus; and/or (d) modifying the integrated precursor of the exchange cassette and isolating (ES) cells having the desired modified functional exchange cassette.

The steps (a) and (b) of the methods (1) to (3) are preferably performed *in vitro.* The step (c) may be performed *in vitro* and *in vivo.*

The invention also provides a method for preparing a transgenenic multi-cell organism having a modified Rosa26 locus which comprises utilizing the method as defined in (1) to (3) above. This includes a method for preparing a non-human mammal comprising modifying starting ES cells according to steps (a) to (c). The ES cells may subsequently processed according one or more of the following steps:
(d) the ES cells obtained in steps (b) or (c) are injected into blastocysts; and/or
(e) transgenic non-human animals carrying one or more functional genes of interest at the Rosa26 locus are generated (viz. by well known breeding procedures).

The transgenic multi-cell organisms and non-human mammals obtainable by the method (6) and (7), respectively; preferably have an operatively functional gene expression cassette (as defined above) integrated into its Rosa26 locus. Such transgenic multi-cell organisms and non-human mammals are suitable for gene function studies, drug development, as disease model animals, etc.

The invention is further explained by the following examples and the attached figures, which are, however not to be construed so as to limit the invention.

### Examples

### Materials and Methods

Cell culture: Culture and targeted mutagenesis of ES cells were carried out as described in Hogan et al., (Cold Spring Harbor Laboratory Press, Cold Spring Harbor NY.), pp. 253-289 with ES cell lines derived from both inbred and F1 embryos (Examples 1 and 2). In Example 3 Art4.12 ES cells (Seibler et al., Nucl. Acid Res., 31(4):e12 (2003) were used.

Mice: All mice were kept in the animal facility at Artemis Pharmaceuticals GmbH in microisolator cages (Tecniplast Sealsave). B6D2F1 Mice for the generation of tetraploid blastocysts were obtained from Janvier. The *polb*^{*flox*}/*rosa(CreER*^{*T2*}*)* and *ect2*^{*flox*}/*rosa(CreER*^{*T2*}*)* mice were generated by breeding of *rosa(CreER*^{*T2*}*)* ES mice with βT14 (Gu et al., *Science,* 265, 103-106.), respectively.

Production of ES mice by tetraploid embryo complementation: The production of mice by tetraploid embryo complementation was essentially performed as described (Eggan et al., *Proc Natl Acad Sci USA*, 98, 6209-6214.).

Ligand administration: 100 mg Tamoxifen-free base (Sigma, T5648) was suspended in 100 µl Ethanol and solved in 1 ml sunflower oil (Sigma). This 10 mg/100 µl tamoxifen solution was sonicated for 1-2 minutes and then stored at -20°C. For p.o. administration the solution was thawed at 55°C and administrated to 4-8 week old mice by a feeding needle (FST Fine Science Tools GmbH, 18061-20).

Western blot analysis: Western blot analysis was performed using SDS-PAGE (NuPAGE, Invitrogen) and the Breeze Immunodetection System (Invitrogen) according to the manufacturer protocols. Immunodetection was done using sc-543 (HC-20, Santa Cruz Biotechnology, Inc.) against ER, PRB-106C against cre, actin sc-1616 Actin (I-19) against actin and rabbit polyclonal IgG (Santa Cruz Biotechnology, Inc.) antibodies.

X-Gal staining on tissue sections: To detect beta-galactosidase activity, tissues were embedded in Tissue Tec OCT (Sakura Finetek Europe B.V., The Netherlands), frozen on dry ice and cut into microsections. The sections were mounted onto slides and dried for 1-4 hours at room temperature. Sections were fixed for 5 min at room temperature in fixing solution (0,2% glutaraldehyde, 5 mM EGTA, 2mM MgCl₂ in 0.1 M PB ((0.1 M K₂HPO₄, pH 7.3)) and washed three times for 15 min at room temperature in washing buffer (2 mM MgCl₂, 0.02% Nonidet-40 in 0.1 M PB). Subsequently, tissues were stained for beta-galactosidase activity over night at 37°C using X-Gal solution (0.6 mg/ml X-Gal (predissolved in DMSO), 5 mM potassium hexacyanoferrat III, 5 mM potassium hexacyanoferrat II, in washing buffer). Sections were washed twice for 5 min at room temperature in PBS, counterstained with Nuclear Fast Red Solution for 10 min, rinsed shortly in aqua dest., dehydrated through a graded ethanol series and mounted in Eukitt (Sigma, Germany).

### Example 1 (Reference Example)

CreER Rosa-targeting vector: A 129 SV/EV-BAC library (Incyte Genomics) was screened with a probe against *exon2* of the *Rosa26* locus (amplified from mouse genomic DNA using Rscreen1s (GACAGGACAGTGCTTGTTTAAGG) (SEQ ID NO:1) and Rscreenlas (TGACTACACAATATTGCTCGCAC) (SEQ ID NO:2)). Out of the identified BACclone a 11 kb EcoRV subfragment was inserted into the HindII site of pBS. Two fragments (a 1 kb SacII/XbaI- and a 4 kb XbaI-fragment, SEQ ID Nos:4 and 5, respectively) were used as homology arms and inserted into a vector containing a FRT-flanked neomycin resistance gene (unpublished) to generate the basic Rosa26 targeting vector. The CAGGS-promoter (SEQ ID NO:6, nucleotides 1-1616) or a splice acceptor site (SA) from adenovirus (Friedrich G., Soriano P., Genes Dev., 5:1513-23 (1991)) were inserted between the 5' arm and the FRT flanked neomycin resistance gene. The CreER^{T2} and a polyadenylation site (pA; SEQ ID NO:6, nucleotides 3921-4099) were cloned 3' of the SA or the CAGGS-promoter. The vector is free of a transcriptional stop sequence 5' to the CAGGS-promoter

A CreER^{T2} gene (Feil et al., (1997) *Biochem Biophys Res Commun.,* 237, 752-757) under the control of the CAGGS-promoter (Okabe, Fabs Letters 407:313-19 (1997)) was inserted into the rosa26 locus by homologous recombination in ES cells by utilizing the CreER Rosa-targeting vector as described above (Fig. 1). In addition to the *CreER*^{*T2*} gene a splice acceptor sequence (Friedrich and Soziano (1991), *Genes Dev.,* 9, 1513-1523) was introduced as a control for the endogenous activity of the rosa26 gene promoter (Fig. 1). A *loxP*-flanked hygromycin resistance gene was introduced into the second allele of *rosa26* to provide test substrate for Cre *ER*^{*T2*} (Seibler et al., Nucl. Acids. Res. Feb. 15, 2003, 31(4):(12) (2003)), in press). ES cells modified at both rosa26 alleles were injected into tetraploid blastocysts and completely ES cell derived mice were generated (Eggan et al., (2001). *PNAS,* 98, 6209-6214). *Rosa(SA-CreER*^{*T2*}/*reporter)* and *Rosa(CAGGS-CreER*^{*T2*}/*reporter)* mice were fed with daily 5 mg Tamoxifen for 5 days and recombination of the reporter was analyzed 3 days after the last administration. Southern analysis of genomic DNA from different organs showed up to 50% recombination in the *Rosa(SA-CreER*^{*T2*}/*reporter)* mice and up to 90% recombination in the *rosa(CAGGS-CreER*^{*T2*}/*reporter)* mice, respectively (Fig. 2A). As the second substrate, we used the loxP flanked DNA *polymerase β* gene segment (*polβ*^{*flox*}) (Gu et al., (1994). *Science,* 265, 103-106). The *polβ*^{*flox*}/*rosa(SA-CreER*^{*T2*}*)* and *polβ*^{*flox*}/*rosa(CAGGS-CreER*^{*T2*}mice were fed with 5 mg tamoxifen per day for 5 days and analyzed 3 days later. Southern blot analysis revealed that the *loxP*-flanked *polymerase β* gene segment was excised in more than 90% of cells in all organs except brain in the *rosa(SA-CreER*^{*T2*}/*reporter)* mice (Fig. 2B). In contrast, the degree of inducible recombination was significantly higher in *rosa(CAGGS-CreER*^{*T2*}/*reporter)* mice, reaching 100% efficiency in most organs and up to 70% in brain.

To investigate the pattern and level of *CreER*^{*T2*} expression in *rosa(SA-CreER*^{*T2*}*)* and *rosa(CAGGS- CreER*^{*T2*}*)* mice, we performed Western analysis using antibodies specific for Cre. The 74 kDa band corresponding to the CreER^{T2} fusion protein was detectable in all organs of *rosa(CAGGS- CreER*^{*T2*}*)* mice, including brain (Fig. 3). In contrast, the CreER^{T2} expression level in *rosa(SA-CreER*^{*T2*}*)* mice was significantly lower compared to the *rosa(CAGGS-CreER*^{*T2*}*)* strain and appeared to be undetectable in brain (Fig. 3).

### Example 2 (Reference Example)

FABP-Cre Rosa-targeting vector (SEO ID NO:8): The splice acceptor site from adenovirus (SEQ ID NO:8, nucleotides 18569-18689) was inserted into the basic Rosa26 targeting vector described in 1. above. Into the SwaI and AscI restriction sites of the resulting plasmid was inserted a 3195 bp Xba_{blunt}/AscI DNA fragment comprising in 5' to 3' order the polyadenylation signal from the human growth hormone gene (SEQ ID NO:8, nucleotides 18760-688; Bond et al, Science 289:1942-1946 (2000)), a modified Fabpl promoter (SEQ ID NO:8, nucleotides 702-1481; Fabpl^{4x at -132}; Simon et al., J. Biol. Chem. 272:10652-10663 (1997)), a synthetic intron (SEQ ID NO:8, nucleotides 1521-1758), the Cre coding sequence (SEQ ID NO:8, nucleotides 1778-2830) and a synthetic polyA signal (SEQ ID NO:8, nucleotides 2888-3066).

A Cre gene under the control of the Fabpl^{4x at -132}-promoter (SEQ ID NO:8; Fig. 4) was inserted into the Rosa26 locus by homologous recombination in F1 ES cells carrying a Cre reporter substrate in the second Rosa26 allele. LacZ expression from the reporter construct (SEQ ID NO:9; Fig. 5) is activated upon Cre-mediated recombination. Targeted ES cells were injected into tetraploid blastocysts to generate FABP-Cre/reporter-substrate double transgenic ES mice. The Cre recombination pattern in these mice was examined by analyzing beta-galactosidase activity in tissues sections (Fig. 6). Cre-mediated recombination in these mice was restricted to the intestinal epithelium, liver and part of the cells in the epithelium of the tubuli in the kidney, thus exactly reflecting the expression pattern of the endogenous Fabpl gene (Simon et al., J. Biol. Chem., 272:10652-10663 (1997)).

### Example 3

### Rosa targeting and exchange vectors

*Rosa26 RMCE targeting vector* (SEQ ID NO:11): A 129 SV/EV-BAC library (Incyte Genomics) was screened with a probe against *exon2* of the *Rosa26* locus (SEQ ID NO:3). The *exon2* probe was amplified from mouse genomic DNA using primers Rscreen1s (GACAGGACAGTGCTTGTTTAAGG; SEQ ID NO:1) and Rscreenlas (TGACTACACAATATTGCTCGCAC; SEQ ID NO:2). A 11 kb EcoRV fragment isolated from the identified BAC clone a was inserted into the HindII site of pBS. Two subfragments from the 11 kb EcoRV fragment, the 1 kb SacII/XbaI- (SEQ ID NO:4) and the 4 kb XbaI-fragment (SEQ ID NO:5), were used as homology arms and inserted into a vector containing a FRT-flanked neomycin resistance gene (unpublished) to generate the basic Rosa26 targeting vector (SEQ ID NO:10). A splice acceptor site (SA) from adenovirus (Friedrich G., Soriano P., Genes Dev., 5:1513-23 (1991)) was inserted between the 5' arm and the FRT flanked neomycin resistance gene. The neomycin was deleted by Flp-mediated deletion in bacteria (Buchholz et al., Nucleic Acids Res. 1996, 24:3118-9). The final Rosa(RMCE) targeting vector (SEQ ID NO:11, Fig. 7A) was generated by standard cloning procedures and has the following order in 5' to 3' direction: a ATG start codon, a F3 site (Schlake & Bode (1994) Biochemistry 33, 12746- 12751; (SEQ ID NO:11, nucleotides 1292-1339)), a zsgreen ORF (Clontech; SEQ ID NO:11, nucleotides 1407-2099), a synthetic polyA signal (SEQ ID NO:11, nucleotides 2121-2299), a PGK-hygro resistance gene (SEQ ID NO:11, nucleotides 2314-4335), a CAGGS-promoter (SEQ ID NO:11, nucleotides 4397-6012), a Flp^{e-}recombinase gene (Buchholz et al., Nat Biotechnol. 1998, 16:657-62.), a synthetic polyA signal (SEQ ID NO:11, nucleotides 7728-7906), and a FRT site (SEQ ID NO:11, nucleotides 7922-7969) 5' of the 3' homology arm.

*Exchange vector* (SEQ ID NO:12): The vector contains the F3 site and the FRT site in the same configuration as in the Rosa26 targeting vector described above. The vector was generated using standard cloning procedures and has the following order in 5' to 3' direction: a synthetic polyA signal (SEQ ID NO:12, nucleotides 23-201), a F3-site (SEQ ID NO:12, nucleotides 216-263), a neomycin-resistance gene lacking the start ATG (SEQ ID NO:12, nucleotides 271-1559), a H1-promoter (SEQ ID NO:12, nucleotides 1763-1996), a hairpin sequence (SEQ ID NO:12, nucleotides 1997-2051), and a FRT site (SEQ ID NO:12, nucleotides 2161-2208).

### Cell culture

### ES cell culture and homologous recombination were carried out as previously described (Hogan et al., (Cold Spring Harbor Laboratory Press, Cold Spring Harbor NY.), pp. 253-289.)

*Transfection of cells with the exchange vector:* 1 day before transfection, 2 x 10⁵ ES cells were plated on a 3 cm dish in 2 ml medium. Before transfection 2 ml fresh medium was given to the cells. 3 µl Fugene6 Reagent (Roche; Cat No. 1 814 443) was mixed with 100 µl serum free medium (OptiMEM 1 with Glutamax-I Invitrogen; Cat.No. Cat.No. 51985-035) and incubated for 5 min. 100 µl of the Fugene/OptiMEM solution was added to 2 µg circular DNA (c = 0.33 µg/µl) and incubated for 15 min. This transfection complex was added drop wise to the medium and mixed by a circuiting movement. Fresh medium was added to the transfected cells the following day. From day 2, the medium was changed daily replaced by medium containing 250 µg/ml G418 (Geneticin; Invitrogen; Cat.No. 10131-019). 7 days after transfection, single clones were isolated by standard procedures as described (Hogan et al., (Cold Spring Harbor Laboratory Press, Cold Spring Harbor NY.), pp. 253-289.).

The targeting vector to prepare the Rosa26 locus for RMCE is depicted in figure 7A. The vector carries a FLP^{e} expression cassette to provide the recombinase for RMCE. The hygromycine resistance gene was used for positive selection of homologous recombinant clones. In addition, a zsGreen gene was placed between the FRT and F3 sites to allow for the identification of recombinant clones that have not undergone RMCE following secondary transfection of the exchange vector. The splice acceptor site (SA) and the ATG start codon should facilitate expression of the truncated neomycine resistance gene (Δ5'neo^{R}) on the exchange vector by employing the endogenous rosa26 promoter following RMCE.
The hybrid ES cell line ART4.12 ([C57BL/6 x 12956/SvEvTac] F1) was used for homologous recombination, since these lines are capable to derive completely ES cell derived mice (ES mice) through tetraploid blastocyst complementation with high efficiency (Seibler et al., Nucl. Acid Res., 31(4):e12 (2003). ART4.12 cells where transfected with the rosa26 targeting vector and incubated in cell culture medium containing hygromycin B. Independent recombinant Rosa(RMCE) ES cell clones were obtained at a frequency of 2 % as verified by Southern blot analysis (Fig. 8, first and second lane.
The exchange vector (Fig.7B) carries the FRT and F3 sites together with a truncated neo^{R} gene for positive selection of RMCE. The shRNA expression cassette served as a test transgene for targeted integration into the Rosa26 locus. The upstream polyA signal was included to prevent expression of the truncated neo^{R} gene in ES cells carrying randomly integrated vectors. The configuration of the targeted Rosa26 locus following RMCE is depicted in Fig. 7C.
Rosa(RMCE) ES cells where transfected with the exchange vector and selected in medium containing G418. Southern blot analysis of G418 resistant colonies revealed that successful RMCE had occurred in >90 % of clones (Fig. 8). This is the first demonstration of efficient RMCE for targeted transgenesis at a ubiquitously expressed locus.

## Claims

1. Method for generating transgenic eukaryotic cells having an ubiquitous locus modified by an expression cassette comprising a short hairpin RNA construct operatively linked to a promoter or an inactive precursor thereof, which method comprises introducing the expression cassette into the ubiquitous locus of eukaryotic cells by recombinase mediated cassette exchange.

2. The method of claim 1, which comprises
(a) introducing a functional DNA sequence into the Rosa26 locus of starting eukaryotic cells by homologous recombination with a targeting vector comprising flanking DNA sequences homologous to the ubiquitous locus and an acceptor DNA, which integrates into the genome of the starting cell, the acceptor DNA comprising two mutally incompatible first recombinase recognition sites (RRSs), and
(b) effecting recombinase mediated cassette exchange of the recombination product of step (a) having RMCE target sites with an exchange vector comprising a donor DNA, which comprises the expression cassette flanked by the same two mutually incompatible first RRSs contained in the acceptor DNA, by utilizing a recombinase which catalyzes recombination between the RRSs of the acceptor and donor DNA.

3. The method of claim 1 or 2, wherein
(i) the eukaryotic cells are derived from multi-cell organisms including vertebrates, invertebrates and plants, preferably are vertebrate cells, more preferably are derived from mammals, such as humans or non-human mammals, including rodents such as mouse, rat, etc., or are derived from fish such as zebrafish; and/or
(ii) the eukaryotic cells are primary cells or immortalized cells, preferably the cells are mammalian embryonic stem (ES) cells; and/or
(iii) the ubiquitous locus is selected from Rosa26, Collagen, β-Actin, HPRT, U6, H1, tRNA, 7SL RNA, etc., preferably is a Rosa26 locus; and or
(iv) the method is performed *in vitro.*

4. The method according to any one of claims 1 to 3, wherein
(i) the promoter of the expression cassette is a heterologous promoter and preferably is a ubiquitous or tissue specific promoter, either constitutive or inducible; and/or
(ii) the targeting vector, the exchange vector and/or the expression cassette further comprises one or more additional functional sequences including but not limited to marker genes, second recombinase recognition sites differing from the first recombinase recognition sites, poly A signal, introns, etc.; and/or
(iii) the targeting vector and the exchange vector further comprises tags for protein detection, enhancers, selection markers, etc.; and/or
(iv) the targeting vector further comprises a gene coding for the recombinase which catalyses recombination between acceptor and donor DNA; and/or
(v) in step (a) the DNA sequences homologous to the ubiquitous locus are 0.2 to 20 kB, preferably 1 to 10 kB long; and/or
(vi) the mutually incompatible RRS are selected from pairs of mutually incompatible IoxP, FRT, and Att sites or variants thereof, preferably are selected from the following group of mutually incompatible RRS pairs: F3/FRT, F5/FRT, F5/F3, lox/lox511, lox/lox2722, lox66/lox71 and AttB/AttP; and/or
(vii) the recombinase, which may be added to the cell or may be expressed by the cell, is selected from recombinases suitable for cassette exchange of the first RSSs present in the acceptor/donor DNA, such as Cre, Flp, ΦC31 or mutants thereof; and/or
(viii) the shRNA comprises
(I) at least one DNA segment A-B-C wherein
A is a 15 to 35, preferably 19 to 29 bp DNA sequence with at least 95%, preferably 100% complementarily to the gene to be knocked down;
B is a spacer DNA sequence having 5 to 9 bp forming the loop of the expressed RNA hair pin molecule, and
C is a 15 to 35, preferably 19 to 29 bp DNA sequence with at least 85% complementarily to the sequence A; and
(II) a stop and/or polyadenylation sequence.

5. The method according to claims 4, wherein
(i) the ubiquitous promoter is selected from polymerase I, II and III dependent promoters, preferably is a polymerase II or III dependent promoter, most preferably is a CMV promoter, a CAGGS promoter, a Mx promoter, a PGK promoter, a snRNA promoter such as U6, a RNAse P RNA promoter such as H1, a tRNA promoter, a 7SL RNA promoter, and a 5 S rRNA promoter, and the tissue specific promoter is FABP, Lck, CamKII, CD19, Keratin, Albumin, aP2, Insulin, MCK, MyHC, WAP, Col2A promoter, etc.; and/or
(ii) the ubiquitous promoter is a constitutive promoter, or is an inducible promoter, preferably a promoter containing an operator sequence selected from tet, Gal4, lac, or RRSs for recombinase mediated control, etc.

6. The method of claim 4, wherein the promoter is
(i) a Pol III dependent promoter, preferably constitutive H1 or U6, driven shRNA construct suitable to be integrated into a ubiquitously active Pol II dependent locus;
(ii) a Pol III dependent promoter, preferably inducible U6 or H1, driven shRNA construct suitable to be integrated into a ubiquitously active Pol II dependent locus; or
(iii) a Pol II dependent promoter, preferably inducible CMV, driven shRNA construct suitable to be integrated into a ubiquitously active Pol II dependent locus.

7. The method of claim 3 to 6, wherein the transgenic eukaryotic cells are derived from mouse and the ubiquitous locus is a Rosa26 locus, and
(i) the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and/or
(ii) the RRSs of the targeting and exchange vectors are F3/Frt and the targeting vectors encodes the recombinase Flp or a mutant thereof, preferably Flp^{e}; and/or
(iii) the targeting vector comprises a negative selection marker; and/or
(iv) the exchange vector comprises a promoter-less positive selection marker; and/or
(v) the promoter of the expression cassette is a H1 or H6;
most preferably the targeting vector has the sequence shown in SEQ ID NO:11 and the exchange vector has the sequence shown in SEQ ID NO:12 or a variant thereof with modification in the short hairpin RNA construct.

8. The method according to any one of claims 2 to 7, which further comprises one or more of the steps
(c) isolating the eukaryotic cells, preferably the ES cells having the desired functional exchange cassette or the inactive precursor integrated into the ubiquitous locus; and
(d) optionally modifying the integrated precursor of the expression cassette to activate the precursor and isolating ES cells having the desired modified functional exchange cassette.

9. An exchange vector comprising the expression cassette and a donor DNA, as defined in claims 2 to 8.

10. A eukaryotic cell having a modified ubiquitous locus obtainable by the method of claims 1 to 8.

11. A method for preparing transgenenic multi-cell organism having a modified ubiquitous locus which comprises
(a) transfecting eukaryotic cells according to the method defined in claims 1 to 8; or
(b) injecting an exchange vector as defined in claim 9 into an early stage embryo of a non-human mammal having corresponding RMCE target sites.

12. The method of claim 11, wherein the transgenenic multi-cell organism is a non-human mammal, said ubiquitous locus is a Rosa26 locus, and said method comprises modifying an ES cell as defined in claims 1 to 8.

13. The method of claim 11 or 12 which further comprises one or more of the steps (e) injecting ES cells obtained in steps (c) or (d) into blastocysts; and (f) generating transgenic non-human multi-cell organisms or non-human mammals carrying one or more functional genes of interest at the Rosa26 locus.

14. A transgenic multi-cell organism or a tissue culture derived therefrom, and a transgenic non-human mammal or a tissue culture derived therefrom, which are obtainable by the method of claims 11 to 13, respectively, and having an operatively functional gene expression cassette integrated into at least one of its loci.

15. Use of an exchange vector comprising a short hairpin RNA construct (shRNA) under control of a promoter, preferably as defined in claims 2 to 8, for preparing (i) an agent for constitutive and/or inducible gene knock down in a multi-cell organism, or in tissue culture or cells of a cell culture derived from a multi-cell organism, or (ii) an agent for injecting the exchange vector as defined in claim 9 into cells of an eight cell stage embryo of non-human mammals.

16. The use of claim 15, wherein the expression vector is suitable for stable integration into the genome of the multi-cell organism, or into the genome of the tissue culture or of cells of the cell culture thereof, at an ubiquitous locus, preferably at the Rosa26 locus thereof.

17. A method for constitutive and/or inducible gene knock down in a multi-cell organism, or in a tissue culture or cells of a cell culture derived from said multi-cell organism, which comprises stably integrating an expression vector as defined in any one of claims 2 to 8 into the genome of the living organism, of the tissue culture or of the cells of the cell culture.

18. The method of claim 17, wherein
(i) the expression vector is integrated at the Rosa26 locus of the multi-cell organism, tissue culture or cell culture; and/or
(ii) the method for constitutive and/or inducible gene knock down in a vertebrate comprises integrating the expression vector into ES cells of the vertebrate.

19. Use of the eukaryotic cell of claim 11, the transgenic multi-cell organism, tissue culture, or non-human mammal of claim 14 for gene function studies, drug development, as disease model animals, etc.
